# EUROPÄISCHE PATENTSCHRIFT

(11) **EP 3 347 354 B1**
(45) Veröffentlichungstag und Bekanntmachung des Hinweises auf die Patenterteilung: **17.06.2020**
(21) Anmeldenummer: 16753585.5
(22) Anmeldetag: 12.08.2016
(51) Int. Cl.: C07D 405/14, C07D 401/10, C07D 403/10, C07D 405/10, C07D 409/10, C07D 409/14, C07D 491/04, C07D 209/08, C07D 209/10, C09K 11/06, H01L 51/50

(54) **MATERIALIEN FÜR ORGANISCHE ELEKTROLUMINESZENZVORRICHTUNGEN**
MATERIALS FOR ORGANIC ELECTROLUMINESCENT DEVICES
MATIÈRES POUR DISPOSITIFS ÉLECTROLUMINESCENTS ORGANIQUES

(30) Priorität: 08.09.2015 EP 15184191
(43) Veröffentlichungstag der Anmeldung: 18.07.2018
(73) Patentinhaber: Merck Patent GmbH, 64293 Darmstadt (DE)
(72) Erfinder: JATSCH, Anja, 60489 Frankfurt am Main (DE); PARHAM, Amir Hossain, 60486 Frankfurt am Main (DE); EBERLE, Thomas, 76829 Landau (DE); GROSSMANN, Tobias, 64297 Darmstadt (DE); KROEBER, Jonas Valentin, 60311 Frankfurt am Main (DE)
(86) Internationale Anmeldenummer: PCT/EP2016/001391
(87) Internationale Veröffentlichungsnummer: WO 2017/041874

(56) Entgegenhaltungen:
- EP-A1- 2 599 773
- WO-A1-2014/204464
- WO-A2-2011/037429

## Beschreibung

Die vorliegende Erfindung betrifft Materialien für die Verwendung in elektronischen Vorrichtungen, insbesondere in organischen Elektrolumineszenzvorrichtungen, sowie elektronische Vorrichtungen, insbesondere organische Elektrolumineszenzvorrichtungen, enthaltend diese Materialien.

In organischen Elektrolumineszenzvorrichtungen (OLEDs) werden als emittierende Materialien häufig metallorganische Komplexe eingesetzt, die Phosphoreszenz statt Fluoreszenz zeigen. Generell gibt es bei OLEDs, insbesondere auch bei OLEDs, die Triplettemission (Phosphoreszenz) zeigen, jedoch immer noch Verbesserungsbedarf, beispielsweise im Hinblick auf Effizienz, Betriebsspannung und Lebensdauer. Die Eigenschaften phosphoreszierender OLEDs werden nicht nur von den eingesetzten Triplettemittern bestimmt. Hier sind insbesondere auch die anderen verwendeten Materialien, insbesondere auch die Matrixmaterialien, von besonderer Bedeutung. Verbesserungen dieser Materialien können somit auch zu deutlichen Verbesserungen der OLED-Eigenschaften führen.

Gemäß dem Stand der Technik werden unter anderem Indenocarbazolderivate, z. B. gemäß WO 2010/136109, WO 2012/014500 oder WO 2013/041176, als Matrixmaterialien für phosphoreszierende Emitter in organischen Elektrolumineszenzvorrichtungen eingesetzt, beispielsweise Indenocarbazolderivate, welche mit Diarylaminogruppen bzw. Triarylaminogruppen substituiert sind. Bei den in diesen Dokumenten offenbarten Materialien sind weitere Verbesserungen wünschenswert, insbesondere in Bezug auf die Lebensdauer.

Aufgabe der vorliegenden Erfindung ist die Bereitstellung von verbesserten Indenocarbazolmaterialien, welche sich insbesondere für den Einsatz als Matrixmaterial für phosphoreszierende Emitter eignen.

Überraschend wurde gefunden, dass bestimmte, unten näher beschriebene Verbindungen diese Aufgabe lösen, sich gut für die Verwendung in OLEDs eignen und zu Verbesserungen der organischen Elektrolumineszenzvorrichtung führen, wobei die Verbesserungen insbesondere die Lebensdauer betreffen. Es handelt sich bei diesen Materialien um Indenocarbazolverbindungen, welche einen Triarylamin-Substituenten aufweisen, wobei das Amin mit einer 1-, 3- oder 4-Fluorenylgruppe, einer 1-, 3- oder 4-Dibenzofurangruppe oder einer 1-, 3- oder 4-Dibenzothiophengruppe substituiert ist. Diese Verbindungen sowie elektronische Vorrichtungen, insbesondere organische Elektrolumineszenzvorrichtungen, welche derartige Verbindungen enthalten, sind daher der Gegenstand der vorliegenden Erfindung.

Gegenstand der vorliegenden Erfindung ist daher eine Verbindung gemäß der folgenden Formel (1), 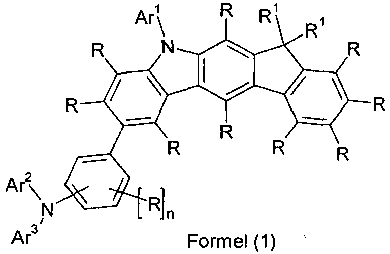 wobei für die verwendeten Symbole und Indizes gilt:
- Ar¹, Ar²: ist bei jedem Auftreten gleich oder verschieden ein aromatisches oder heteroaromatisches Ringsystem mit 5 bis 30 aromatischen Ringatomen, das mit einem oder mehreren Resten R, bevorzugt nicht-aromatischen Resten R, substituiert sein kann;
- Ar³: ist eine Gruppe der folgenden Formel (2), 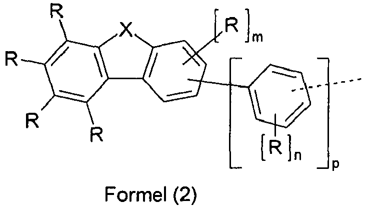 wobei die gestrichelte Bindung die Verknüpfung dieser Gruppe mit dem Stickstoffatom andeutet und die Fluoren- bzw. Dibenzofuran- bzw. Dibenzothiophengruppe über die 1-, 3- oder 4-Position für p = 1 mit der Phenylengruppe bzw. für p = 0 mit dem Stickstoffatom verknüpft ist;
- X: ist C(R¹)₂, O oder S;
- R, R¹: ist bei jedem Auftreten gleich oder verschieden ausgewählt aus der Gruppe bestehend aus H, D, F, Cl, Br, I, CN, NO₂, N(R²)₂, OR², SR², C(=O)R², P(=O)R², Si(R²)₃, einer geradkettigen Alkylgruppe mit 1 bis 20 C-Atomen oder einer verzweigten oder cyclischen Alkylgruppe mit 3 bis 20 C-Atomen oder einer Alkenyl- oder Alkinylgruppe mit 2 bis 20 C-Atomen, wobei die Alkyl-, Alkenyl- oder Alkinylgruppe jeweils mit einem oder mehreren Resten R² substituiert sein kann und wobei eine oder mehrere nicht-benachbarte CH₂-Gruppen durch R²C=CR², C≡C, Si(R²)₂, C=O, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R² substituiert sein kann; dabei können optional zwei benachbarte Substituenten R bzw. zwei benachbarte Substituenten R¹ ein monocyclisches oder polycyclisches, aliphatisches, aromatisches oder heteroaromatisches Ringsystem bilden, das mit einem oder mehreren Resten R² substituiert sein kann;
- R²: ist bei jedem Auftreten gleich oder verschieden ausgewählt aus der Gruppe bestehend aus H, D, F, CN, einem aliphatischen Kohlenwasserstoffrest mit 1 bis 20 C-Atomen, oder einem aromatischen oder heteroaromatischen Ringsystem mit 5 bis 30 aromatischen Ringatomen, in dem ein oder mehrere H-Atome durch D, F, CN oder eine Alkylgruppe mit 1 bis 10 C-Atomen ersetzt sein können, wobei zwei oder mehr benachbarte Substituenten R² miteinander ein mono- oder polycyclisches, aliphatisches Ringsystem bilden können;
- m: ist 0, 1, 2 oder 3;
- n: ist bei jedem Auftreten gleich oder verschieden 0, 1, 2, 3 oder 4;
- p: ist 0 oder 1.

Der Übersichtlichkeit halber wird im Folgenden die in dieser Anmeldung verwendete Nummerierung der Kohlenstoffatome im Fluoren bzw. Dibenzofuran bzw. Dibenzothiophen, wie es in der Struktur der Formel (2) auftritt, aufgeführt:

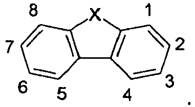

Eine Arylgruppe im Sinne dieser Erfindung enthält 6 bis 60 C-Atome; eine Heteroarylgruppe im Sinne dieser Erfindung enthält 2 bis 60 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei enthält die Heteroarylgruppe bevorzugt maximal drei Heteroatome, von denen maximal eines ausgewählt ist aus O oder S und die weiteren Heteroatome N sind. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl oder Bipyridin, werden dagegen nicht als Aryl- oder Heteroarylgruppe, sondern als aromatisches bzw. heteroaromatisches Ringsystem bezeichnet.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Dabei enthält das heteroaromatische Ringsystem bevorzugt pro Heteroarylgruppe, die in dem Ringsystem enthalten ist, maximal drei Heteroatome, von denen maximal eines ausgewählt ist aus O oder S und die weiteren Heteroatome N sind. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden.

Ein elektronenreiches heteroaromatisches Ringsystem im Sinne der vorliegenden Erfindung ist ein heteroaromatisches Ringsystem, welches keine elektronenarmen Heteroarylgruppen enthält. Elektronenarme Heteroarylringe im Sinne der vorliegenden Erfindung sind 5-Ring-Heteroarylringe mit zwei oder mehr Heteroatomen und 6-Ring-Heteroarylringe. Ein elektronenreiches heteroaromatisches Ringsystem enthält somit nur Arylringe sowie 5-Ring-Heteroarylringe mit genau einem Heteroatom, insbesondere Pyrrol, Furan und/oder Thiophen, wobei die Arylringe und die 5-Ring-Heteroarylringe auch aneinander ankondensiert sein können. Beispiele für geeignete elektronenreiche heteroaromatische Ringsysteme sind Carbazol, Dibenzofuran und Dibenzothiophen.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 40 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Gruppe OR² werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Gruppe SR² werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 60 aromatischen Ringatomen, welches noch jeweils mit den oben genannten Resten R² oder einem Kohlenwasserstoffrest substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombination dieser Systeme.

Unter benachbarten Resten bzw. benachbarten Substituenten im Sinne der vorliegenden Anmeldung werden Substituenten verstanden, die an C-Atome gebunden sind, welche wiederum direkt aneinander gebunden sind, oder Substituenten, die an dasselbe C-Atom gebunden sind.

In einer bevorzugten Ausführungsform der Erfindung weist das Indenocarbazolgrundgerüst insgesamt maximal zwei Substituenten R auf, die ungleich H sind, besonders bevorzugt maximal einen Substituenten R, der ungleich H ist, und ganz besonders bevorzugt keinen Substituenten R, der ungleich H ist. In einer weiteren bevorzugten Ausführungsform der Erfindung ist die Phenylengruppe, die die Indenocarbazolgruppe und die Gruppe -NAr²Ar³ miteinander verknüpft unsubstituiert. Ganz besonders bevorzugt handelt es sich also um eine Verbindung gemäß Formel (3), 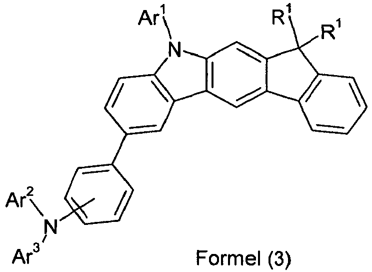 wobei die verwendeten Symbole die oben genannten Bedeutungen aufweisen.

In einer bevorzugten Ausführungsform der Erfindung sind die Reste R¹, die an den Indenocarbazolgrundkörper binden, bei jedem Auftreten gleich oder verschieden ausgewählt aus der Gruppe bestehend aus einer geradkettigen Alkylgruppe mit 1 bis 10 C-Atomen oder einer verzweigten oder cyclischen Alkylgruppe mit 3 bis 10 C-Atomen oder einer Alkenylgruppe mit 2 bis 10 C-Atomen, die jeweils mit einem oder mehreren Resten R² substituiert sein kann, wobei eine oder mehrere nicht-benachbarte CH₂-Gruppen durch O ersetzt sein können und wobei ein oder mehrere H-Atome durch D oder F ersetzt sein können, oder einem aromatischen oder heteroaromatischen Ringsystem mit 6 bis 24 aromatischen Ringatomen, das jeweils mit einem oder mehreren Resten R², bevorzugt nicht-aromatischen Resten R², substituiert sein kann; dabei können die beiden Substituenten R¹ ein monocyclisches oder polycyclisches, aliphatisches, aromatisches oder heteroaromatisches Ringsystem bilden, das mit einem oder mehreren Resten R² substituiert sein kann. Besonders bevorzugt sind die Reste R¹ bei jedem Auftreten gleich oder verschieden ausgewählt aus der Gruppe bestehend aus einer geradkettigen Alkylgruppe mit 1 bis 4 C-Atomen oder einer verzweigten oder cyclischen Alkylgruppe mit 3 bis 10 C-Atomen, die jeweils mit einem oder mehreren Resten R² substituiert sein kann, bevorzugt aber unsubstituiert ist, wobei eine oder mehrere H-Atome durch D oder F ersetzt sein können, oder einem aromatischen Ringsystem mit 6 bis 24 aromatischen Ringatomen, das mit einem oder mehreren nicht-aromatischen Resten R² substituiert sein kann, bevorzugt aber unsubstituiert ist; dabei können die beiden Substituenten R¹ ein monocyclisches oder polycyclisches, aliphatisches oder aromatisches Ringsystem bilden, das mit einem oder mehreren Resten R² substituiert sein kann, bevorzugt aber unsubstituiert ist. Ganz besonders bevorzugt sind die Reste R¹ gleich oder verschieden bei jedem Auftreten ausgewählt aus Methyl, Phenyl oder zwei Phenylgruppen, die miteinander ein Ringsystem bilden und so ein Spirosystem aufspannen. Bevorzugt sind also Verbindungen gemäß einer der folgenden Formeln (3-1) bis (3-4), 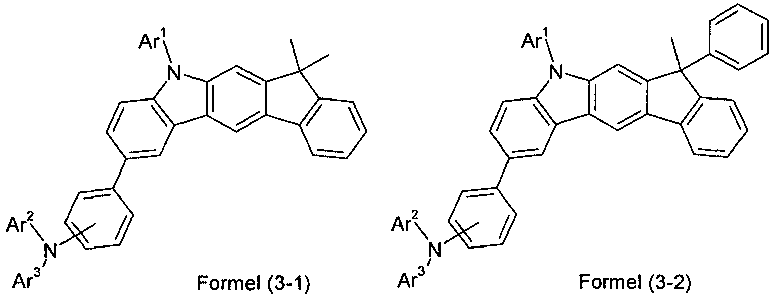 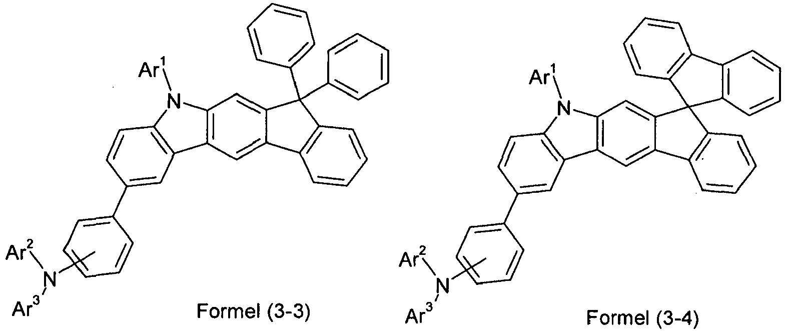 wobei die verwendeten Symbole die oben genannten Bedeutungen aufweisen.

In den Verbindungen der Formel (1) sind die Indenocarbazolgruppe und die Gruppe -NAr²Ar² durch eine optional substituierte, jedoch bevorzugt unsubstituierte Phenylengruppe miteinander verknüpft. Dabei kann die Phenylengruppe über die ortho-, meta- oder para-Positionen verknüpft sein, bevorzugt über die meta- oder para-Positionen und besonders bevorzugt über die para-Positionen. Bevorzugte Ausführungsformen der Verbindung der Formel (1) sind also die Verbindungen der folgenden Formeln (1a) und (1b), wobei die Verbindung der Formel (1b) besonders bevorzugt ist, 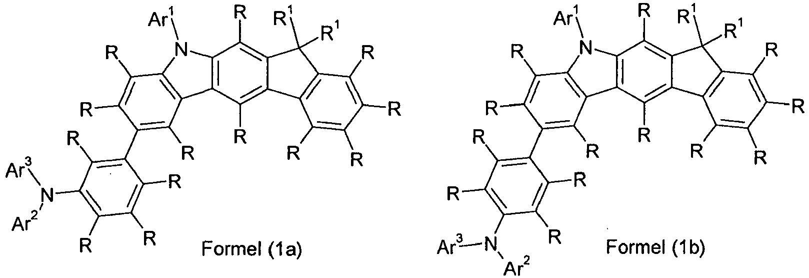 wobei die verwendeten Symbole die oben genannten Bedeutungen aufweisen.

Besonders bevorzugt sind die Verbindungen der folgenden Formeln (3a) und (3b), wobei die Verbindungen der Formel (3b) ganz besonders bevorzugt sind, 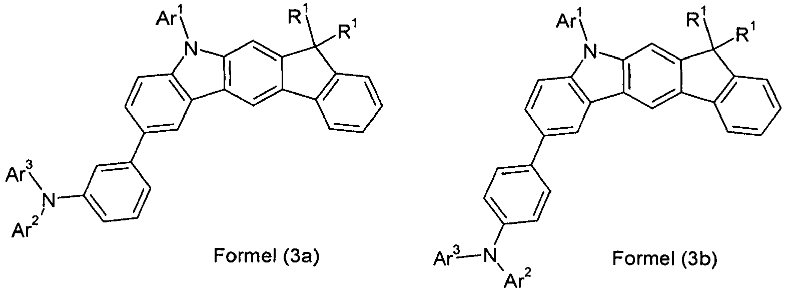 wobei die verwendeten Symbole die oben genannten Bedeutungen aufweisen.

Im Folgenden wird die Gruppe Ar¹ genauer beschrieben. In einer bevorzugten Ausführungsform der Erfindung ist Ar¹ ein aromatisches Ringsystem mit 6 bis 24 aromatischen Ringatomen, welches mit einem oder mehreren Resten R substituiert sein kann, oder ein elektronenreiches heteroaromatisches Ringsystem mit 5 bis 24 aromatischen Ringatomen, welches mit einem oder mehreren Resten R, bevorzugt mit nicht-aromatischen Resten R, substituiert sein kann. Besonders bevorzugt ist Ar¹ ein aromatisches Ringsystem mit 6 bis 24 aromatischen Ringatomen, welches mit einem oder mehreren nicht-aromatischen Resten R substituiert sein kann, bevorzugt aber unsubstituiert ist. Bevorzugte Gruppen Ar¹ sind ausgewählt aus der Gruppe bestehend aus Phenyl, Biphenyl, insbesondere ortho-, meta- oder para-Biphenyl, Terphenyl, insbesondere ortho-, meta-, para- oder verzweigtes Terphenyl, Quaterphenyl, insbesondere ortho-, meta-, para- oder verzweigtes Quaterphenyl, Fluorenyl, insbesondere 1-, 2-, 3- oder 4-Fluorenyl, Spirobifluorenyl, insbesondere 1-, 2-, 3- oder 4-Spirobifluorenyl, Dibenzofuranyl, insbesondere 1-, 2-, 3- oder 4-Dibenzofuranyl, Dibenzothienyl, insbesondere 1-, 2-, 3- oder 4-Dibenzothienyl, oder N-Phenyl-Carbazolyl, insbesondere N-Phenyl-1-, 2-, 3- oder 4-Carbazolyl, wobei diese Gruppen jeweils durch einen oder mehrere nicht-aromatische Reste R substituiert sein können, bevorzugt aber unsubstituiert sind. Ganz besonders bevorzugt sind Phenyl, Biphenyl, Terphenyl, Quaterphenyl und Fluorenyl.

Beispiele für geeignete Gruppen Ar¹ sind die im Folgenden abgebildeten Gruppen (Ar¹-1) bis (Ar¹-15), 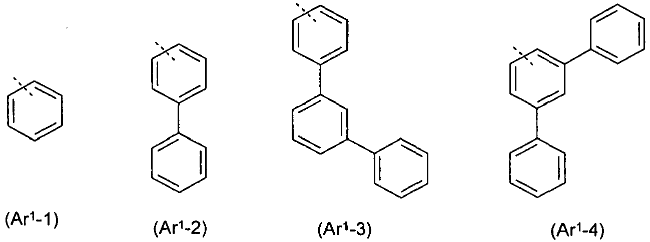 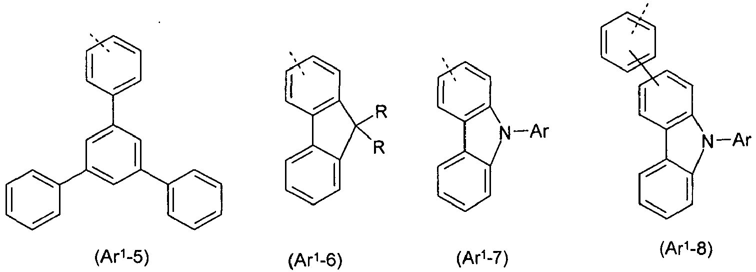 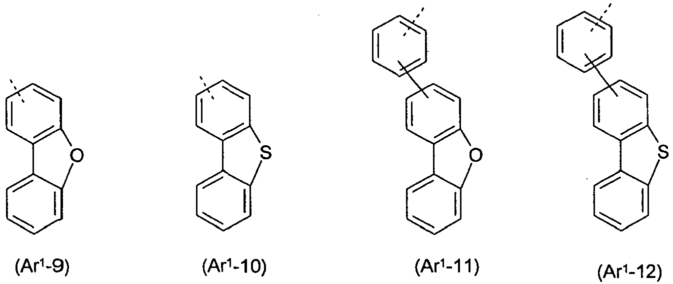 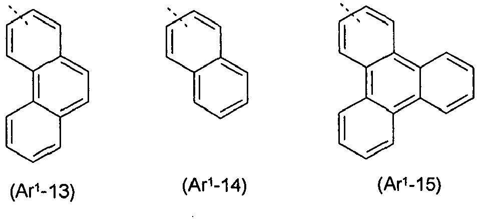 wobei die gestrichelte Bindung die Verknüpfung dieser Gruppe darstellt, die Strukturen auch durch einen oder mehrere Reste R, bevorzugt nicht-aromatische Reste R, substituiert sein können und die weiteren verwendeten Symbole die oben genannten Bedeutungen aufweisen. Bevorzugt sind die oben genannten Gruppen unsubstituiert.

Besonders bevorzugte Ausführungsformen der Gruppe Ar¹ sind die Gruppen der folgenden Formeln (Ar¹-1-a) bis (Ar¹-15-b), 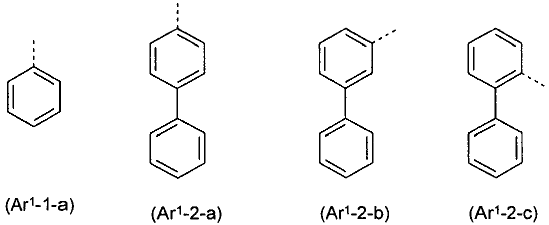 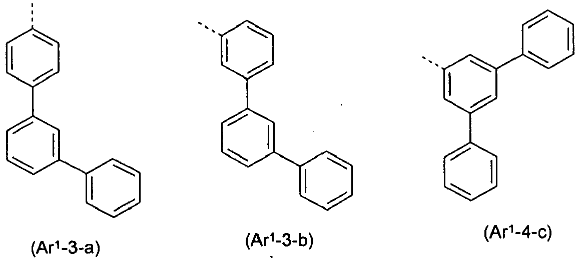 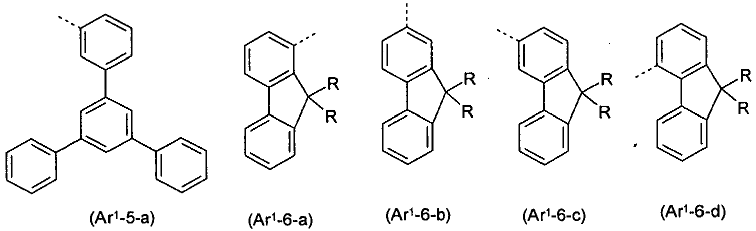 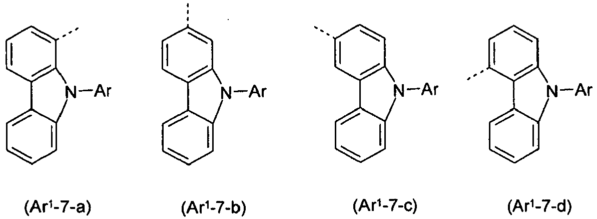 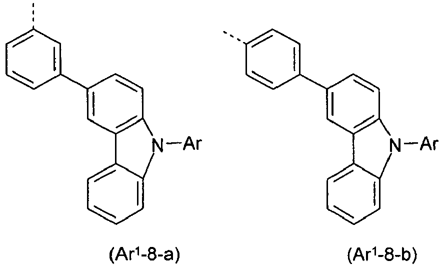 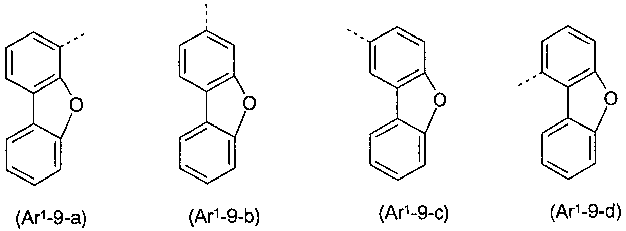 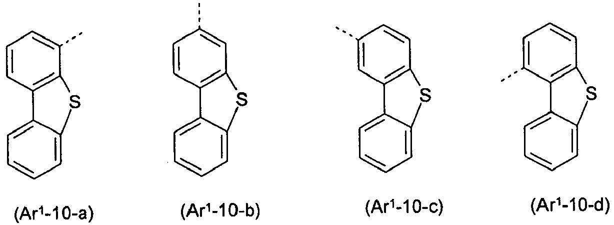 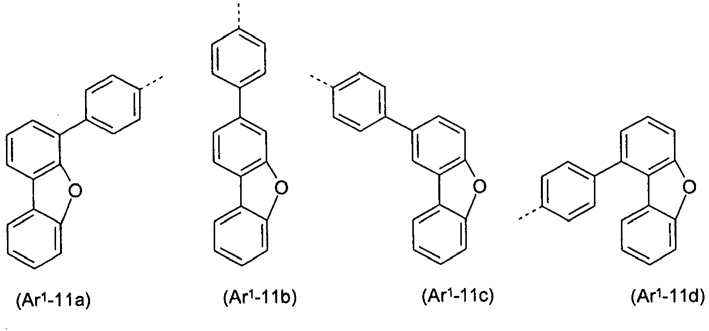 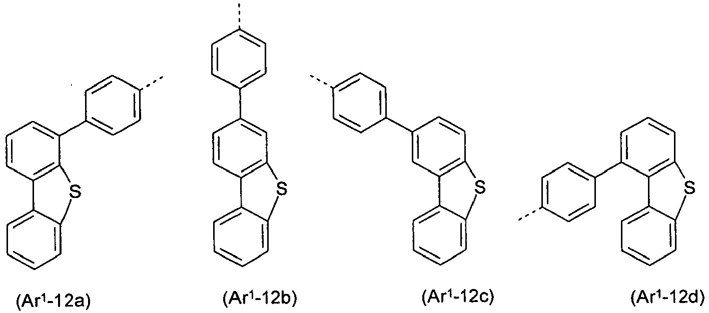 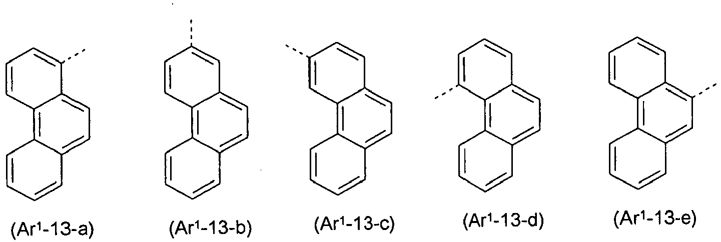 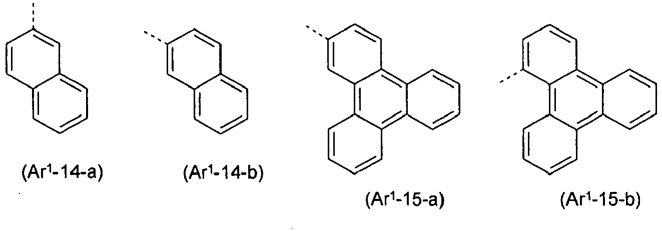 wobei die verwendeten Symbole die oben genannten Bedeutungen haben und die Strukturen auch durch einen oder mehrere Reste R substituiert sein können, bevorzugt aber unsubstituiert sind.

Im Folgenden wird die Gruppe Ar² genauer beschrieben. In einer bevorzugten Ausführungsform der Erfindung ist Ar² ein aromatisches Ringsystem mit 6 bis 24 aromatischen Ringatomen, welches mit einem oder mehreren Resten R substituiert sein kann, oder ein elektronenreiches heteroaromatisches Ringsystem mit 5 bis 24 aromatischen Ringatomen, welches mit einem oder mehreren Resten R, bevorzugt mit nicht-aromatischen Resten R, substituiert sein kann. Besonders bevorzugt ist Ar² ein aromatisches Ringsystem mit 6 bis 24 aromatischen Ringatomen, welches mit einem oder mehreren nicht-aromatischen Resten R substituiert sein kann, bevorzugt aber unsubstituiert ist. Bevorzugte Gruppen Ar² sind ausgewählt aus der Gruppe bestehend aus Phenyl, Biphenyl, insbesondere ortho-, meta- oder para-Biphenyl, Terphenyl, insbesondere ortho-, meta-, para- oder verzweigtes Terphenyl, Quaterphenyl, insbesondere ortho-, meta-, para- oder verzweigtes Quaterphenyl, Fluorenyl, insbesondere 1-, 2-, 3- oder 4-Fluorenyl, Spirobifluorenyl, insbesondere 1-, 2-, 3- oder 4-Spirobifluorenyl, Dibenzofuranyl, insbesondere 1-, 2-, 3- oder 4-Dibenzofuranyl, Dibenzothienyl, insbesondere 1-, 2-, 3- oder 4-Dibenzothienyl, oder N-Phenyl-Carbazolyl, insbesondere N-Phenyl-1-, 2-, 3- oder 4-Carbazolyl, wobei diese Gruppen jeweils durch einen oder mehrere nicht-aromatische Reste R substituiert sein können, bevorzugt aber unsubstituiert sind.

Beispiele für geeignete Gruppen Ar² sind die im Folgenden abgebildeten Gruppen (Ar²-1) bis (Ar²-15), 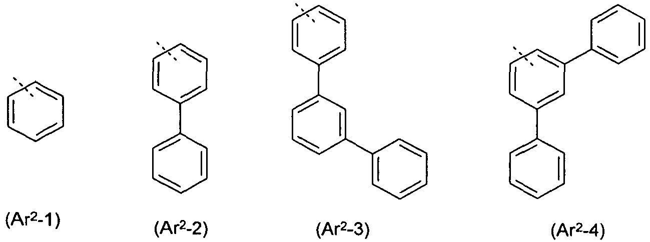 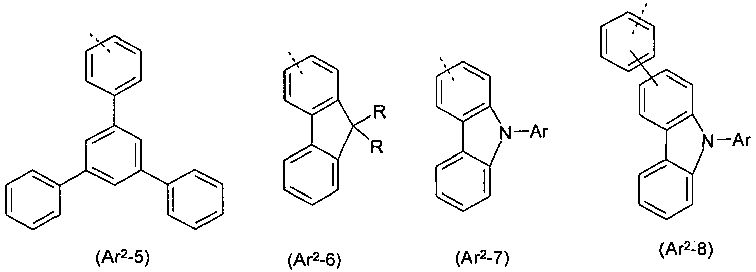 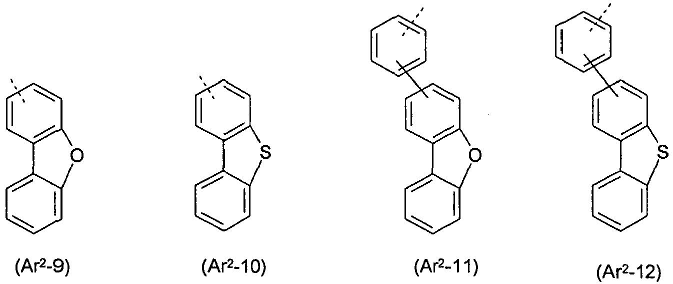 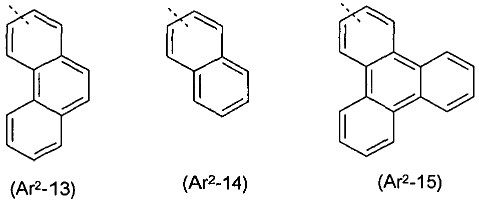 wobei die gestrichelte Bindung die Verknüpfung dieser Gruppe darstellt, die Strukturen auch durch einen oder mehrere Reste R, bevorzugt nicht-aromatische Reste R, substituiert sein können und die weiteren verwendeten Symbole die oben genannten Bedeutungen aufweisen.

Besonders bevorzugte Ausführungsformen der Gruppe Ar² sind die Gruppen der folgenden Formeln (Ar²-1a) bis (Ar²-15b), 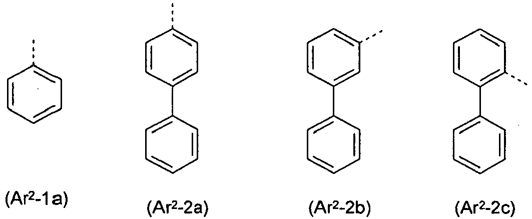 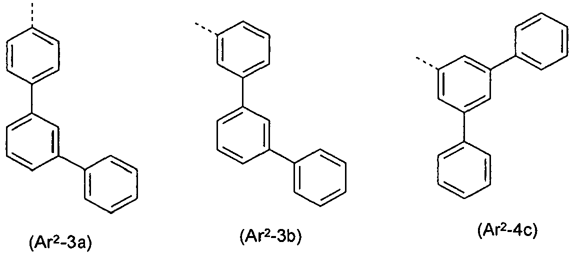 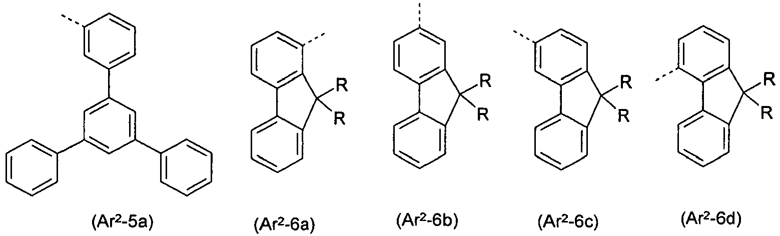 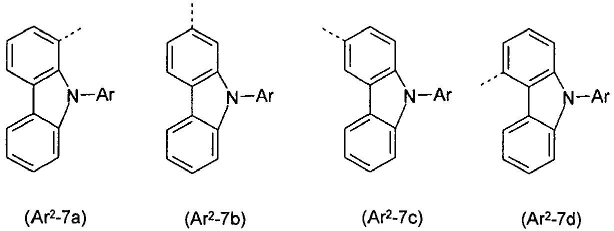 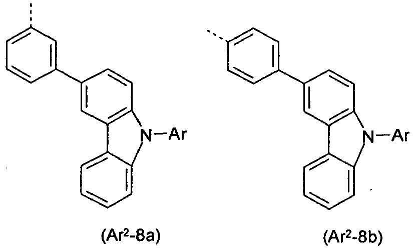 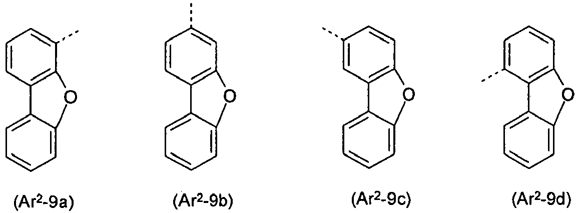 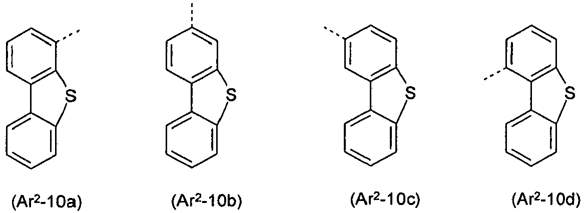 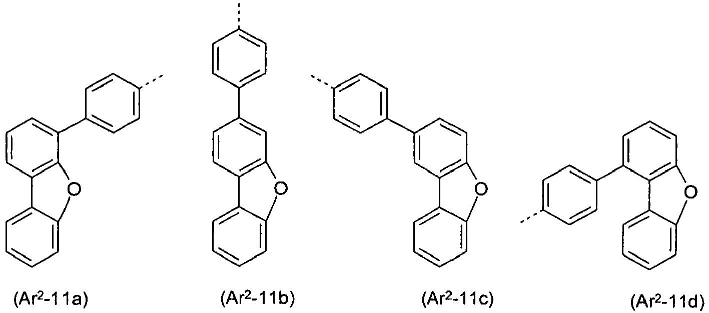 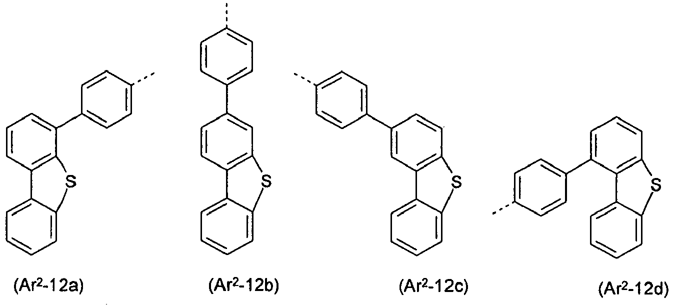 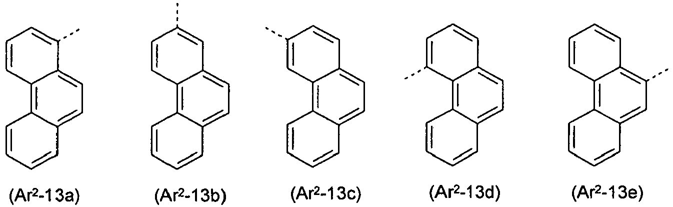 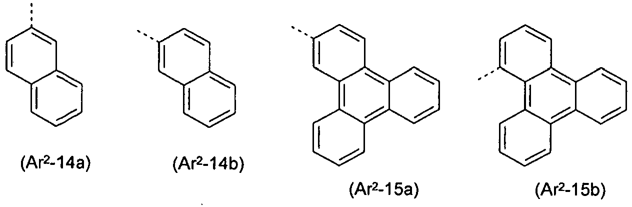 wobei die verwendeten Symbole die oben genannten Bedeutungen haben und die Strukturen auch durch einen oder mehrere Reste R substituiert sein können, bevorzugt aber unsubstituiert sind.

Im Folgenden wird die Gruppe Ar³, also die Gruppe der Formel (2), genauer beschrieben. In einer bevorzugten Ausführungsform der Erfindung ist p = 0. In einer weiteren bevorzugten Ausführungsform der Erfindung ist p = 1 und die Phenylengruppe steht für eine unsubstituierte meta- oder para-verknüpfte Phenylengruppe, insbesondere für eine para-verknüpfte Phenylengruppe. Bevorzugte Ausführungsformen der Gruppe der Formel (2) sind somit die Gruppen der Formeln (2a) bis (2c), 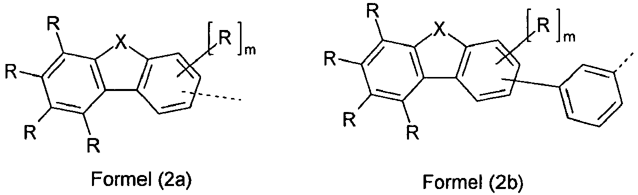 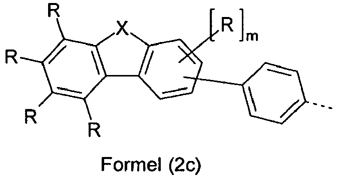 wobei die verwendeten Symbole und Indizes die oben genannten Bedeutungen aufweisen und die Fluorengruppe bzw. Dibenzofurangruppe bzw. Dibenzothiophengruppe erfindungsgemäß über die 1-, 3- oder 4-Position verknüpft ist. Besonders bevorzugt ist eine Gruppe der Formel (2a).

Ausführungsformen der Gruppe (2a) sind die Gruppen der folgenden Formeln (2a-1) bis (2a-9), 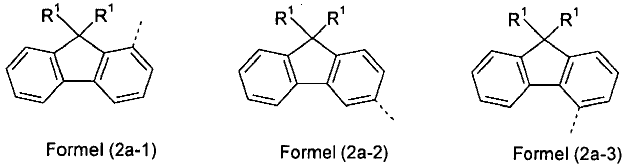 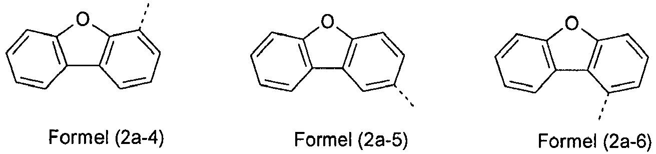 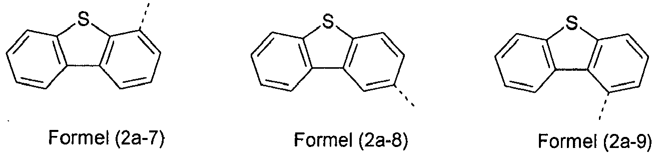 wobei die gestrichelte Bindung die Verknüpfung dieser Gruppe mit dem Stickstoffatom andeutet und die weiteren verwendeten Symbole die oben genannten Bedeutungen aufweisen.

Analog sind Gruppen der Formeln (2b) und (2c) möglich, wobei diese sich von den Strukturen der Formeln (2a-1) bis (2a-9) lediglich dadurch unterscheiden, dass sie zwischen der Fluorengruppe bzw. Dibenzofurangruppe bzw. Dibenzothiophengruppe und dem Stickstoffatom des Amins noch eine meta- bzw. para-Phenylengruppe enthalten.

Bevorzugt steht in der Gruppe der Formel (2) bzw. den bevorzugten Ausführungsformen X für C(R¹)₂ oder O, besonders bevorzugt für C(R¹)₂.

Wenn X für C(R¹)₂ steht, ist die Gruppe Ar³ bevorzugt eine Gruppe der oben genannten Formel (2a-1) oder (2a-3), besonders bevorzugt eine Gruppe der oben genannten Formel (2a-3). Wenn X für O steht, ist die Gruppe Ar³ bevorzugt eine Gruppe der oben genannten Formel (2a-4) oder (2a-6). Wenn X für S steht, ist die Gruppe Ar³ bevorzugt eine Gruppe der oben genannten Formel (2a-7) oder (2a-9).

In einer bevorzugten Ausführungsform der Erfindung sind die Reste R¹, die für X = C(R¹)₂ an das Fluoren der Formel (2) bzw. die bevorzugten Ausführungsformen binden, bei jedem Auftreten gleich oder verschieden ausgewählt aus der Gruppe bestehend aus einer geradkettigen Alkylgruppe mit 1 bis 10 C-Atomen oder einer verzweigten oder cyclischen Alkylgruppe mit 3 bis 10 C-Atomen oder einer Alkenylgruppe mit 2 bis 10 C-Atomen, die jeweils mit einem oder mehreren Resten R² substituiert sein kann, wobei eine oder mehrere nicht-benachbarte CH₂-Gruppen durch O ersetzt sein können und wobei ein oder mehrere H-Atome durch D oder F ersetzt sein können, oder einem aromatischen oder heteroaromatischen Ringsystem mit 6 bis 24 aromatischen Ringatomen, das jeweils mit einem oder mehreren Resten R², bevorzugt nicht-aromatischen Resten R², substituiert sein kann; dabei können die beiden Substituenten R¹ ein monocyclisches oder polycyclisches, aliphatisches, aromatisches oder heteroaromatisches Ringsystem bilden, das mit einem oder mehreren Resten R² substituiert sein kann. Besonders bevorzugt sind die Reste R¹ bei jedem Auftreten gleich oder verschieden ausgewählt aus der Gruppe bestehend aus einer geradkettigen Alkylgruppe mit 1 bis 4 C-Atomen oder einer verzweigten oder cyclischen Alkylgruppe mit 3 bis 10 C-Atomen, die jeweils mit einem oder mehreren Resten R² substituiert sein kann, bevorzugt aber unsubstituiert ist, wobei eine oder mehrere H-Atome durch D oder F ersetzt sein können, oder einem aromatischen Ringsystem mit 6 bis 24 aromatischen Ringatomen, das mit einem oder mehreren nicht-aromatischen Resten R² substituiert sein kann, bevorzugt aber unsubstituiert ist; dabei können die beiden Substituenten R¹ ein monocyclisches oder polycyclisches, aliphatisches oder aromatisches Ringsystem bilden, das mit einem oder mehreren Resten R² substituiert sein kann, bevorzugt aber unsubstituiert ist. Ganz besonders bevorzugt sind die Reste R¹ gleich oder verschieden bei jedem Auftreten ausgewählt aus Methyl, Phenyl oder zwei Phenylgruppen, die miteinander ein Ringsystem bilden und so ein Spirosystem aufspannen. Insbesondere bevorzugt sind beide Reste R¹ entweder Methyl, oder beide Reste R¹ sind Phenylgruppen, die miteinander ein Ringsystem bilden und so ein Spirobifluoren aufspannen.

In einer weiteren bevorzugten Ausführungsform der Erfindung ist R gleich oder verschieden bei jedem Auftreten ausgewählt aus der Gruppe bestehend aus H, D, F, CN, N(R²)₂, OR², einer geradkettigen Alkylgruppe mit 1 bis 10 C-Atomen oder einer verzweigten oder cyclischen Alkylgruppe mit 3 bis 10 C-Atomen oder einer Alkenylgruppe mit 2 bis 10 C-Atomen, die jeweils mit einem oder mehreren Resten R² substituiert sein kann, wobei eine oder mehrere nicht-benachbarte CH₂-Gruppen durch O ersetzt sein können und wobei ein oder mehrere H-Atome durch D oder F ersetzt sein können, oder einem aromatischen oder heteroaromatischen Ringsystem mit 5 bis 30 aromatischen Ringatomen, das jeweils mit einem oder mehreren Resten R² substituiert sein kann. Besonders bevorzugt ist R gleich oder verschieden bei jedem Auftreten ausgewählt aus der Gruppe bestehend aus H, N(R²)₂, einer geradkettigen Alkylgruppe mit 1 bis 6 C-Atomen, insbesondere mit 1 bis 4 C-Atomen, oder einer verzweigten oder cyclischen Alkylgruppe mit 3 bis 8 C-Atomen, insbesondere mit 3 bis 6 C-Atomen, die jeweils mit einem oder mehreren Resten R² substituiert sein kann, bevorzugt aber unsubstiutiert ist, oder einem aromatischen oder heteroaromatischen Ringsystem mit 5 bis 24 aromatischen Ringatomen, das jeweils mit einem oder mehreren Resten R² substituiert sein kann, bevorzugt aber unsubstituiert ist.

Wenn R für ein aromatisches oder heteroaromatisches Ringsystem steht, dann ist dieser Rest R bevorzugt gleich oder verschieden bei jedem Auftreten aus denselben Gruppen ausgewählt, wie oben als geeignete Gruppen für Ar¹ und Ar² angegeben.

Dabei haben in Verbindungen, die durch Vakuumverdampfung verarbeitet werden, die Alkylgruppen bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgruppen, insbesondere verzweigten Alkylgruppen, mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

Wenn die Verbindung der Formel (1) bzw. die bevorzugten Ausführungsformen als Matrixmaterial für einen phosphoreszierenden Emitter oder in einer Schicht, die direkt an eine phosphoreszierende Schicht angrenzt, verwendet werden, ist es weiterhin bevorzugt, wenn die Triplettenergie der Verbindung gleich hoch oder höher ist als die des phosphoreszierenden Emitters. Dies kann insbesondere für grün und blau phosphoreszierende Emitter, welche eine höhere Triplettenergie als rot phosphoreszierende Emitter aufweisen, dadurch erreicht werden, dass die erfindungsgemäße Verbindung keine kondensierten Aryl- bzw. Heteroarylgruppen enthält, in denen mehr als zwei Sechsringe direkt aneinander ankondensiert sind. Solche Verbindungen sind daher eine weitere bevorzugte Ausführungsform der Erfindung. Insbesondere ist es für diese Verwendung bevorzugt, dass die Reste R, R¹, R², Ar¹ und Ar² keine kondensierten Aryl- bzw. Heteroarylgruppen enthalten, in denen zwei oder mehr Sechsringe direkt aneinander ankondensiert sind. Optional können diese Gruppen auch Phenanthren oder Triphenylen enthalten, da diese Arylgruppen trotz der drei bzw. vier kondensierten Sechsringe eine ausreichend hohe Triplettenergie aufweisen.

Die oben genannten bevorzugten Ausführungsformen können beliebig miteinander kombiniert werden. In einer besonders bevorzugten Ausführungsform der Erfindung treten die oben genannten Bevorzugungen gleichzeitig auf.

Beispiele für geeignete Verbindungen gemäß den oben aufgeführten Ausführungsformen sind die in der folgenden Tabelle aufgeführten Verbindungen.

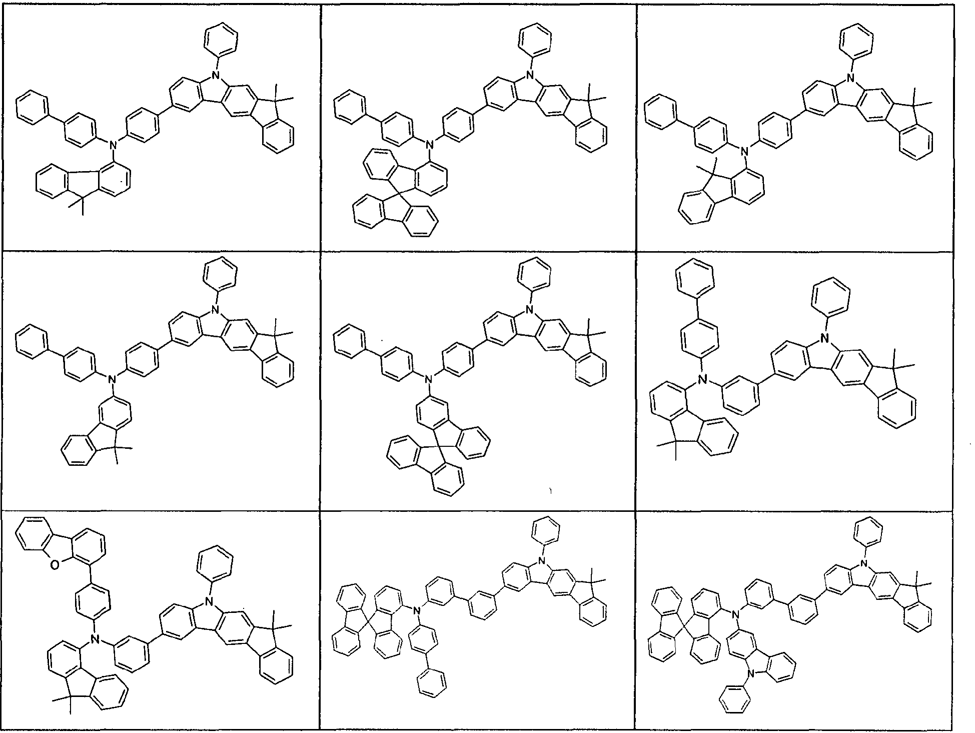

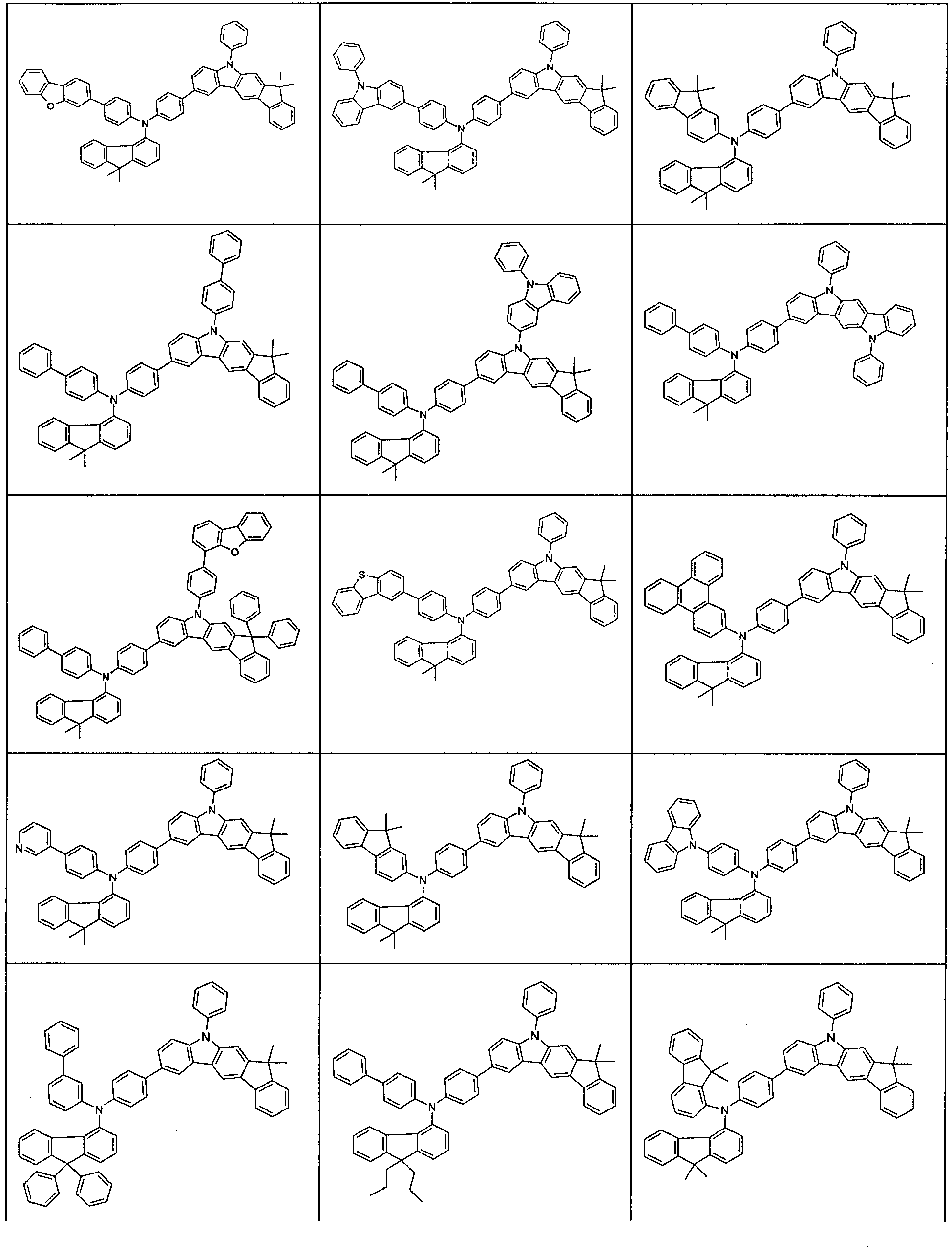

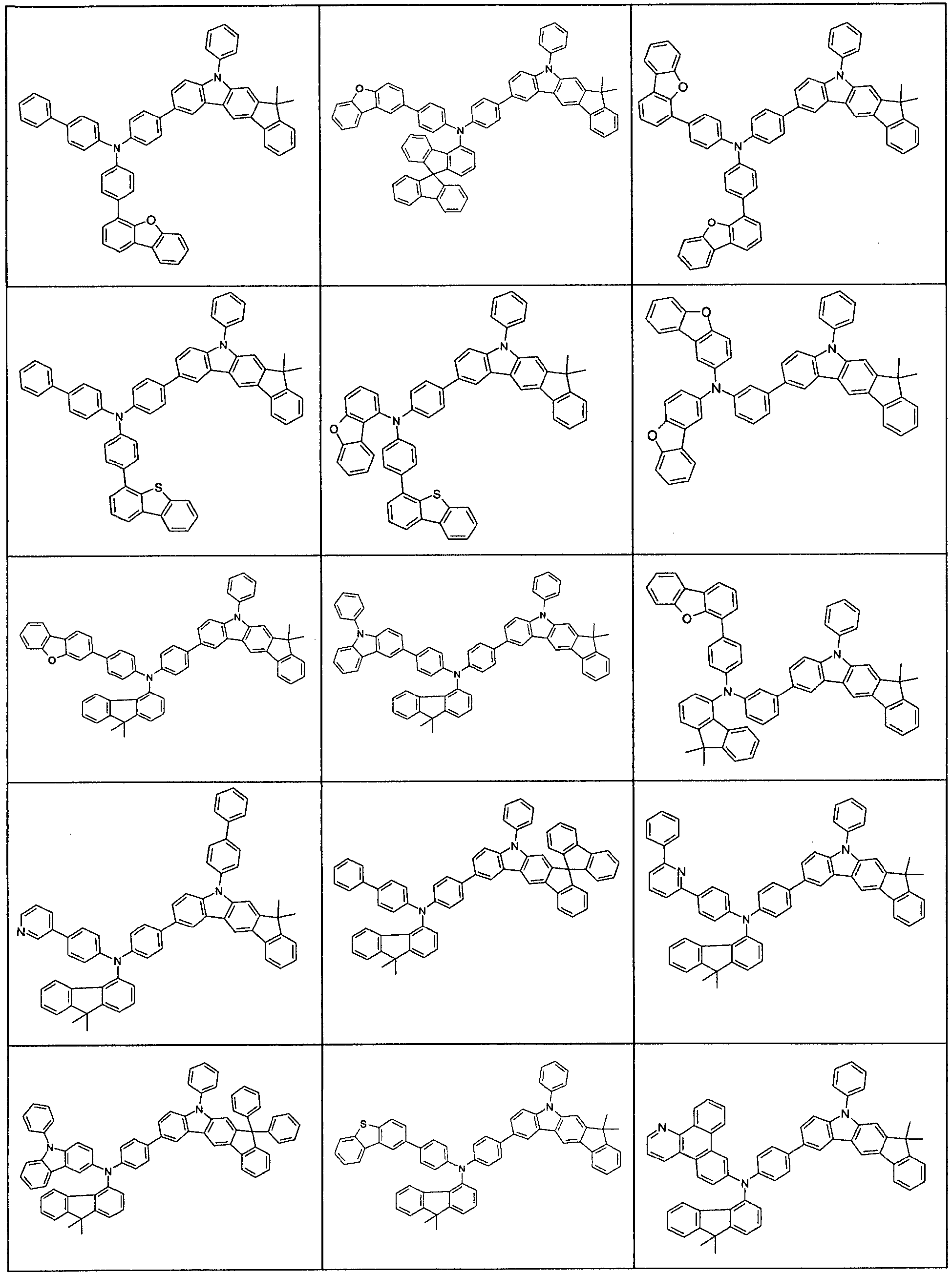

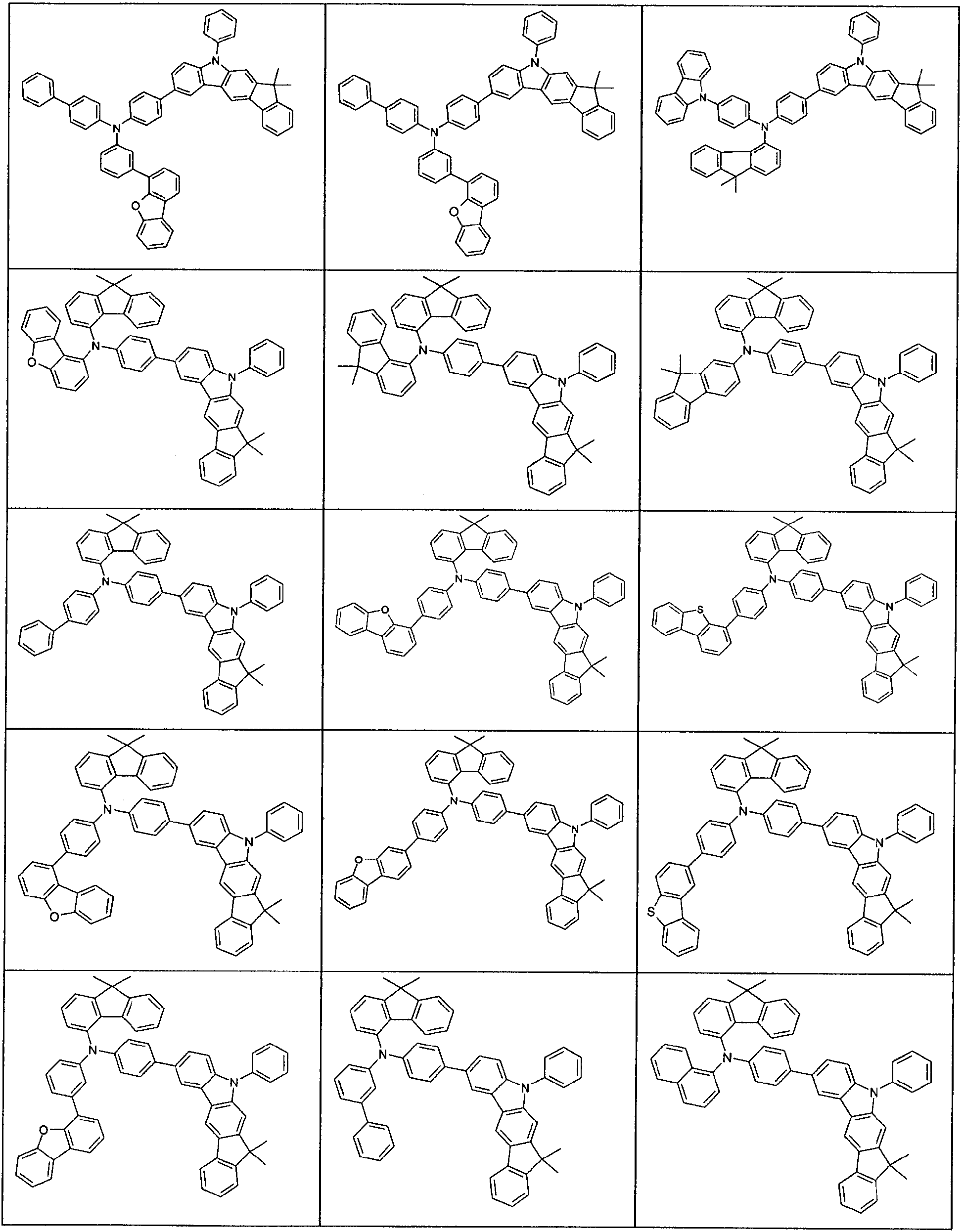

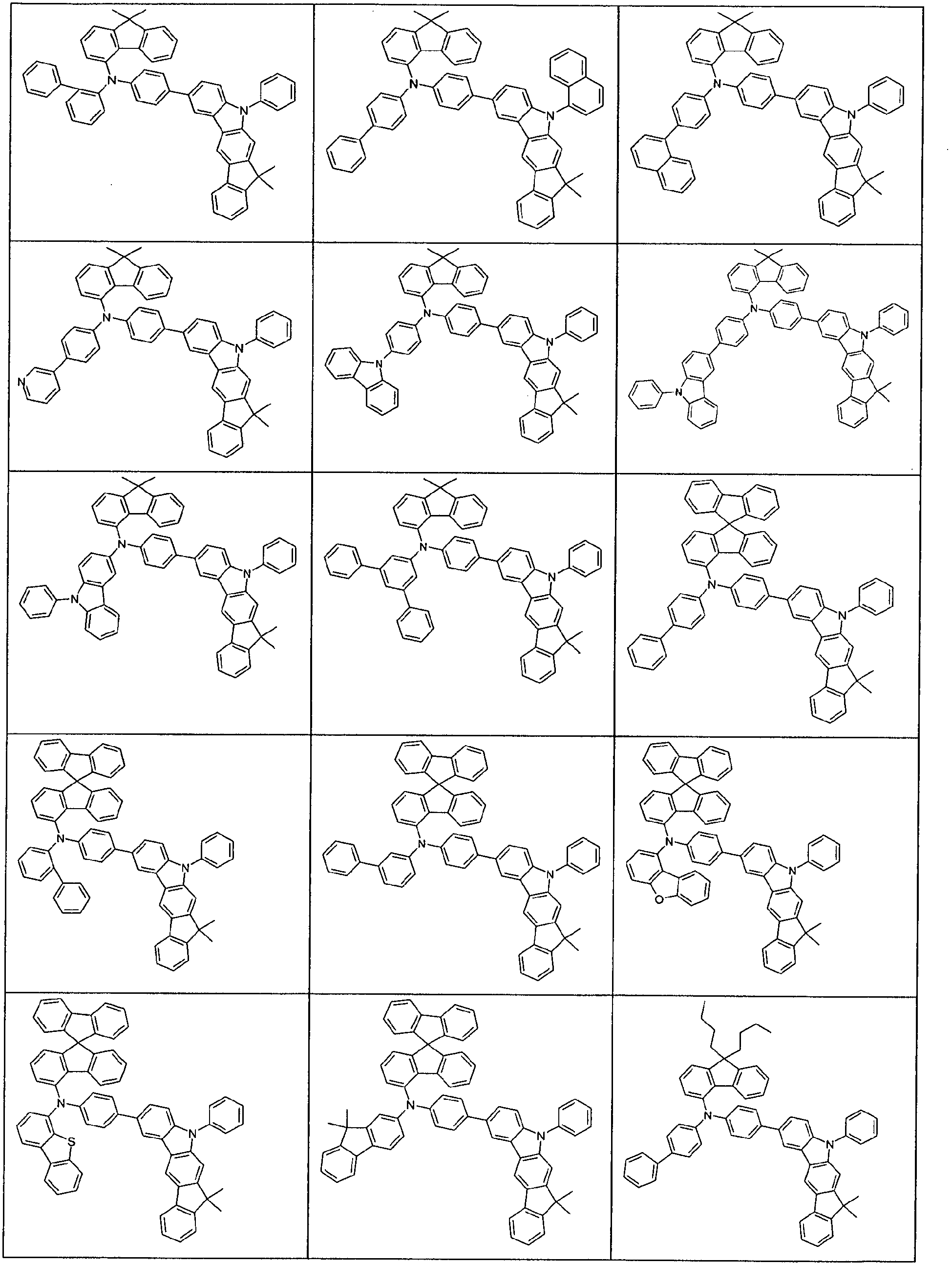

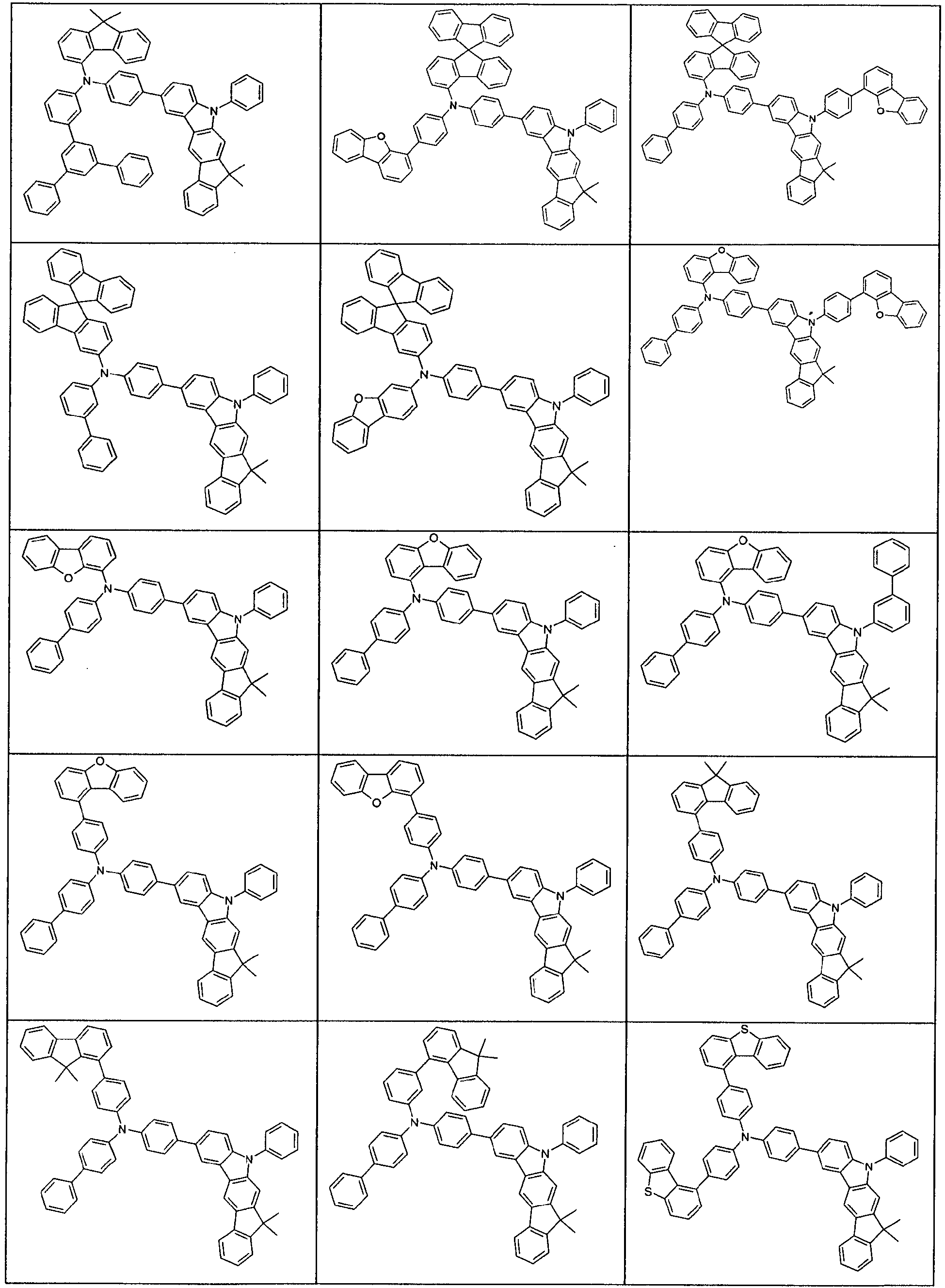

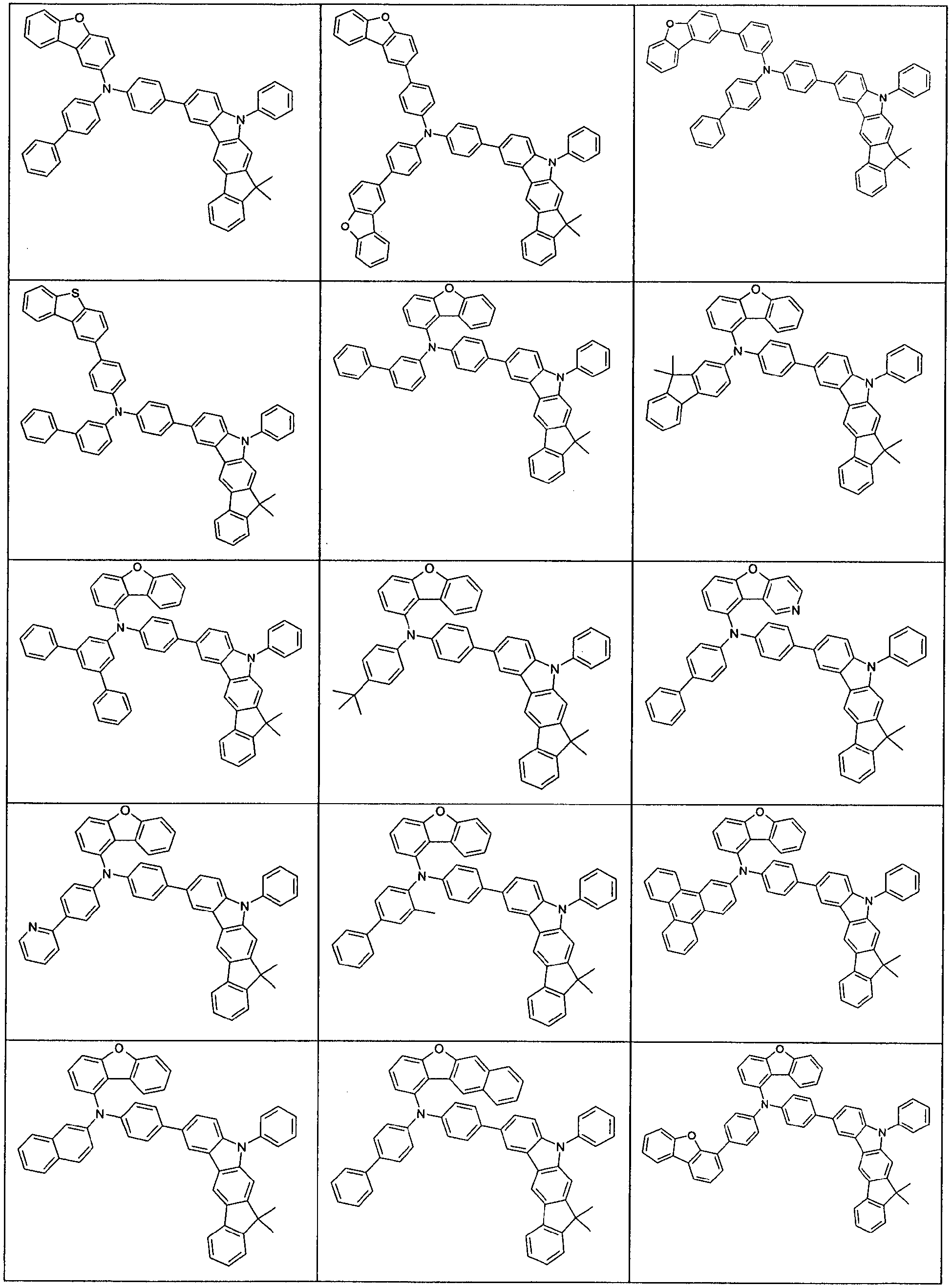

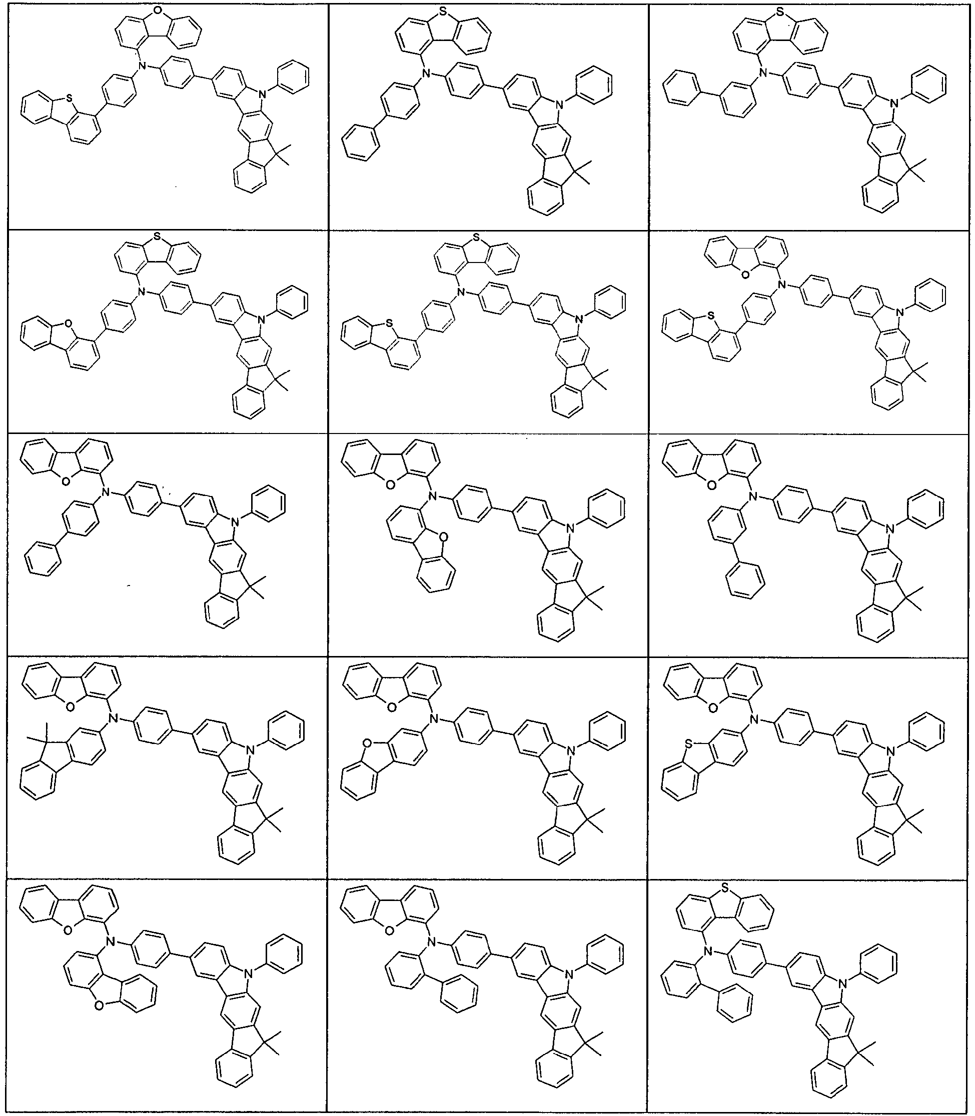

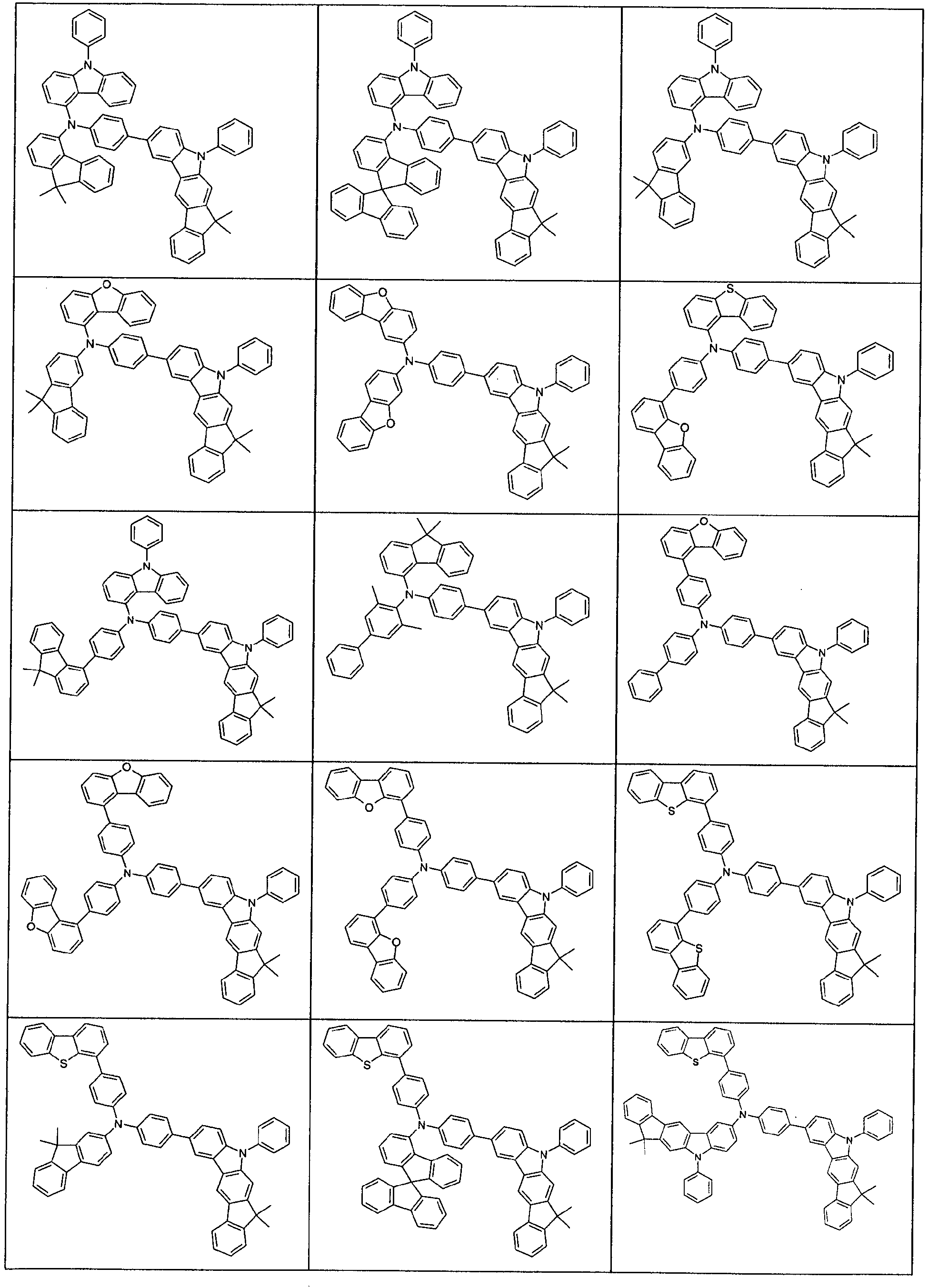

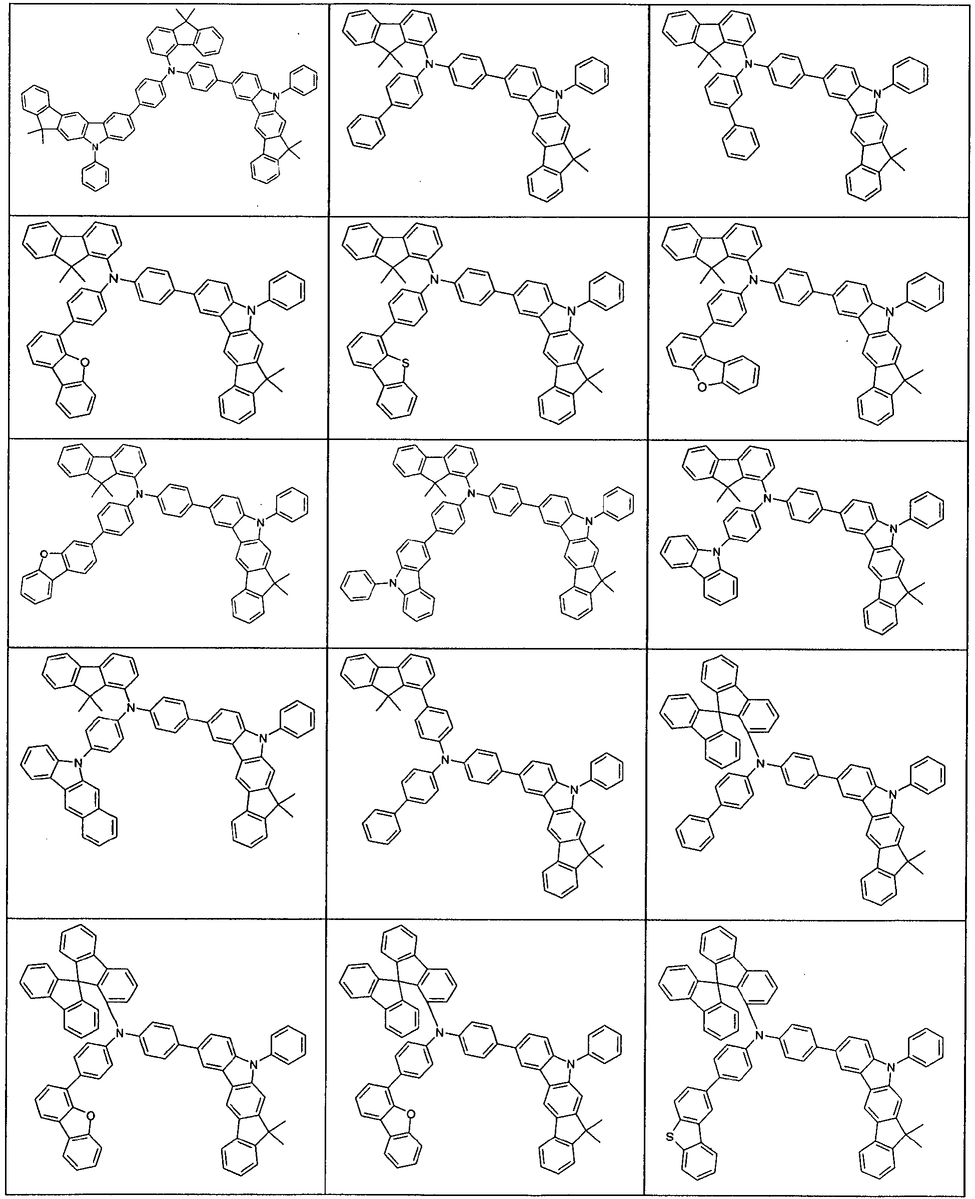

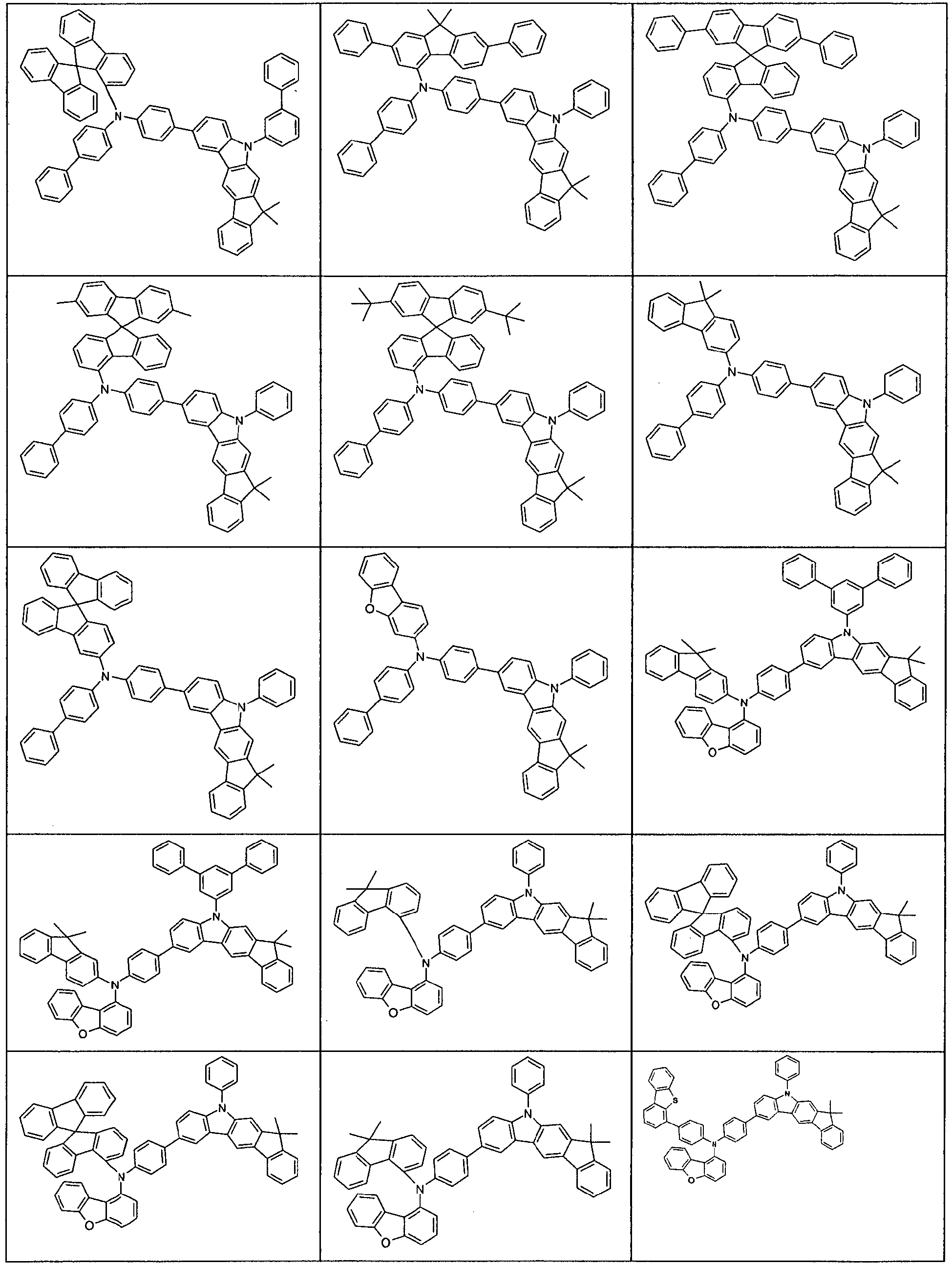

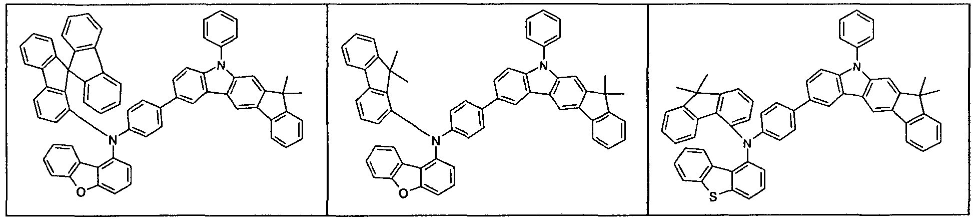

Die Synthese der erfindungsgemäßen Verbindungen kann auf dem im folgenden Schema allgemein skizzierten und nachfolgend beschriebenen Weg erfolgen:

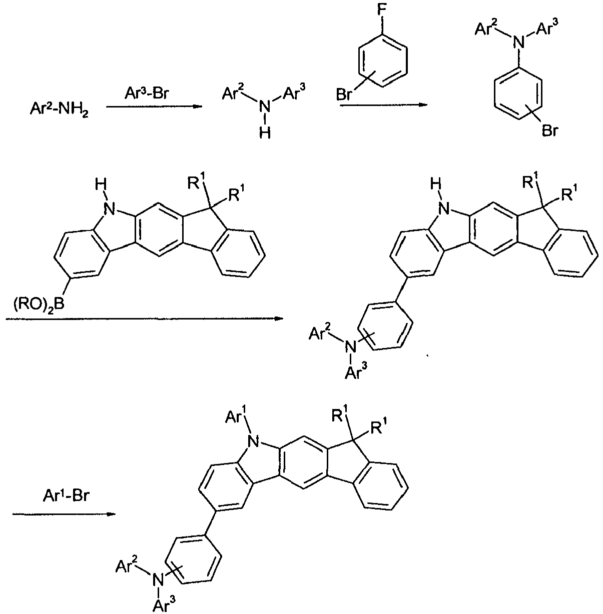

Ein primäres Amin Ar²-NH₂ wird in einer C-N-Kupplungsreaktion, beispielsweise einer Hartwig-Buchwald-Kupplung, mit einer Arylgruppe Ar³, welche eine reaktive Abgangsgruppe trägt, gekuppelt. Es folgt Reaktion mit einem Fluor-substituierten Benzol, welches eine weitere reaktive Abgangsgruppe trägt, gefolgt von einer C-C-Kupplungsreaktion, beispielsweise einer Suzuki-Kupplung, mit dem Indenocarbazol. Im letzten Schritt wird die Gruppe Ar¹ am Indenocarbazol durch eine C-N-Kupplungsreaktion, beispielsweise einer Hartwig-Buchwald-Kupplung, mit einer Arylgruppe Ar¹, welche eine reative Abgangsgruppe trägt, eingeführt. Als reaktive Abgangsgruppe eignen sich in den vorstehend beschriebenen Reaktionen beispielsweise Brom, Iod oder Tosylat. Weiterhin können zur Synthese entsprechend substituierter Verbindungen die entsprechend substituierten Edukte eingesetzt werden.

Ein weiterer Gegenstand der vorliegenden Erfindung ist daher ein Verfahren zur Synthese der erfindungsgemäßen Verbindungen, umfassend eine Kupplungsreaktion zwischen der Einheit Ar²Ar³N-Phenylen, welche mit einer reaktiven Abgangsgruppe substituiert ist, und einem Indenocarbazol, welches mit einer reaktiven Abgangsgruppe substituiert ist.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Hexamethylindan, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung und/oder ein weiteres Matrixmaterial. Geeignete emittierende Verbindungen und weitere Matrixmaterialien sind hinten im Zusammenhang mit der organischen Elektrolumineszenzvorrichtung aufgeführt. Diese weitere Verbindung kann auch polymer sein.

Die erfindungsgemäßen Verbindungen eignen sich für die Verwendung in einer elektronischen Vorrichtung, insbesondere in einer organischen Elektrolumineszenzvorrichtung.

Ein weiterer Gegenstand der vorliegenden Erfindung ist daher die Verwendung einer erfindungsgemäßen Verbindung in einer elektronischen Vorrichtung, insbesondere in einer organischen Elektrolumineszenzvorrichtung.

Ein nochmals weiterer Gegenstand der vorliegenden Erfindung ist eine elektronische Vorrichtung enthaltend mindestens eine erfindungsgemäße Verbindung.

Eine elektronische Vorrichtung im Sinne der vorliegenden Erfindung ist eine Vorrichtung, welche mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus organischen Elektrolumineszenzvorrichtungen (OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), farbstoffsensibilisierten organischen Solarzellen (DSSCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices", bevorzugt aber organischen Elektrolumineszenzvorrichtungen (OLEDs), besonders bevorzugt phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 2005/011013). Es kann sich bei der erfindungsgemäßen organischen Elektrolumineszenzvorrichtung auch um eine Tandem-OLED handeln, insbesondere auch für weiß emittierende OLEDs.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) bzw. den oben ausgeführten bevorzugten Ausführungsformen als Matrixmaterial für phosphoreszierende Emitter in einer emittierenden Schicht. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält. Bevorzugt ist weiterhin eine organische Elektrolumineszenzvorrichtung, enthaltend eine Verbindung gemäß Formel (1) bzw. den oben ausgeführten bevorzugten Ausführungsformen als Elektronenblockiermaterial in einer Elektronenblockierschicht oder als Lochtransportmaterial in einer Lochtransport- oder Lochinjektionsschicht.

Wenn die erfindungsgemäße Verbindung als Matrixmaterial für eine phosphoreszierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Die Mischung aus der erfindungsgemäßen Verbindung und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der erfindungsgemäßen Verbindung bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 30 Vol.-% des Emitters bezogen auf die Gesamtmischung aus Emitter und Matrixmaterial.

Eine weitere bevorzugte Ausführungsform der vorliegenden Erfindung ist der Einsatz der erfindungsgemäßen Verbindung als Matrixmaterial für einen phosphoreszierenden Emitter in Kombination mit einem weiteren Matrixmaterial. Wenn die erfindungsgemäße Verbindung in Kombination mit einem weiteren Matrixmaterial eingesetzt wird, liegt deren Anteil bevorzugt bei 20 bis 50 Vol.-%, bezogen auf die Gesamtmischung. Dabei ist das weitere Matrixmaterial bevorzugt ein elektronentransportierendes Material. Geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527, WO 2008/086851 oder WO 2013/041176, Indolocarbazolderivate, z. B. gemäß WO 2007/063754 oder WO 2008/056746, Indenocarbazolderivate, z. B. gemäß WO 2010/136109, WO 2011/000455, WO 2013/041176 oder WO 2013/056776, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte Carbazol-Derivate, z. B. gemäß WO 2011/042107, WO 2011/060867, WO 2011/088877 und WO 2012/143080, oder Triphenylenderivate, z. B. gemäß WO 2012/048781. Ebenso kann ein weiterer phosphoreszierender Emitter, welcher kürzerwellig als der eigentliche Emitter emittiert, als Co-Host in der Mischung vorhanden sein oder eine Verbindung, die nicht oder nicht in wesentlichem Umfang am Ladungstransport teilnimmt, wie beispielsweise in WO 2010/108579 beschrieben.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 2001/41512, WO 2002/02714, WO 2002/15645, EP 1191613, EP 1191612, EP 1191614, WO 2005/033244, WO 2005/019373, US 2005/0258742, WO 2010/086089, WO 2011/044988, WO 2011/157339, WO 2012/007086, WO 2012/163471, WO 2013/000531 und WO 2013/020631, WO 2014/008982, WO 2014/023377, WO 2014/094962, WO 2014/094961, WO 2014/094960, WO 2015/039723, WO 2015/104045, WO 2015/117718 und WO 2016/015815 entnommen werden. Weiterhin eignen sich beispielsweise die in den nicht offen gelegten Anmeldung EP 15000307.7 offenbarten Metallkomplexe. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Die erfindungsgemäßen Verbindungen sind insbesondere auch geeignet als Matrixmaterialien für phosphoreszierende Emitter in organischen Elektrolumineszenzvorrichtungen, wie sie z. B. in WO 98/24271, US 2011/0248247 und US 2012/0223633 beschrieben sind. In diesen mehrfarbigen Display-Bauteilen wird eine zusätzliche blaue Emissionsschicht vollflächig auf alle Pixel, auch diejenigen mit einer von Blau verschiedenen Farbe, aufgedampft.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. den oben ausgeführten bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92,053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen, eingesetzt in OLEDs als Matrixmaterial für phosphoreszierende Emitter oder als Elektronenblockiermaterial in einer Elektronenblockierschicht, führen zu verbesserten Lebensdauern im Vergleich zu den analogen Matrixmaterialien gemäß dem Stand der Technik, die statt einer Gruppe der Formel (2) ein anderes aromatisches Ringsystem enthalten. Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

### Synthesebeispiele

Die nachfolgenden Synthesen werden, sofern nicht anders angegeben, unter einer Schutzgasatmosphäre in getrockneten Lösungsmitteln durchgeführt. Die Lösungsmittel und Reagenzien können von ALDRICH bzw. ABCR bezogen werden. Die bei den nicht kommerziell erhältlichen Edukten angegeben Nummern sind die entsprechenden CAS-Nummern.

Die Synthese der erfindungsgemäßen Verbindungen ist im folgenden Schema exemplarisch für eine Beispielstruktur angegeben. Derivate mit anderen Gruppen Ar¹, Ar², Ar², R und R¹ werden entsprechend synthetisiert.

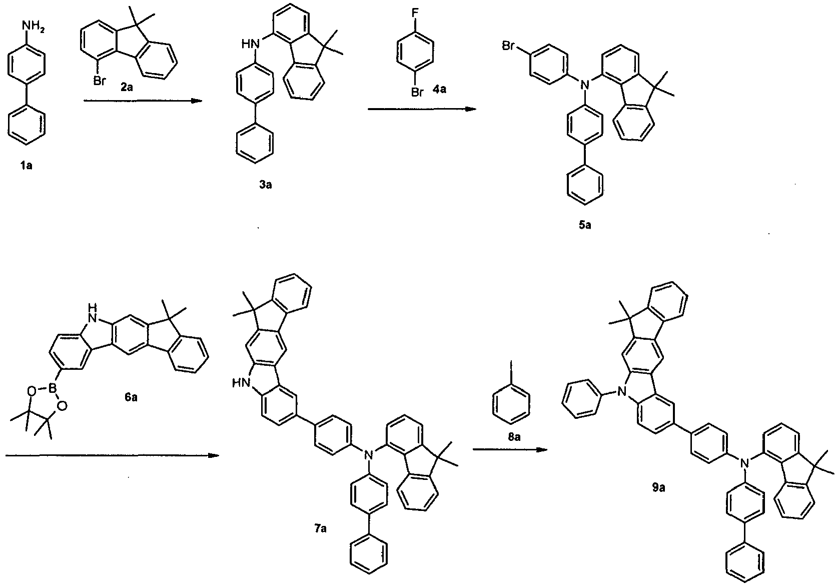

### Stufe 1: Darstellung von Biphenyl-4-yl-(9,9-dimethyl-9H-fluoren-4-yl)-amin 3a

In einem 2L-Vierhalskolben werden 30.0g (177mmol, 1.0eq) 4-Aminobiphenyl [92-67-1] **1a** zusammen mit 48.4g (177mmol, 1.0eq) 4-Bromo-9,9-dimethyl-9H-fluoren [942615-32-9] **2a** und 23.4g (212mmol, 1.20eq) Natrium-*t*-pentoxid [14593-46-5] in 600ml absolutem Toluol vorgelegt und für 30 Minuten entgast. Anschließend werden 398mg (1.77mmol, 0.01eq) Palladium(II)-acetat [3375-31-3] und 1.46g (3.56mmol, 0.02eq) 2-Dicyclo-hexylphosphino-2',6'-dimethoxybiphenyl SPHOS [657408-07-6] zugegeben und der Ansatz über Nacht unter Rückfluss erhitzt. Nach beendeter Reaktion wird der Ansatz auf Raumtemperatur abgekühlt und mit 500ml Wasser extrahiert. Anschließend wird die wässrige Phase dreifach mit Toluol gewaschen, die vereinten organischen Phasen über Natriumsulfat getrocknet und das Lösungsmittel am Rotationsverdampfer entfernt. Der braune Rückstand wird in ca. 200ml Toluol aufgenommen und über Silikagel filtriert. Zur weiteren Aufreinigung wird eine Umkristallisation aus Toluol/Heptan durchgeführt. Es werden 51.3g (142mmol, 80%) des Amins **3a** erhalten.

**Analog werden dargestellt:**

| | **Edukt 1** | **Edukt 2** | **Produkt 3** | **Ausbeute [%]** |
|---|---|---|---|---|
| **3b** | 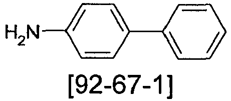 | 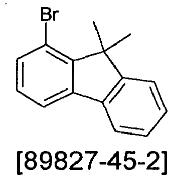 | 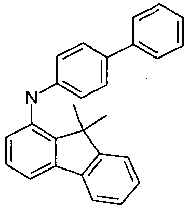 | 45 |
| **3c** | 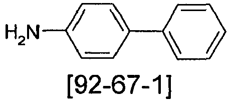 | 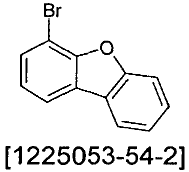 | 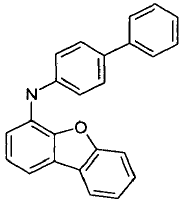 | 61 |
| **3d** | 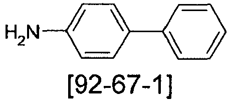 | 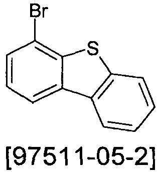 | 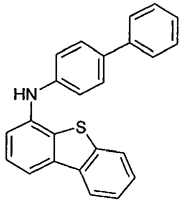 | 68 |
| **3e** | 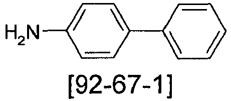 | 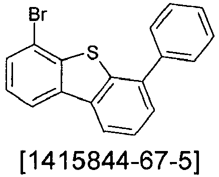 | 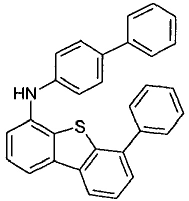 | 34 |
| **3f** | 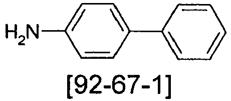 | 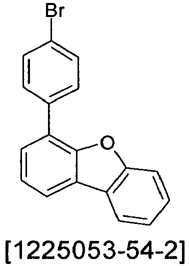 | 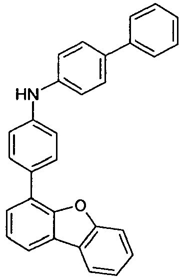 | 78 |
| **3g** | 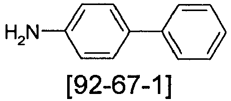 | 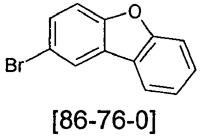 | 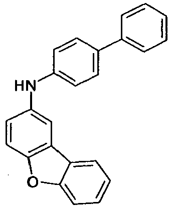 | 51 |
| **3h** | 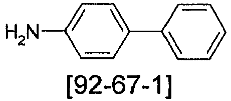 | 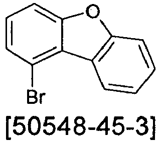 | 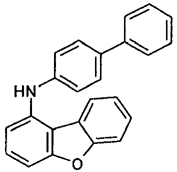 | 65 |
| **3i** | 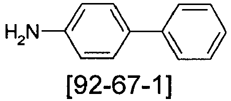 | 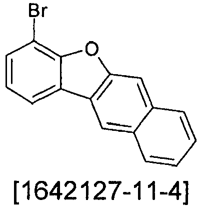 | 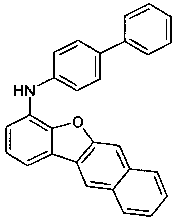 | 81 |
| **3j** | 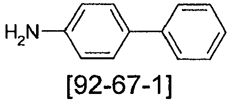 | 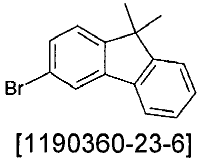 | 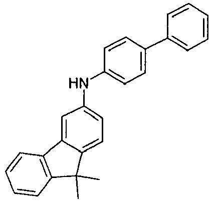 | 33 |
| **3k** | 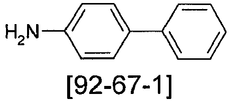 | 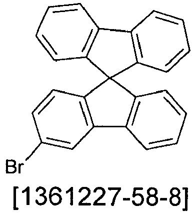 | 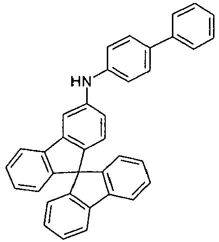 | 63 |
| **3l** | 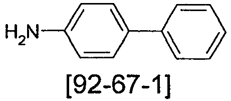 | 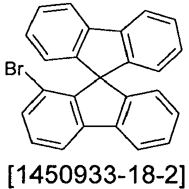 | 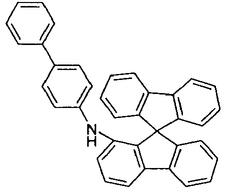 | 23 |
| **3m** | 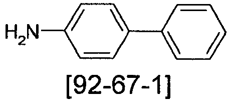 | 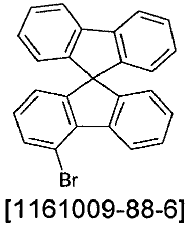 | 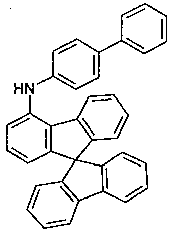 | 55 |
| **3n** | 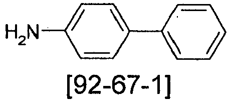 | 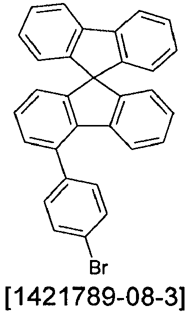 | 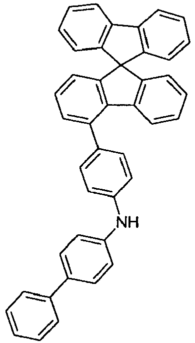 | 68 |
| **30** | 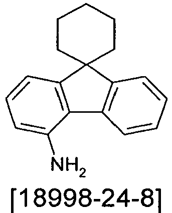 | 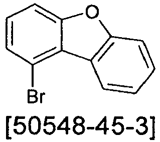 | 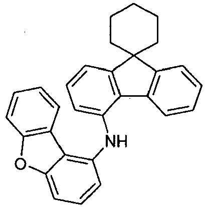 | 43 |
| **3p** | 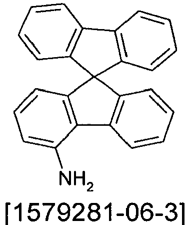 | 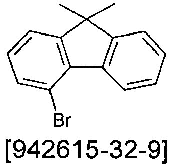 | 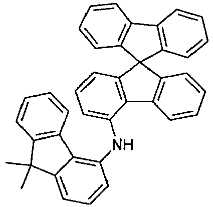 | 29 |
| **3q** | 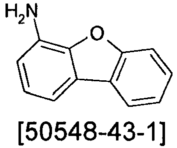 | 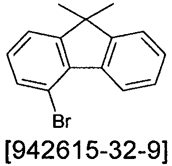 | 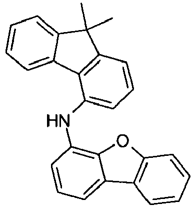 | 34 |
| **3r** | 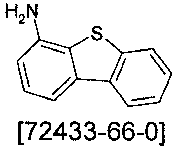 | 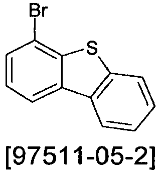 | 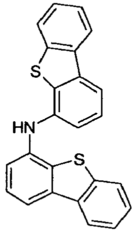 | 55 |
| **3s** | 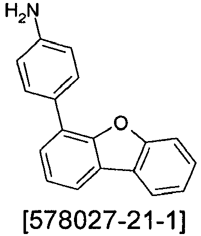 | 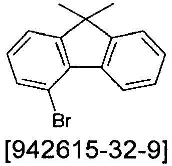 | 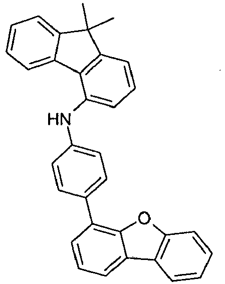 | 62 |
| **3t** | 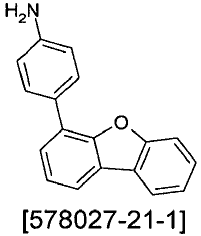 | 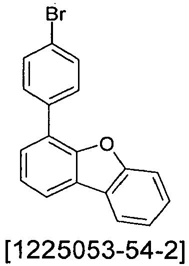 | 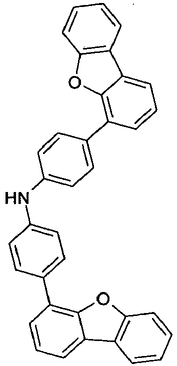 | 72 |
| **3u** | 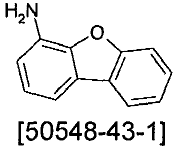 | 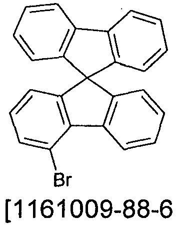 | 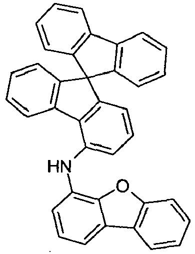 | 45 |

### Stufe 2: Darstellung von (4-Bromo-phenyl)-(9,9-dimethyl-9H-fluoren-4-yl)-{4-[(E)-((Z)-1-propenyl)-buta-1,3-dienyl]-phenyl}-amin 5a

In einem 1L-Vierhalskolben werden 51.3g (142mmol, 1.00eq) des Amins **3a,** sowie 75.6g (426mmol, 3.00eq) 1-Bromo-4-fluorobenzol [460-00-4] **4a** und 92.5g (284mmol, 2.00eq) Cäsiumcarbonat [534-17-8] vorgelegt und mit 500ml Dimethylacetamid versetzt. Das Reaktionsgemisch wird für drei Tage bei 150°C gerührt. Nach beendeter Reaktion wird der Ansatz auf Raumtemperatur abgekühlt und der Feststoff über Celite abfiltriert. Die Mutterlauge wird eingeengt und der ausgefallene Feststoff nach Filtration mit heißem Methanol ausgerührt. Nach Trocknung werden 43.5g (135mmol, 95%) des farblosen Produktes **5a** erhalten.

**Analog werden dargestellt:**

| | **Edukt 3** | **Produkt 5** | **Ausbeute [%]** |
|---|---|---|---|
| **5b** | 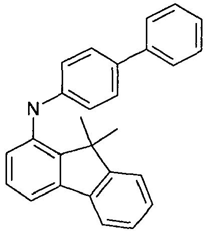 | 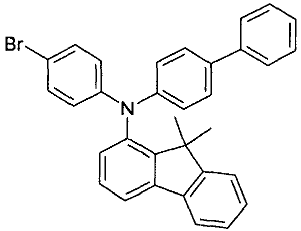 | 78 |
| **5c** | 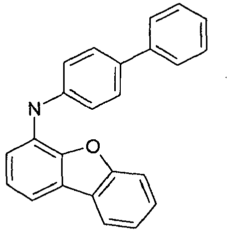 | 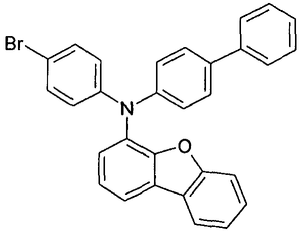 | 81 |
| **5d** | 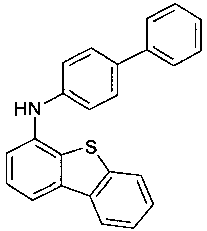 | 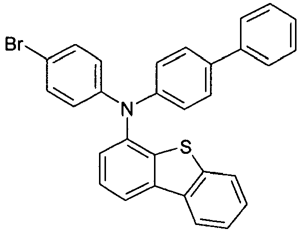 | 94 |
| **5e** | 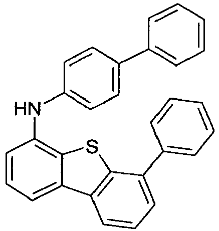 | 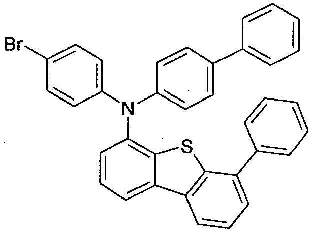 | 92 |
| **5f** | 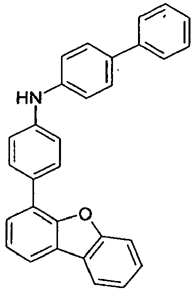 | 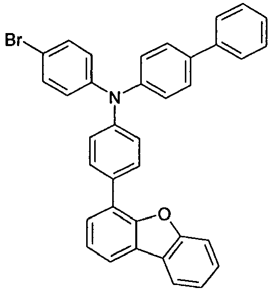 | 71 |
| **5g** | 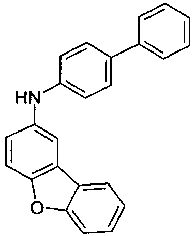 | 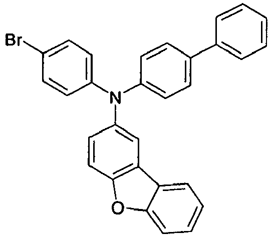 | 88 |
| **5h** | 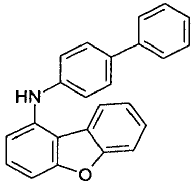 | 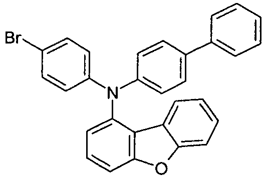 | 65 |
| **5i** | 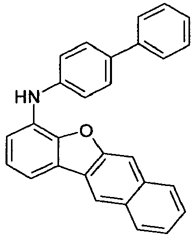 | 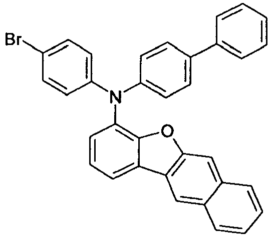 | 79 |
| **5j** | 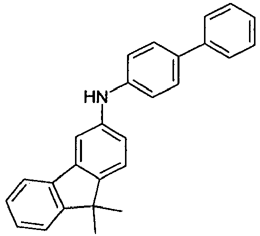 | 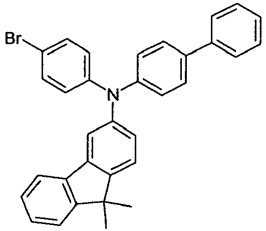 | 36 |
| **5k** | 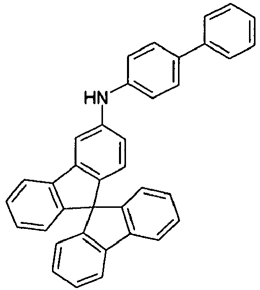 | 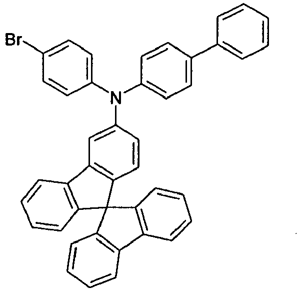 | 91 |
| **5l** | 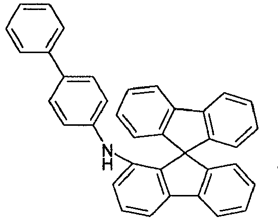 | 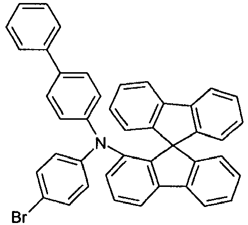 | 26 |
| **5m** | 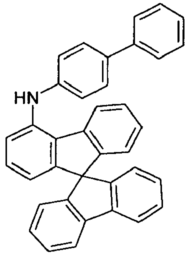 | 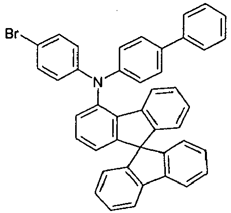 | 84 |
| **5n** | 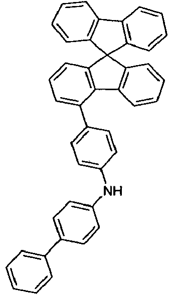 | 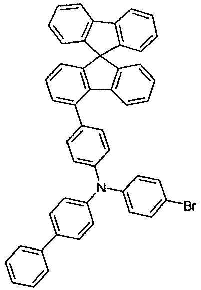 | 68 |
| **5o** | 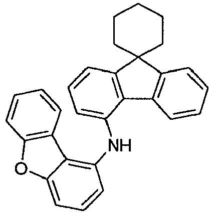 | 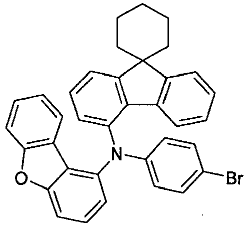 | 52 |
| **5p** | 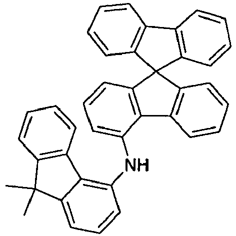 | 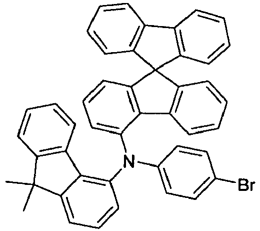 | 48 |
| **5q** | 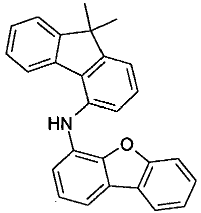 | 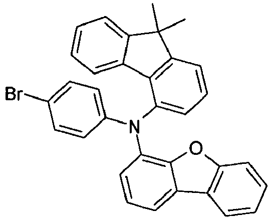 | 67 |
| **5r** | 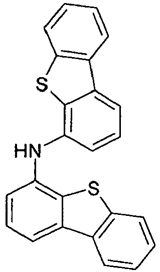 | 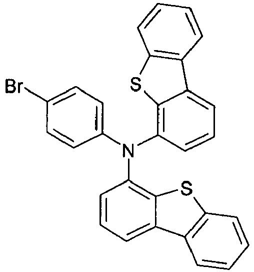 | 56 |
| **5s** | 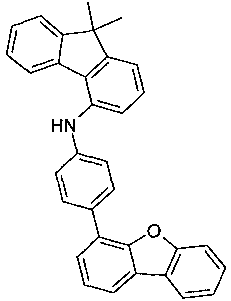 | 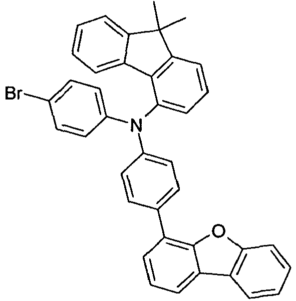 | 44 |
| **5t** | 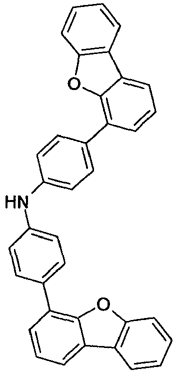 | 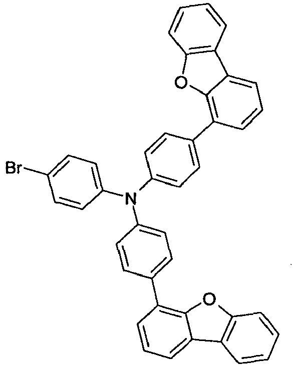 | 21 |
| **5u** | 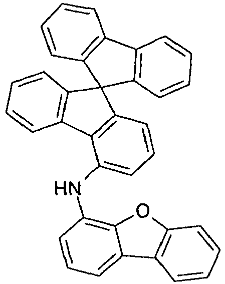 | 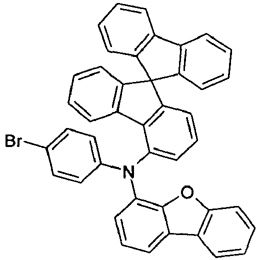 | 49 |

### Stufe 3: Darstellung von Biphenyl-4-yl-[4-(12,12-dimethyl-10,12-dihydro-10-aza-indeno[2,1-b]fluoren-7-yl)-phenyl]-(9,9-dimethyl-9H-fluoren-4-yl)-amin 7a

In einem 2L-Vierhalskolben werden 43.5g (135mmol, 1.00eq) des Amins **5a** zusammen mit 61.0g (149mmol, 1.10eq) 5,7-Dihydro-7,7-dimethyl-2-(44,55-tetramethyl-1,3,2-dioxaborolan-2-yl)-indeno[2,1-*b*]carbazol [1357286-77-1] und 53.9g (270mmol, 2.00eq) Trikaliumphosphat [7778-53-2] in jeweils 300ml Toluol und Dioxan sowie 150ml VE-Waser vorgelegt und für 30 Minuten entgast. Anschließend werden 1.21g (5.40mmol, 0.04eq) Palladium(II)-acetat [3375-31-3] und 2.47g (8.10mmol, 0.06eq) Tri-o-tolylphosphin [6163-58-2] zugegeben und der Ansatz über Nacht unter Rückfluss erhitzt. Nach beendeter Reaktion werden weitere 100ml Wasser zugegeben und die wässrige Phase abgetrennt. Das wässrige Extrakt wird dreimal mit Toluol extrahiert, die vereinten organischen Phasen über Natriumsulfat getrocknet und das Lösungsmittel am Rotationsverdampfer entfernt. Nach Umkristallisation aus Toluol/Heptan werden 81.2g (113mmol, 84%) des gewünschten Produktes **7a** erhalten.

**Analog werden dargestellt:**

| | **Edukt 5** | **Produkt 7** | **Ausbeute [%]** |
|---|---|---|---|
| **7b** | 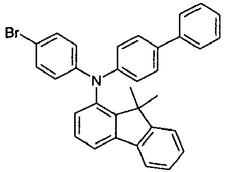 | 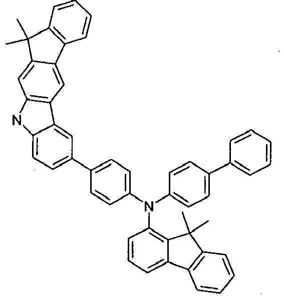 | 67 |
| **7c** | 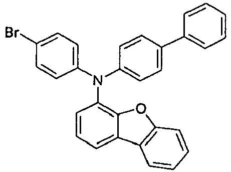 | 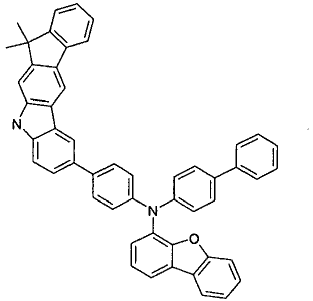 | 81 |
| **7d** | 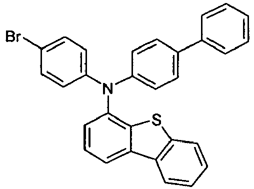 | 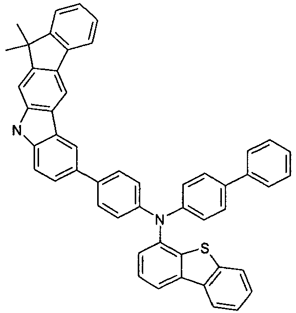 | 89 |
| **7e** | 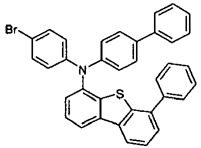 | 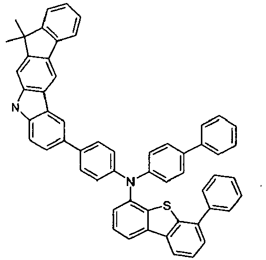 | 77 |
| **7f** | 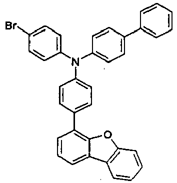 | 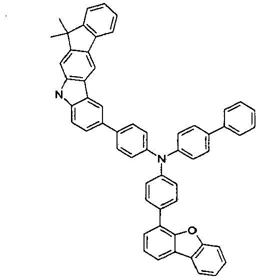 | 67 |
| **7g** | 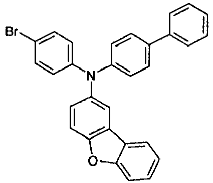 | 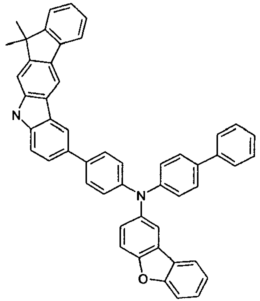 | 45 |
| **7h** | 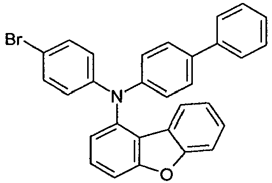 | 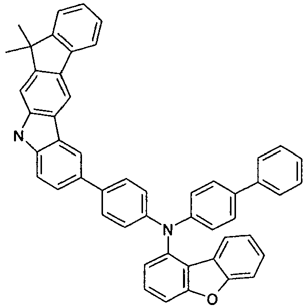 | 91 |
| **7i** | 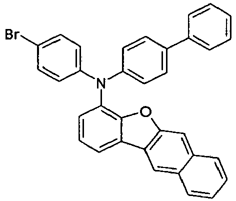 | 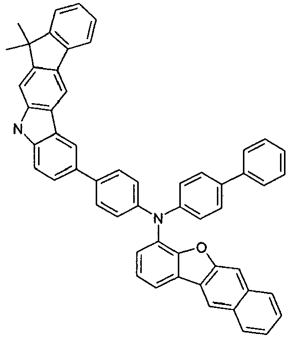 | 74 |
| **7j** | 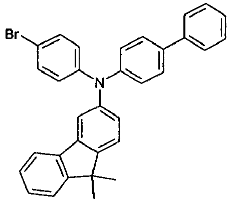 | 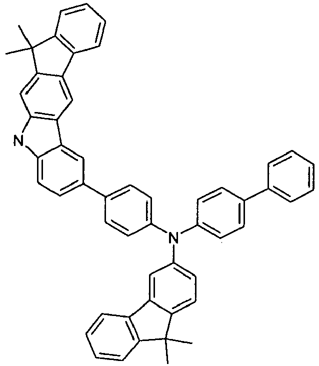 | 66 |
| **7k** | 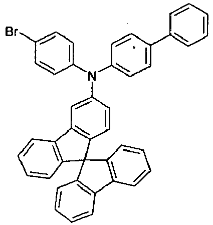 | 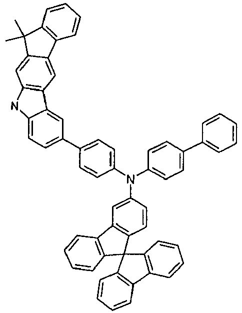 | 58 |
| **7l** | 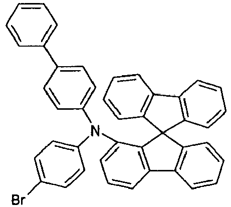 | 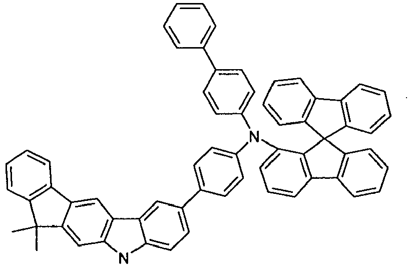 | 31 |
| **7m** | 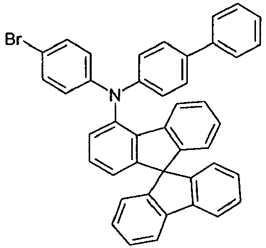 | 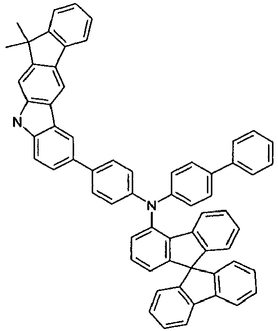 | 63 |
| **7n** | 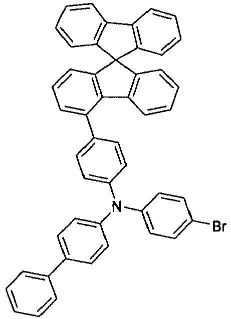 | 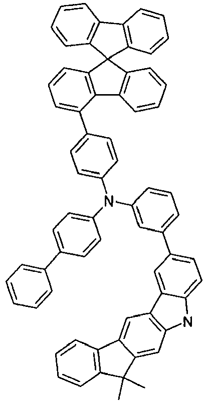 | 54 |
| **7o** | 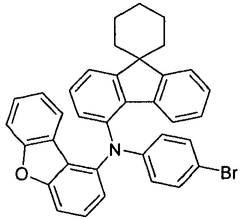 | 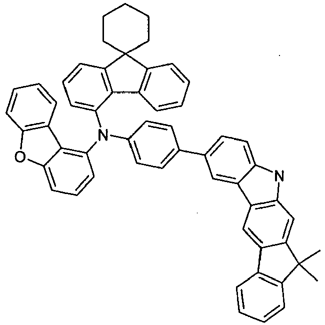 | 87 |
| **7p** | 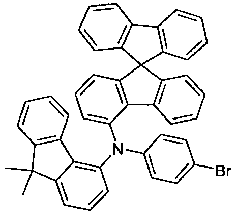 | 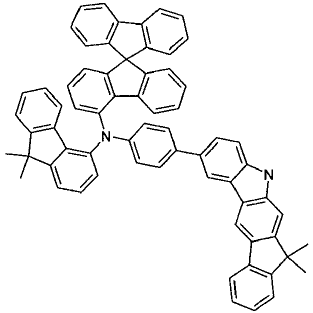 | 81 |
| **7q** | 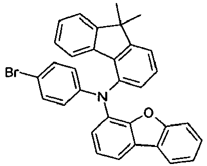 | 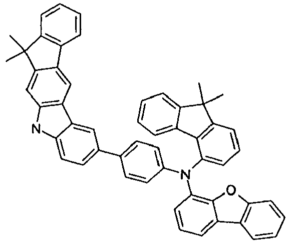 | 73 |
| **7r** | 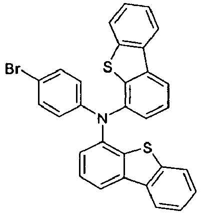 | 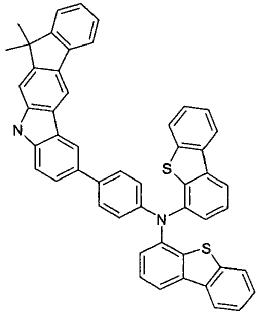 | 64 |
| **7s** | 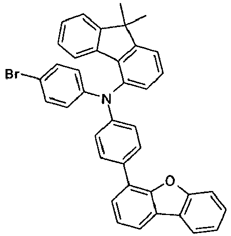 | 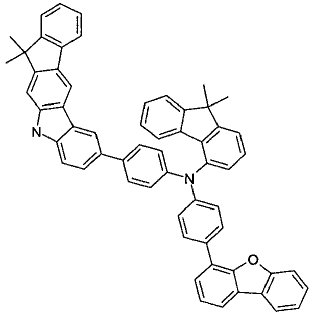 | 78 |
| **7t** | 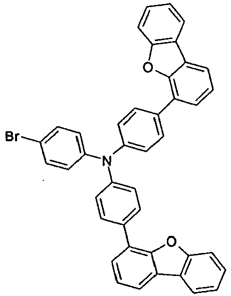 | 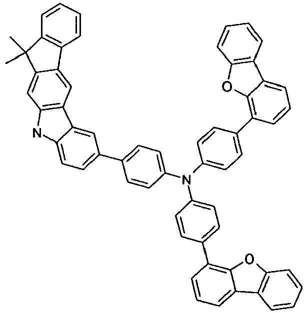 | 93 |
| **7u** | 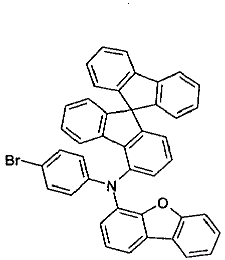 | 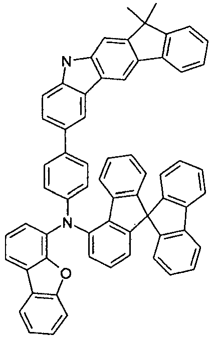 | 68 |

### Stufe 4: Darstellung von Biphenyl-4-yl-(9,9-dimethyl-9H-fluoren-4-yl)-[4-(12,12-dimethyl-10-phenyl-10,12-dihydro-10-aza-indeno[2,1-b]fluoren-7-yl)-phenyl]-amin 9a

In einem 1L-Vierhalskolben werden 20.0g (25.2mmol, 1.00eq) des Zwischenproduktes **7a** zusammen mit 11.3g (55.4mmol, 2.20eq) Iodbenzol **8a** [591-50-4] und 14.9g (101 mmol, 4.00eq) Kaliumcarbonat [584-08-7] in 300ml absolutem DMF vorgelegt und für 30 Minuten entgast. Anschließend werden 570mg (2.52mmol, 0.10eq) 1,3-Di(2-pyridyl)-1,3-propandion [10198-89-7] und 480mg (2.52mmol, 0.10eq) Kupfer(I)-iodid [7681-65-4] zugegeben und der Ansatz für einen Tag unter Rückfluss erhitzt. Nach beendeter Reaktion wird das Reaktionsgemisch abgekühlt und langsam in 1000ml Wasser überführt. Der ausgefallene Feststoff wird abgesaugt und mit je 400ml gesättigter Ammoniumchlorid-Lösung, Wasser sowie je 200ml Ethanol und Heptan gewaschen. Zur Aufreinigung wird der Feststoff mit Toluol/Heptan heiß extrahiert, zweimal aus Toluol/ Heptan umkristallisiert und abschließend mittels Sublimation aufgereinigt. Es werden 11.2g (14.1mmol, 56%) der gewünschten Zielverbindung **9a** mit einer HPLC-Reinheit >99.9% erhalten.

**Analog werden dargestellt:**

| | **Edukt 7** | **Edukt 8** | **Produkt 9** | **Ausbeute [%]** |
|---|---|---|---|---|
| **9b** | 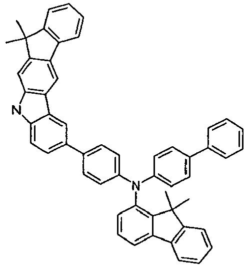 | 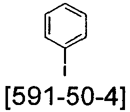 | 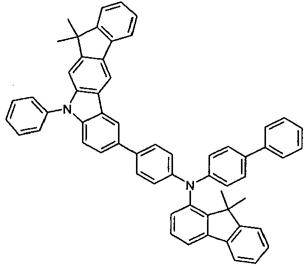 | 34 |
| **9c** | 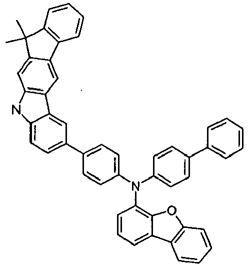 | 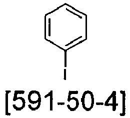 | 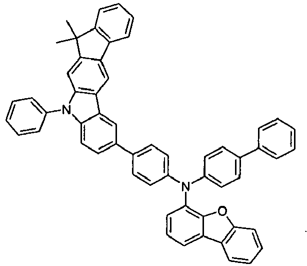 | 51 |
| **9d** | 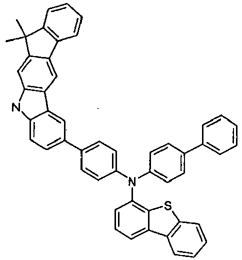 | 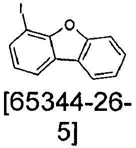 | 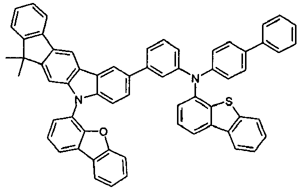 | 23 |
| **9e** | 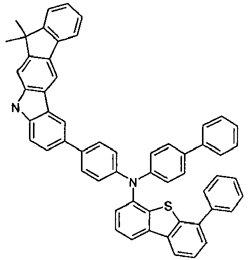 | 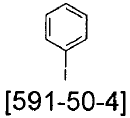 | 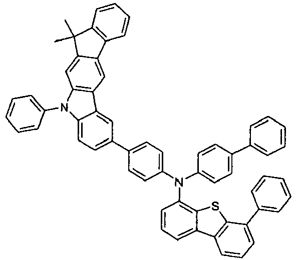 | 37 |
| **9f** | 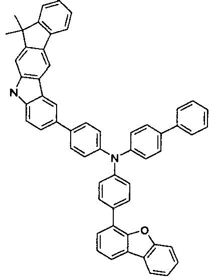 | 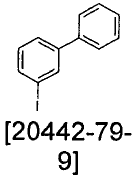 | 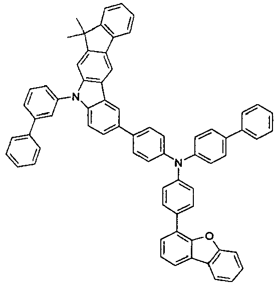 | 14 |
| **9g** | 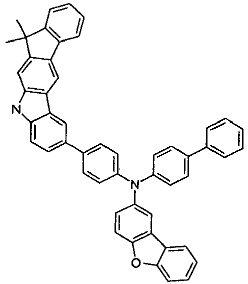 | 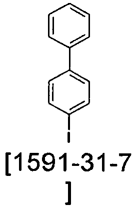 | 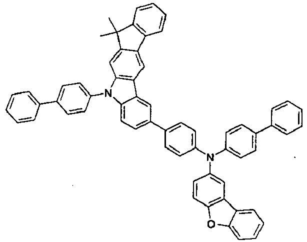 | 65 |
| **9h** | 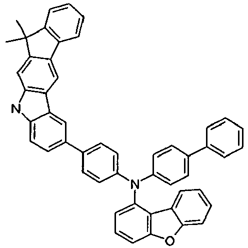 | 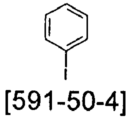 | 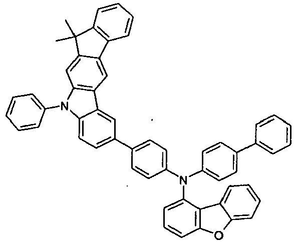 | 47 |
| **9i** | 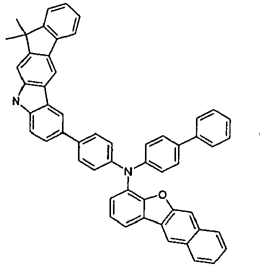 | 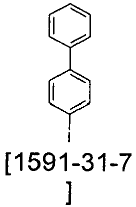 | 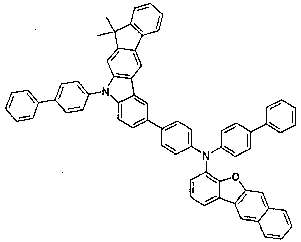 | 34 |
| **9j** | 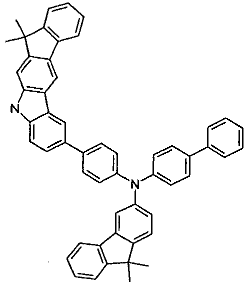 | 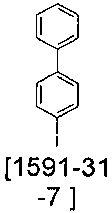 | 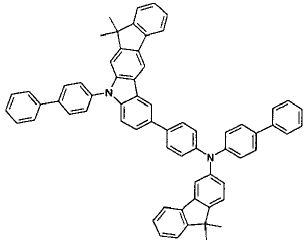 | 52 |
| **9k** | 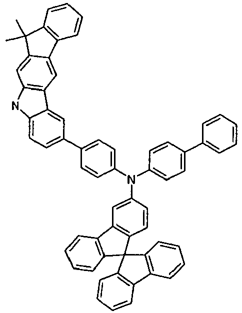 | 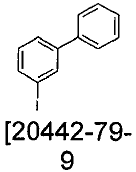 | 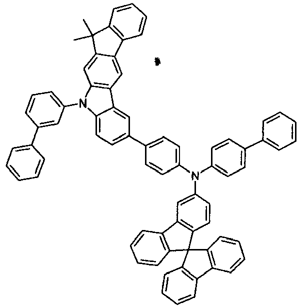 | 13 |
| **9l** | 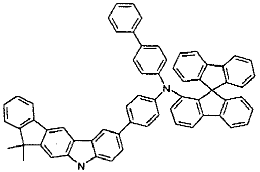 | 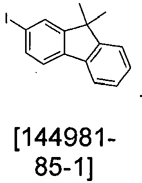 | 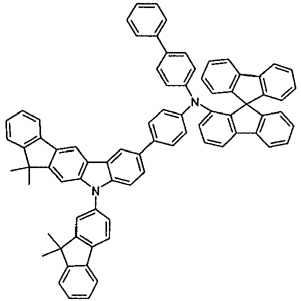 | 46 |
| **9m** | 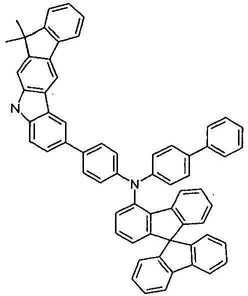 | 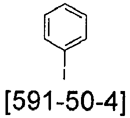 | 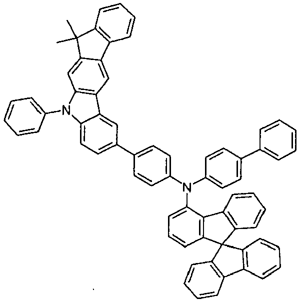 | 61 |
| **9n** | 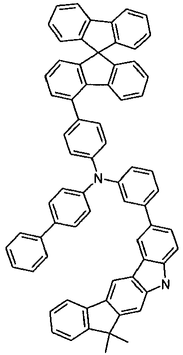 | 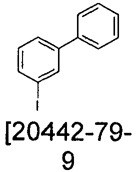 | 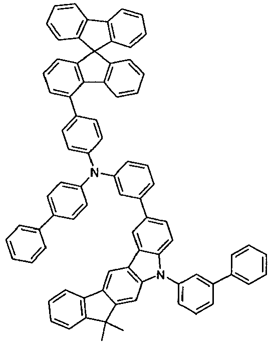 | 49 |
| **9o** | 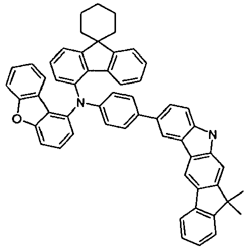 | 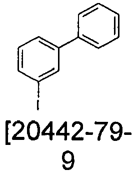 | 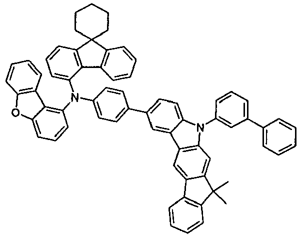 | 19 |
| **9p** | 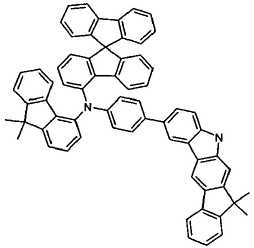 | 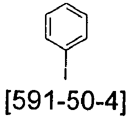 | 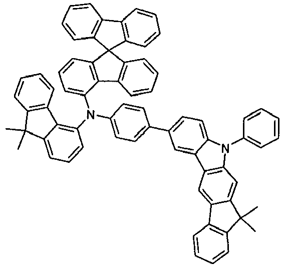 | 33 |
| **9q** | 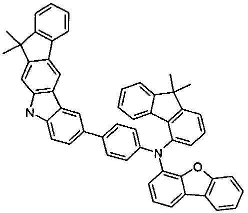 | 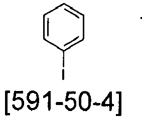 | 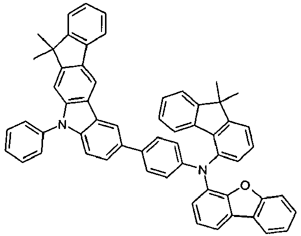 | 23 |
| **9r** | 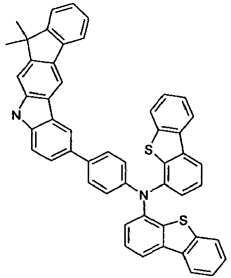 | 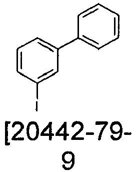 | 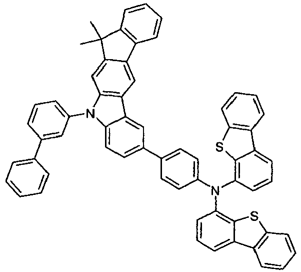 | 21 |
| **9s** | 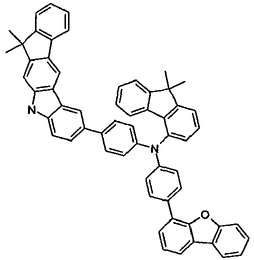 | 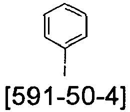 | 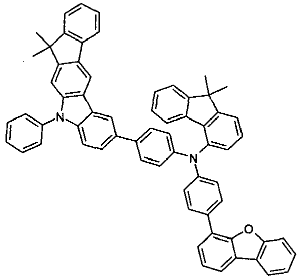 | 55 |
| **9t** | 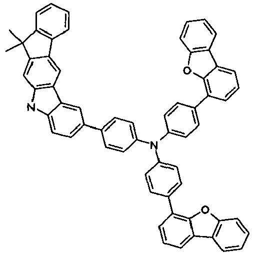 | 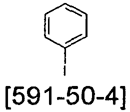 | 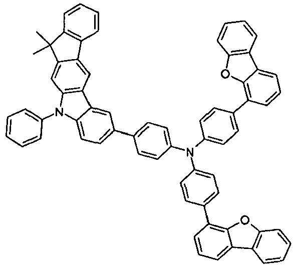 | 13 |
| **9u** | 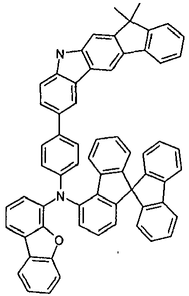 | 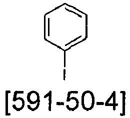 | 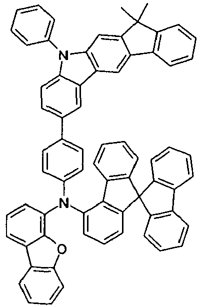 | 63 |

### Herstellung der OLEDs

In den folgenden Beispielen V1 bis V9 und E1 bis E20 (siehe Tabellen 1 und 2) werden die Daten verschiedener OLEDs vorgestellt. **Vorbehandlung für die Beispiele V1-E20:** Glasplättchen, die mit strukturiertem ITO (Indium Zinn Oxid) der Dicke 50 nm beschichtet sind, werden zur verbesserten Prozessierung mit 20 nm PEDOT:PSS beschichtet (Poly(3,4-ethylendioxythiophen) poly(styrolsulfonat), bezogen als CLEVIOS™ P VP AI 4083 von Heraeus Precious Metals GmbH Deutschland, aus wässriger Lösung aufgeschleudert). Diese beschichteten Glasplättchen bilden die Substrate, auf weiche die OLEDs aufgebracht werden.

Die OLEDs haben prinzipiell folgenden Schichtaufbau: Substrat / Lochtransportschicht (HTL) / optionale Zwischenschicht (IL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC5:9a:TEG1 (60%:30%:10%) bedeutet hierbei, dass das Material IC5 in einem Volumenanteil von 60%, 9a in einem Volumenanteil von 30% und TEG1 in einem Volumenanteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 2 bezeichnet die Spannung, die für eine Leuchtdichte von 1000 cd/m² benötigt wird. EQE1000 bezeichnet die externe Quanteneffizienz bei einer Betriebsleuchtdichte von 1000 cd/m². Als Lebensdauer LD wird die Zeit definiert, nach der die Leuchtdichte bei Betrieb mit konstantem Strom von der Startleuchtdichte auf einen gewissen Anteil L₁ absinkt. Eine Angabe von Lo;jo = 4000 cd/m² und L₁ = 70% in Tabelle 2 bedeutet, dass die in Spalte LD angegebene Lebensdauer der Zeit entspricht, nach der die Anfangsleuchtdichte von 4000 cd/m² auf 2800 cd/m² absinkt. Analog bedeutet Lo;jo = 20mA/cm², L₁= 80%, dass die Leuchtdichte bei Betrieb mit 20mA/cm² nach der Zeit LD auf 80% ihres Anfangswertes absinkt.

Die Daten der verschiedenen OLEDs sind in Tabelle 2 zusammengefasst. Die Beispiele V1 bisV29 sind Vergleichsbeispiele gemäß dem Stand der Technik, die Beispiele E1-E20 zeigen Daten von erfindungsgemäßen OLEDs.

Im Folgenden werden einige der Beispiele näher erläutert, um die Vorteile der erfindungsgemäßen OLEDs zu verdeutlichen.

### Verwendung von erfindungsgemäßen Mischungen in der Emissionsschicht phosphoreszenter OLEDs

Die erfindungsgemäßen Materialien ergeben bei Einsatz in der Emissionsschicht (EML) phosphoreszierender OLEDs wesentliche Verbesserungen gegenüber dem Stand der Technik, vor allem bezüglich der Lebensdauer der OLED Bauteile. Durch Einsatz beispielsweise der erfindungsgemäßen Verbindungen 9a, 9b, 9c und 9h in Kombination mit IC5 und dem grünen Dotanden TEG1 lässt sich eine Erhöhung der Lebensdauer um mehr als 40% gegenüber dem Stand der Technik SdT1-SdT9 beobachten (Vergleich der Beispiele V1-V9 mit E1-E4). Dies gilt auch für die weiteren erfindungsgemäßen Verbindungen, wie den Beispielen in Tabelle 2 entnommen werden kann. Dies ist ein überraschendes und nicht vorhersehbares Ergebnis, da die Verbindungen gemäß dem Stand der Technik den erfindungsgemäßen Verbindungen strukturell sehr ähnlich sind.

### Verwendung von erfindungsgemäßen Mischungen in der Elektronenblockierschicht phosphoreszenter OLEDs

Die erfindungsgemäßen Materialien ergeben auch bei Einsatz als Elektronenblockiermaterial in der Elektronenblockierschicht phosphoreszenter OLEDs wesentliche Verbesserungen gegenüber dem Stand der Technik, vor allem bezüglich der Lebensdauer. Durch Einsatz der erfindungsgemäßen Verbindungen, beispielsweise der Verbindungen 9f, 9g, 9j, 9k, 9n, 9o und 9t in den Beispielen E7, E8, E10, E11, E14, E15 und E20 lassen sich OLEDs mit verbesserten Lebensdauern herstellen.

**Tabelle 1: Aufbau der OLEDs**

| Bsp. | HIL Dicke | IL Dicke | HTL Dicke | EBL Dicke | EML Dicke | HBL Dicke | ETL Dicke | EIL Dicke |
|---|---|---|---|---|---|---|---|---|
| V1 | SpA1 | HATCN | SpMA1 | --- | IC5:SdT1 :TEG1 | ST2 | ST2:LiQ | --- |
| | 70nm | 5nm | 70nm | | (60%:30%:10%) 30nm | 10nm | (50%:50%) 30nm | |
| V2 | SpA1 | HATCN | SpMA1 | --- | IC5:SdT2:TEG1 | ST2 | ST2:LiQ | --- |
| | 70nm | 5nm | 70nm | | (60%:30%:10%)30nm | 10nm | (50%:50%) 30nm | |
| V3 | SpA1 | HATCN | SpMA1 | --- | IC5:SdT3:TEG1 | ST2 | ST2:LiQ | --- |
| | 70nm | 5nm | 70nm | | (60%:30%:10%)30nm | 10nm | (50%:50%) 30nm | |
| V4 | SpA1 | HATCN | SpMA1 | --- | IC5:SdT4:TEG1 | ST2 | ST2:LiQ | --- |
| | 70nm | 5nm | 70nm | | (60%:30%:10%)30nm | 10nm | (50%:50%) 30nm | |
| V5 | SpA1 | HATCN | SpMA1 | --- | IC5:SdT5:TEG1 | ST2 | ST2:LiQ | --- |
| | 70nm | 5nm | 70nm | | (60%:30%:10%)30nm | 10nm | (50%:50%) 30nm | |
| V6 | SpA1 | HATCN | SpMA1 | --- | IC5:SdT6:TEG1 | ST2 | ST2:LiQ | --- |
| | 70nm | 5nm | 70nm | | (60%:30%:10%)30nm | 10nm | (50%:50%) 30nm | |
| V7 | SpA1 | HATCN | SpMA1 | --- | IC5:SdT7:TEG1 | ST2 | ST2:LiQ | --- |
| | 70nm | 5nm | 70nm | | (60%:30%:10%) 30nm | 10nm | (50%:50%) 30nm | |
| V8 | SpA1 | HATCN | SpMA1 | --- | IC5:SdT8:TEG1 | ST2 | ST2:LiQ | --- |
| | 70nm | 5nm | 70nm | | (60%:30%:10%) 30nm | 10nm | (50%:50%) 30nm | |
| V9 | SpA1 | HATCN | SpMA1 | --- | IC5:SdT9:TEG1 | ST2 | ST2:LiQ | --- |
| | 70nm | 5nm | 70nm | | (60%:30%:10%) 30nm | 10nm | (50%:50%) 30nm | |
| E1 | SpA1 | HATCN | SpMA1 | --- | IC5:9a:TEG1 | ST2 | ST2:LiQ | --- |
| | 70nm | 5nm | 70nm | | (60%:30%:10%) 30nm | 10nm | (50%:50%) 30nm | |
| E2 | SpA1 | HATCN | SpMA1 | --- | IC5:9b:TEG1 | ST2 | ST2:LiQ | --- |
| | 70nm | 5nm | 70nm | | (60%:30%:10%) 30nm | 10nm | (50%:50%) 30nm | |
| E3 | SpA1 | HATCN | SpMA1 | --- | IC5:9c:TEG1 | ST2 | ST2:LiQ | --- |
| | 70nm | 5nm | 70nm | | (60%:30%:10%) 30nm | 10nm | (50%:50%) 30nm | |
| E4 | SpA1 | HATCN | SpMA1 | --- | IC5:9h:TEG1 | ST2 | ST2:LiQ | --- |
| | 70nm | 5nm | 70nm | | (60%:30%:10%) 30nm | 10nm | (50%:50%) 30nm | |
| E5 | SpA1 | HATCN | SpMA1 | --- | IC5:9d:TEG1 | ST2 | ST2:LiQ | --- |
| | 70nm | 5nm | 70nm | | (60%:30%:10%) 30nm | 10nm | (50%:50%) 30nm | |
| E6 | SpA1 | HATCN | SpMA1 | --- | IC5:9e:TEG1 | ST2 | ST2:LiQ | --- |
| | 70nm | 5nm | 70nm | | (60%:30%:10%) 30nm | 10nm | (50%:50%) 30nm | |
| E7 | SpA1 | HATCN | SpMA1 | 9h | IC5:IC3:TEG1 | ST2 | ST2:LiQ | --- |
| | 70nm | 5nm | 50nm | 20nm | (45%:45%:10%) 30nm | 10nm | (50%:50%) 30nm | |
| E8 | SpA1 | HATCN | SpMA1 | 9g | IC5:IC3:TEG1 | ST2 | ST2:LiQ | --- |
| | 70nm | 5nm | 50nm | 20nm | (45%:45%:10%) 30nm | 10nm | (50%:50%) 30nm | |
| E9 | SpA1 | HATCN | SpMA1 | --- | IC5:9u:TEG1 | ST2 | ST2:LiQ | --- |
| | 70nm | 5nm | 70nm | | (50%:40%:10%) 30nm | 10nm | (50%:50%) 30nm | |
| E10 | SpA1 | HATCN | SpMA1 | 9j | IC5:IC3:TEG1 | ST2 | ST2:LiQ | --- |
| | 70nm | 5nm | 50nm | 20nm | (45%:45%:10%) 30nm | 10nm | (50%:50%) 30nm | |
| E11 | SpA1 | HATCN | SpMA1 | 9k | IC5:IC3:TEG1 | ST2 | ST2:LiQ | --- |
| | 70nm | 5nm | 50nm | 20nm | (45%:45%:10%) 30nm | 10nm | (50%:50%) 30nm | |
| E12 | SpA1 | HATCN | SpMA1 | --- | IC5:9I:TEG1 | ST2 | ST2:LiQ | --- |
| | 70nm | 5nm | 70nm | | (60%:30%:10%) 30nm | 10nm | (50%:50%) 30nm | |
| E13 | SpA1 | HATCN | SpMA1 | 9m | IC5:9m:TEG1 | ST2 | ST2:LiQ | --- |
| | 70nm | 5nm | 50nm | 20nm | (60%:30%:10%) 30nm | 10nm | (50%:50%) 30nm | |
| E14 | SpA1 | HATCN | SpMA1 | 9n | IC1:TEG1 | ST2 | ST2:LiQ | --- |
| | 70nm | 5nm | 50nm | 20nm | (90%:10%) 30nm | 10nm | (50%:50%) 30nm | |
| E 15 | SpA1 | HATCN | SpMA1 | 9o | IC1:TEG1 | ST2 | ST2:LiQ | --- |
| | 70nm | 5nm | 50nm | 20nm | (90%:10%) 30nm | 10nm | (50%:50%) 30nm | |
| E16 | SpA1 | HATCN | SpMA1 | 9p | IC5:9p:TEG1 | ST2 | ST2:LiQ | --- |
| | 70nm | 5nm | 50nm | 20nm | (60%:30%:10%) 30nm | 10nm | (50%:50%) 30nm | |
| E17 | SpA1 | HATCN | SpMA1 | --- | IC5:9q:TEG1 | ST2 | ST2:LiQ | --- |
| | 70nm | 5nm | 70nm | | (60%:30%:10%) 30nm | 10nm | (50%:50%) 30nm | |
| E18 | SpA1 | HATCN | SpMA1 | --- | IC5:9r:TEG1 | ST2 | ST2:LiQ | --- |
| | 70nm | 5nm | 70nm | | (60%:30%:10%)30nm | 10nm | (50%:50%) 30nm | |
| E19 | SpA1 | HATCN | SpMA1 | 9s | IC5:9s:TEG1 | ST2 | ST2:LiQ | --- |
| | 70nm | 5nm | 50nm | 20nm | (60%:30%:10%) 30nm | 10nm | (50%:50%) 30nm | |
| E20 | SpA1 | HATCN | SpMA1 | 9t | IC5:IC3:TEG1 | ST2 | ST2:LiQ | --- |
| | 70nm | 5nm | 50nm | 20nm | (45%:45%:10%) 30nm | 10nm | (50%:50%) 30nm | |
| E21 | SpA1 | HATCN | SpMA1 | --- | IC5:9f:TEG1 | ST2 | ST2:LiQ | --- |
| | 70nm | 5nm | 70nm | | (60%:30%:10%) 30nm | 10nm | (50%:50%) 30nm | |
| E22 | SpA1 | HATCN | SpMA1 | --- | IC5:9g:TEG1 | ST2 | ST2:LiQ | --- |
| | 70nm | 5nm | 70nm | | (60%:30%:10%) 30nm | 10nm | (50%:50%) 30nm | |
| E23 | SpA1 | HATCN | SpMA1 | --- | IC5:9j:TEG1 | ST2 | ST2:LiQ | --- |
| | 70nm | 5nm | 70nm | | (60%:30%:10%) 30nm | 10nm | (50%:50%) 30nm | |
| E24 | SpA1 | HATCN | SpMA1 | --- | IC5:9k:TEG1 | ST2 | ST2:LiQ | --- |
| | 70nm | 5nm | 70nm | | (60%:30%:10%) 30nm | 10nm | (50%:50%) 30nm | |
| E24 | SpA1 | HATCN | SpMA1 | --- | IC5:9n:TEG1 | ST2 | ST2:LiQ | --- |
| | 70nm | 5nm | 70nm | | (60%:30%:10%) 30nm | 10nm | (50%:50%) 30nm | |
| E25 | SpA1 | HATCN | SpMA1 | --- | IC5:9o:TEG1 | ST2 | ST2:LiQ | --- |
| | 70nm | 5nm | 70nm | | (60%:30%:10%) 30nm | 10nm | (50%:50%) 30nm | |
| E26 | SpA1 | HATCN | SpMA1 | --- | IC5:9t:TEG1 | ST2 | ST2:LiQ | --- |
| | 70nm | 5nm | 70nm | | (60%:30%:10%) 30nm | 10nm | (50%:50%) 30nm | |
| E27 | SpA1 | HATCN | SpMA1 | --- | IC5:9m:TEG1 | ST2 | ST2:LiQ | --- |
| | 70nm | 5nm | 70nm | | (60%:30%:10%) 30nm | 10nm | (50%:50%) 30nm | |
| E28 | SpA1 | HATCN | SpMA1 | --- | IC5:9p:TEG1 | ST2 | ST2:LiQ | --- |
| | 70nm | 5nm | 70nm | | (60%:30%:10%) 30nm | 10nm | (50%:50%) 30nm | |
| E29 | SpA1 | HATCN | SpMA1 | --- | IC5:9s:TEG1 | ST2 | ST2:LiQ | --- |
| | 70nm | 5nm | 70nm | | (60%:30%:10%) 30nm | 10nm | (50%:50%) 30nm | |
| E30 | SpA1 | HATCN | SpMA1 | --- | IC5:9u:TEG1 | ST2 | ST2:LiQ | --- |
| | 70nm | 5nm | 70nm | | (60%:30%:10%) 30nm | 10nm | (50%:50%) 30nm | |

**Tabelle 2: Daten der OLEDs**

| Bsp. | U1000 (V) | EQE 1000 | L₀; j₀ | L₁ % | LD (h) |
|---|---|---|---|---|---|
| V1 | 3.1 | 15.3% | 20 mA/cm² | 80 | 130 |
| V2 | 3.2 | 15.4% | 20 mA/cm² | 80 | 120 |
| V3 | 3.2 | 15.2% | 20 mA/cm² | 80 | 140 |
| V4 | 3.3 | 15.5% | 20 mA/cm² | 80 | 110 |
| V5 | 3.1 | 15.6% | 20 mA/cm² | 80 | 105 |
| V6 | 3.2 | 15.7% | 20 mA/cm² | 80 | 100 |
| V7 | 3.1 | 15.6% | 20 mA/cm² | 80 | 115 |
| V8 | 3.2 | 15.1% | 20 mA/cm² | 80 | 75 |
| V9 | 3.1 | 15.8% | 20 mA/cm² | 80 | 125 |
| E1 | 3.2 | 16.1% | 20 mA/cm² | 80 | 260 |
| E2 | 3.3 | 16.3% | 20 mA/cm² | 80 | 230 |
| E3 | 3.3 | 16.2% | 20 mA/cm² | 80 | 240 |
| E4 | 3.2 | 16.0% | 20 mA/cm² | 80 | 255 |
| E5 | 3.3 | 16.2% | 20 mA/cm² | 80 | 235 |
| E6 | 3.2 | 16.1% | 20 mA/cm² | 80 | 225 |
| E7 | 3.2 | 16.7% | 20 mA/cm² | 80 | 265 |
| E8 | 3.3 | 16.5% | 20 mA/cm² | 80 | 225 |
| E9 | 3.4 | 16.2% | 20 mA/cm² | 80 | 250 |
| E10 | 3.4 | 16.6% | 20 mA/cm² | 80 | 225 |
| E11 | 3.5 | 16.5% | 20 mA/cm² | 80 | 220 |
| E12 | 3.1 | 16.0% | 20 mA/cm² | 80 | 210 |
| E13 | 3.3 | 16.3% | 20 mA/cm² | 80 | 245 |
| E14 | 3.5 | 17.4% | 20 mA/cm² | 80 | 125 |
| E15 | 3.4 | 17.5% | 20 mA/cm² | 80 | 115 |
| E16 | 3.4 | 16.2% | 20 mA/cm² | 80 | 255 |
| E17 | 3.3 | 16.0% | 20 mA/cm² | 80 | 250 |
| E18 | 3.3 | 16.1% | 20 mA/cm² | 80 | 245 |
| E19 | 3.3 | 16.2% | 20 mA/cm² | 80 | 240 |
| E20 | 3.3 | 16.5% | 20 mA/cm² | 80 | 270 |
| E21 | 3.4 | 15.6% | 20 mA/cm² | 80 | 150 |
| E22 | 3.4 | 15.5% | 20 mA/cm² | 80 | 155 |
| E23 | 3.3 | 15.8% | 20 mA/cm² | 80 | 160 |
| E24 | 3.4 | 15.9% | 20 mA/cm² | 80 | 150 |
| E24 | 3.4 | 16.1% | 20 mA/cm² | 80 | 145 |
| E25 | 3.3 | 16.0% | 20 mA/cm² | 80 | 220 |
| E26 | 3.4 | 15.7% | 20 mA/cm² | 80 | 155 |
| E27 | 3.4 | 16.1% | 20 mA/cm² | 80 | 230 |
| E28 | 3.5 | 16.2% | 20 mA/cm² | 80 | 245 |
| E29 | 3.3 | 16.0% | 20 mA/cm² | 80 | 250 |
| E30 | 3.4 | 16.2% | 20 mA/cm² | 80 | 235 |

**Tabelle 3: Strukturformeln der Materialien für die OLEDs**

| | |
|---|---|
| 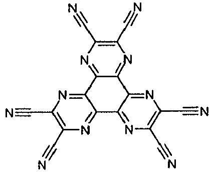 | 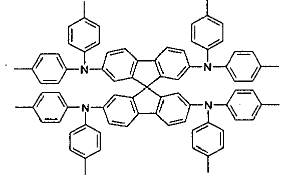 |
| HATCN | SpA1 |
| 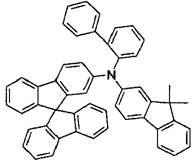 | 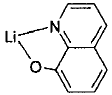 |
| SpMA1 | LiQ |
| 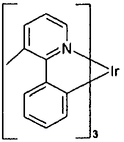 | 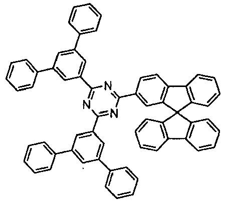 |
| TEG1 | ST2 |
| 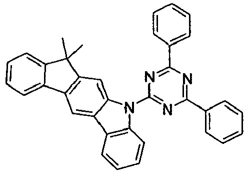 | 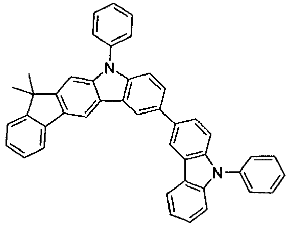 |
| IC1 | IC3 |
| 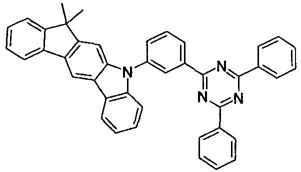 | 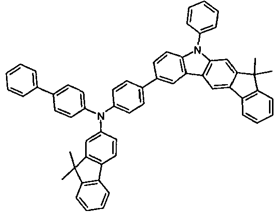 |
| IC5 | SdT1 (gemäß WO 2012/014500) |
| 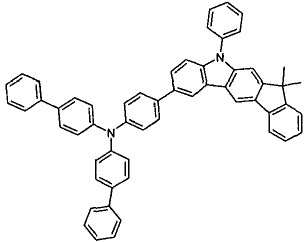 | 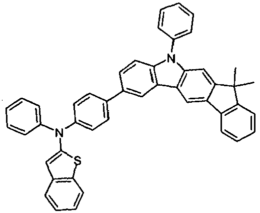 |
| SdT2 (gemäß WO 2012/014500) | SdT3 (gemäß WO 2012/014500) |
| 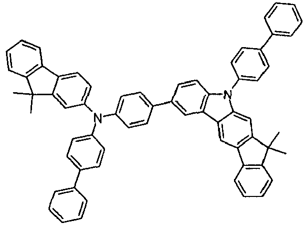 | 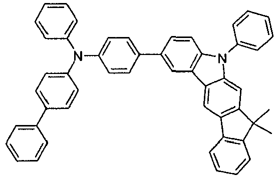 |
| SdT4 (gemäß WO 2010/136109) | SdT5 (gemäß WO 2010/136109) |
| 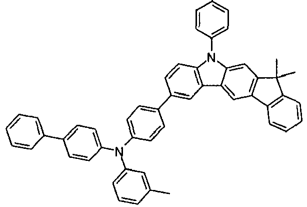 | 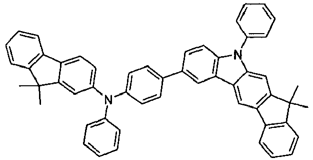 |
| SdT6 (gemäß WO 2010/136109) | SdT7 (gemäß WO 2010/136109) |
| 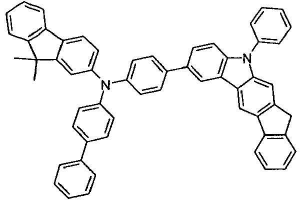 | 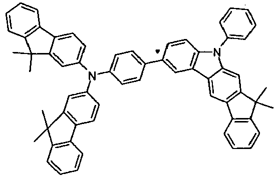 |
| SdT8 (gemäß WO 2010/136109) | SdT9 (gemäß WO 2012/014500) |
| 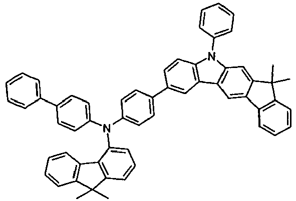 | 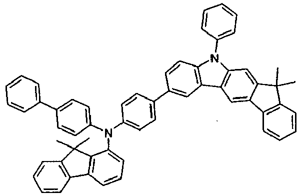 |
| 9a | 9b |
| 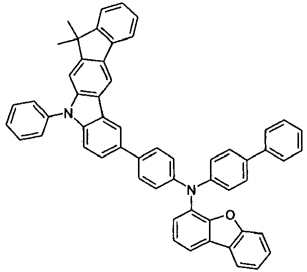 | 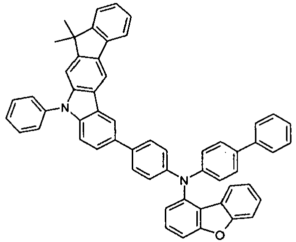 |
| 9c | 9h |
| 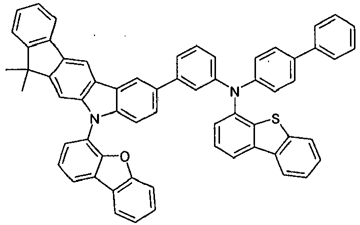 | 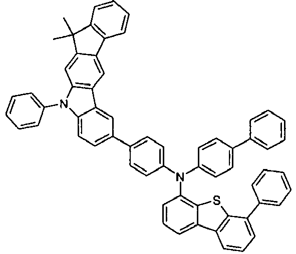 |
| 9d | 9e |
| 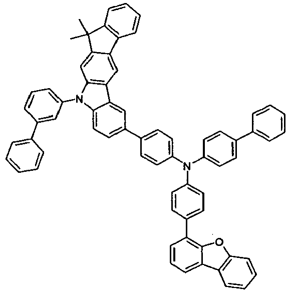 | 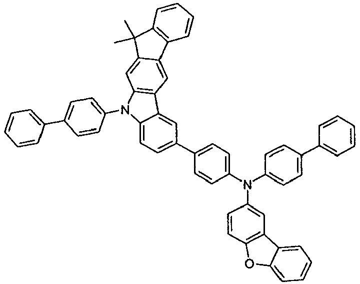 |
| 9f | 9g |
| 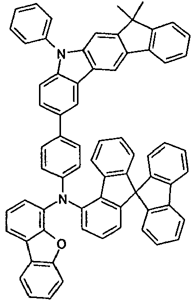 | 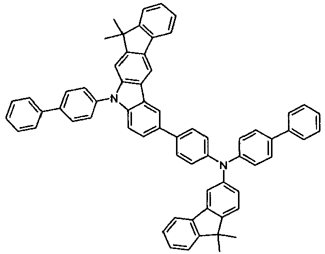 |
| 9u | 9j |
| 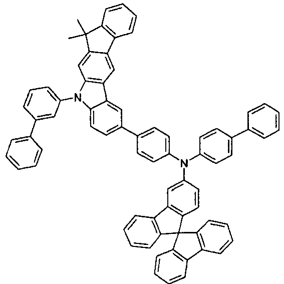 | 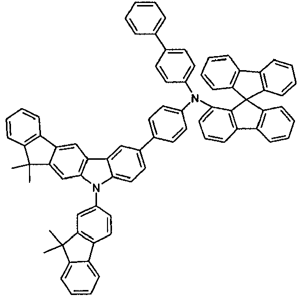 |
| 9k | 9l |
| 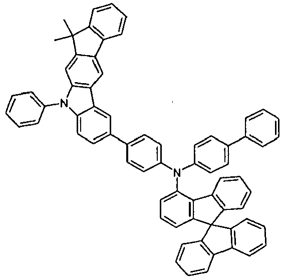 | 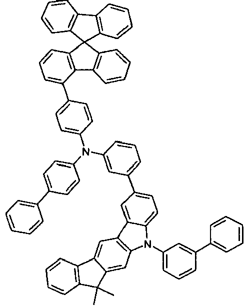 |
| 9m | 9n |
| 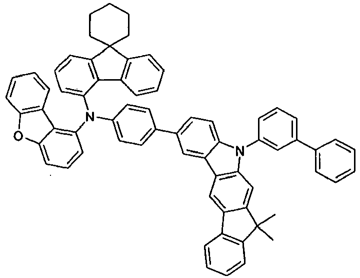 | 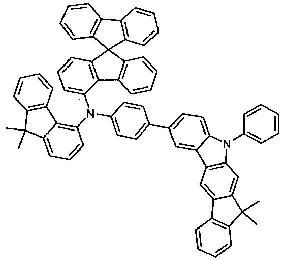 |
| 9o | 9p |
| 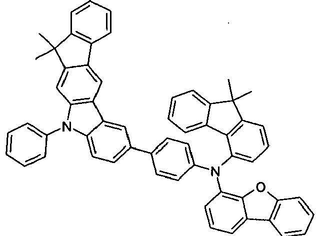 | 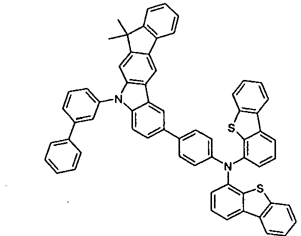 |
| 9q | 9r |
| 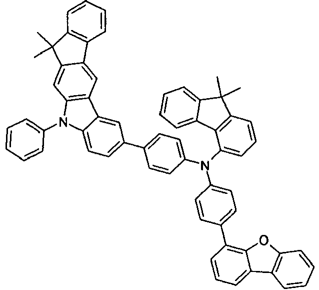 | 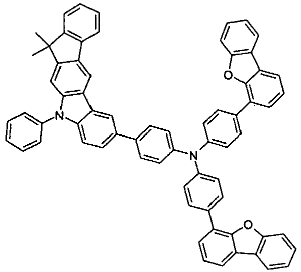 |
| 9s | 9t |

## Patentansprüche

1. Verbindung gemäß Formel (1), 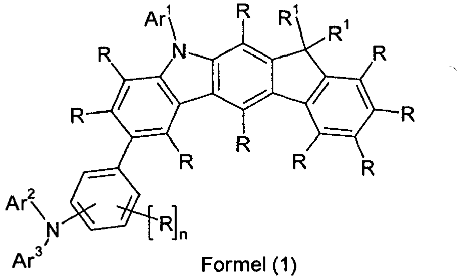 wobei für die verwendeten Symbole und Indizes gilt:
Ar¹, Ar² ist bei jedem Auftreten gleich oder verschieden ein aromatisches oder heteroaromatisches Ringsystem mit 5 bis 30 aromatischen Ringatomen, das mit einem oder mehreren Resten R substituiert sein kann;
Ar³ ist eine Gruppe gemäß Formel (2), 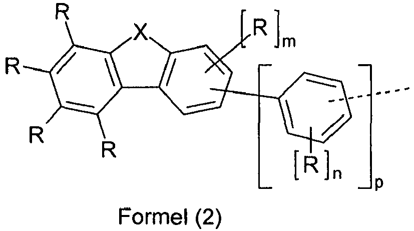 wobei die gestrichelte Bindung die Verknüpfung dieser Gruppe mit dem Stickstoffatom andeutet und die Fluoren- bzw. Dibenzofuran- bzw. Dibenzothiophengruppe über die 1-, 3- oder 4-Position für p = 1 mit der Phenylengruppe bzw. für p = 0 mit dem Stickstoffatom verknüpft ist;
X ist C(R¹)₂, O oder S;
R, R¹ ist bei jedem Auftreten gleich oder verschieden ausgewählt aus der Gruppe bestehend aus H, D, F, Cl, Br, I, CN, NO₂, N(R²)₂, OR², SR², C(=O)R², P(=O)R², Si(R²)₃, einer geradkettigen Alkylgruppe mit 1 bis 20 C-Atomen oder einer verzweigten oder cyclischen Alkylgruppe mit 3 bis 20 C-Atomen oder einer Alkenyl- oder Alkinylgruppe mit 2 bis 20 C-Atomen, wobei die Alkyl-, Alkenyl- oder Alkinylgruppe jeweils mit einem oder mehreren Resten R² substituiert sein kann und wobei eine oder mehrere nicht-benachbarte CH₂-Gruppen durch R²C=CR², C≡C, Si(R²)₂, C=O, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R² substituiert sein kann; dabei können optional zwei benachbarte Substituenten R bzw. zwei benachbarte Substituenten R¹ ein monocyclisches oder polycyclisches, aliphatisches, aromatisches oder heteroaromatisches Ringsystem bilden, das mit einem oder mehreren Resten R² substituiert sein kann;
R² ist bei jedem Auftreten gleich oder verschieden ausgewählt aus der Gruppe bestehend aus H, D, F, CN, einem aliphatischen Kohlenwasserstoffrest mit 1 bis 20 C-Atomen, oder einem aromatischen oder heteroaromatischen Ringsystem mit 5 bis 30 aromatischen Ringatomen, in dem ein oder mehrere H-Atome durch D, F, CN oder eine Alkylgruppe mit 1 bis 10 C-Atomen ersetzt sein können, wobei zwei oder mehr benachbarte Substituenten R² miteinander ein mono- oder polycyclisches, aliphatisches Ringsystem bilden können;
m ist 0, 1, 2 oder 3;
n ist bei jedem Auftreten gleich oder verschieden 0, 1, 2, 3 oder 4;
p ist 0 oder 1,
wobei die 1-, 3- oder 4-Position wie unten definiert ist:

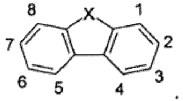

2. Verbindung nach Anspruch 1, ausgewählt aus den Verbindungen der Formeln (1a) und (1b), 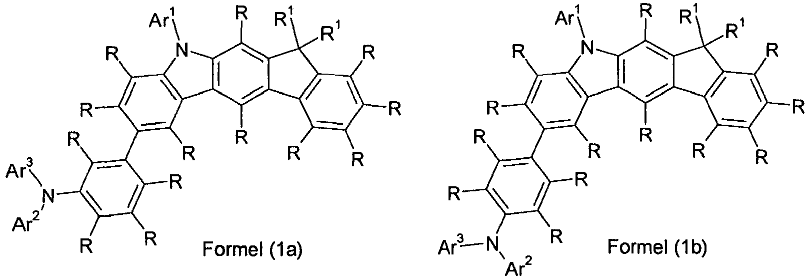 wobei die verwendeten Symbole die in Anspruch 1 genannten Bedeutungen aufweisen.

3. Verbindung nach Anspruch 1 oder 2 gemäß Formel (3), 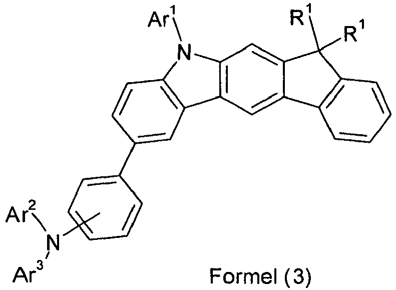 wobei die verwendeten Symbole die in Anspruch 1 genannten Bedeutungen aufweisen.

4. Verbindung nach einem oder mehreren der Ansprüche 1 bis 3, **dadurch gekennzeichnet, dass** die Reste R¹ am Indenocarbazol bei jedem Auftreten gleich oder verschieden ausgewählt aus der Gruppe bestehend aus einer geradkettigen Alkylgruppe mit 1 bis 10 C-Atomen oder einer verzweigten oder cyclischen Alkylgruppe mit 3 bis 10 C-Atomen oder einer Alkenylgruppe mit 2 bis 10 C-Atomen, die jeweils mit einem oder mehreren Resten R² substituiert sein kann, wobei eine oder mehrere nicht-benachbarte CH₂-Gruppen durch O ersetzt sein können und wobei ein oder mehrere H-Atome durch D oder F ersetzt sein können, oder einem aromatischen oder heteroaromatischen Ringsystem mit 6 bis 24 aromatischen Ringatomen, das jeweils mit einem oder mehreren Resten R² substituiert sein kann; dabei können die beiden Substituenten R¹ ein monocyclisches oder polycyclisches, aliphatisches, aromatisches oder heteroaromatisches Ringsystem bilden, das mit einem oder mehreren Resten R² substituiert sein kann.

5. Verbindung nach einem oder mehreren der Ansprüche 1 bis 4, ausgewählt aus den Verbindungen der Formeln (3a) und (3b), 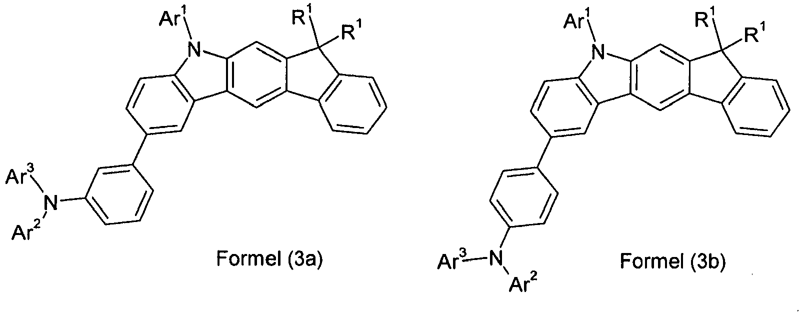 wobei die verwendeten Symbole die in Anspruch 1 genannten Bedeutungen aufweisen.

6. Verbindung nach einem oder mehreren der Ansprüche 1 bis 5, **dadurch gekennzeichnet, dass** Ar¹ und Ar², gleich oder verschieden bei jedem Auftreten, ausgewählt ist aus der Gruppe bestehend aus Phenyl, Biphenyl, Terphenyl, Quaterphenyl, Fluorenyl, Spirobifluorenyl, Dibenzofuranyl, Dibenzothienyl oder N-Phenyl-Carbazolyl, wobei diese Gruppen jeweils durch einen oder mehrere nicht-aromatische Reste R substituiert sein können.

7. Verbindung nach einem oder mehreren der Ansprüche 1 bis 6, **dadurch gekennzeichnet, dass** die Gruppe der Formel (2) ausgewählt ist aus den Formeln (2a) bis (2c), 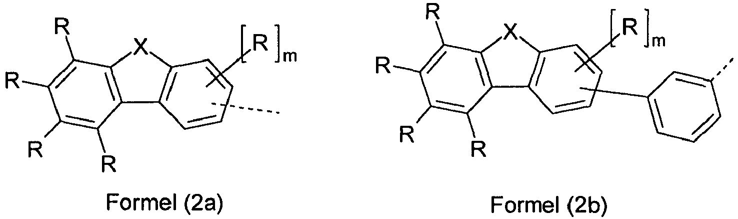 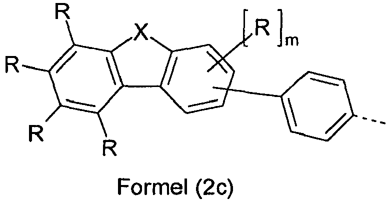 wobei die verwendeten Symbole und Indizes die in Anspruch 1 genannten Bedeutungen aufweisen und die Fluorengruppe bzw. Dibenzofurangruppe bzw. Dibenzothiophengruppe über die 1-, 3- oder 4-Position verknüpft ist.

8. Verbindung nach einem oder mehreren der Ansprüche 1 bis 7, **dadurch gekennzeichnet, dass** die Gruppe der Formel (2) ausgewählt ist aus den Gruppen der Formeln (2a-1) bis (2a-9), 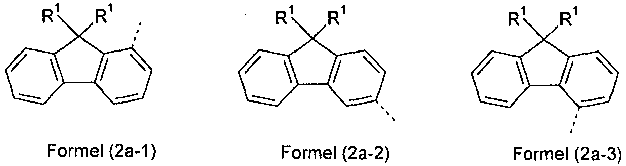 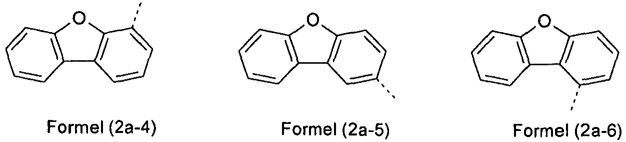 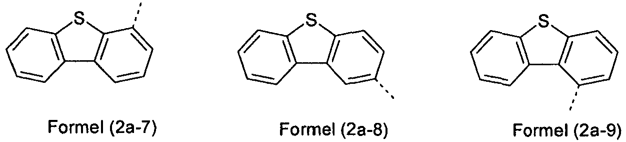 wobei die gestrichelte Bindung die Verknüpfung dieser Gruppe mit dem Stickstoffatom andeutet und die weiteren verwendeten Symbole die in Anspruch 1 genannten Bedeutungen aufweisen.

9. Verbindung nach einem oder mehreren der Ansprüche 1 bis 8, **dadurch gekennzeichnet, dass** X für C(R¹)₂ oder O steht und dass für X = C(R¹)₂ R¹ gleich oder verschieden bei jedem Auftreten ausgewählt ist aus der Gruppe bestehend aus einer geradkettigen Alkylgruppe mit 1 bis 10 C-Atomen oder einer verzweigten oder cyclischen Alkylgruppe mit 3 bis 10 C-Atomen oder einer Alkenylgruppe mit 2 bis 10 C-Atomen, die jeweils mit einem oder mehreren Resten R² substituiert sein kann, wobei eine oder mehrere nicht-benachbarte CH₂-Gruppen durch O ersetzt sein können und wobei ein oder mehrere H-Atome durch D oder F ersetzt sein können, oder einem aromatischen oder heteroaromatischen Ringsystem mit 6 bis 24 aromatischen Ringatomen, das jeweils mit einem oder mehreren Resten R² substituiert sein kann; dabei können die beiden Substituenten R¹ ein monocyclisches oder polycyclisches, aliphatisches, aromatisches oder heteroaromatisches Ringsystem bilden, das mit einem oder mehreren Resten R² substituiert sein kann.

10. Verbindung nach einem oder mehreren der Ansprüche 1 bis 9, **dadurch gekennzeichnet, dass** R gleich oder verschieden bei jedem Auftreten ausgewählt ist aus der Gruppe bestehend aus H, D, F, CN, N(R²)₂, OR², einer geradkettigen Alkylgruppe mit 1 bis 10 C-Atomen oder einer verzweigten oder cyclischen Alkylgruppe mit 3 bis 10 C-Atomen oder einer Alkenylgruppe mit 2 bis 10 C-Atomen, die jeweils mit einem oder mehreren Resten R² substituiert sein kann, wobei eine oder mehrere nicht-benachbarte CH₂-Gruppen durch O ersetzt sein können und wobei ein oder mehrere H-Atome durch D oder F ersetzt sein können, oder einem aromatischen oder heteroaromatischen Ringsystem mit 5 bis 30 aromatischen Ringatomen, das jeweils mit einem oder mehreren Resten R² substituiert sein kann.

11. Verfahren zur Herstellung einer Verbindung nach einem oder mehreren der Ansprüche 1 bis 10, umfassend eine Kupplungsreaktion zwischen der Einheit Ar²Ar³N-Phenylen, welche mit einer reaktiven Abgangsgruppe substituiert ist, und einem Indenocarbazol, welches mit einer reaktiven Abgangsgruppe substituiert ist.

12. Formulierung, enthaltend mindestens eine Verbindung nach einem oder mehreren der Ansprüche 1 bis 10 und mindestens eine weitere Verbindung, insbesondere ein Lösemittel.

13. Verwendung einer Verbindung nach einem oder mehreren der Ansprüche 1 bis 10 in einer elektronischen Vorrichtung.

14. Elektronische Vorrichtung enthaltend eine Verbindung nach einem oder mehreren der Ansprüche 1 bis 10.

15. Elektronische Vorrichtung nach Anspruch 14, wobei es sich um eine organische Elektrolumineszenzvorrichtung handelt, **dadurch gekennzeichnet, dass** die Verbindung nach einem oder mehreren der Ansprüche 1 bis 13 als Matrixmaterial für phosphoreszierende Emitter in einer emittierenden Schicht oder in einer Elektronenblockierschicht oder in einer Lochtransport- oder Lochinjektionsschicht eingesetzt wird.

## Claims

1. Compound of formula (1) 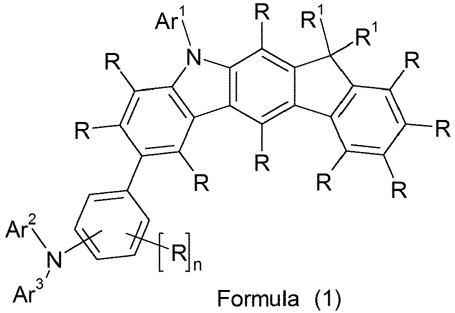 where the symbols and indices used are as follows:
Ar¹, Ar² is the same or different at each instance and is an aromatic or heteroaromatic ring system which has 5 to 30 aromatic ring atoms and may be substituted by one or more R radicals;
Ar³ is a group of formula (2) 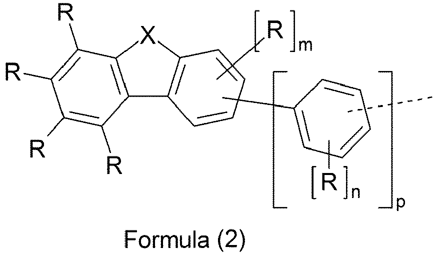 where the dotted bond indicates the linkage of this group to the nitrogen atom and the fluorene or dibenzofuran or dibenzothiophene group is joined to the phenylene group via the 1, 3 or 4 position when p = 1 and to the nitrogen atom when p = 0;
X is C(R¹)₂, O or S;
R, R¹ is the same or different at each instance and is selected from the group consisting of H, D, F, Cl, Br, I, CN, NO₂, N(R²)₂, OR², SR², C(=O)R², P(=O)R², Si(R²)₃, a straight-chain alkyl group having 1 to 20 carbon atoms or a branched or cyclic alkyl group having 3 to 20 carbon atoms or an alkenyl or alkynyl group having 2 to 20 carbon atoms, where the alkyl, alkenyl or alkynyl group may be substituted in each case by one or more R² radicals and where one or more nonadjacent CH₂ groups may be replaced by R²C=CR², C≡C, Si(R²)₂, C=O, C=NR², P(=O)(R²), SO, SO₂, NR², O, S or CONR² and where one or more hydrogen atoms may be replaced by D, F, Cl, Br, I, CN or NO₂, an aromatic or heteroaromatic ring system which has 5 to 40 aromatic ring atoms and may be substituted in each case by one or more R² radicals; at the same time, it is optionally possible for two adjacent R substituents or two adjacent R¹ substituents to form a monocyclic or polycyclic, aliphatic, aromatic or heteroaromatic ring system which may be substituted by one or more R² radicals;
R² is the same or different at each instance and is selected from the group consisting of H, D, F, CN, an aliphatic hydrocarbyl radical having 1 to 20 carbon atoms, or an aromatic or heteroaromatic ring system having 5 to 30 aromatic ring atoms in which one or more hydrogen atoms may be replaced by D, F, CN or an alkyl group having 1 to 10 carbon atoms, where two or more adjacent R² substituents together may form a mono- or polycyclic, aliphatic ring system;
m is 0, 1, 2 or 3;
n is the same or different at each instance and is 0, 1, 2, 3 or 4;
p is 0 or 1,
where the 1, 3 or 4 position is as defined below:

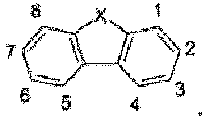

2. Compound according to Claim 1, selected from the compounds of the formulae (1a) and (1b) 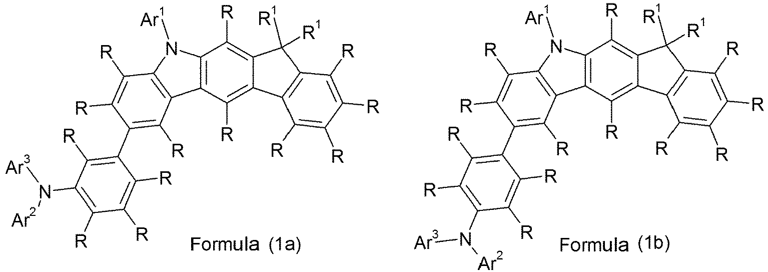 where the symbols used have the definitions given in Claim 1.

3. Compound according to Claim 1 or 2 of formula (3) 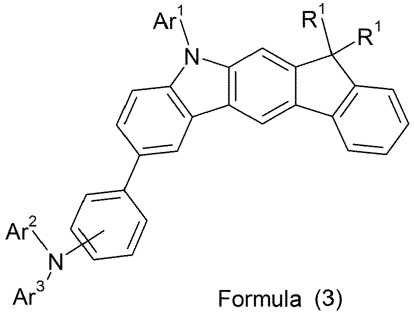 where the symbols used have the definitions given in Claim 1.

4. Compound according to one or more of Claims 1 to 3, **characterized in that** the R¹ radicals on the indenocarbazole are the same or different at each instance and are selected from the group consisting of a straight-chain alkyl group having 1 to 10 carbon atoms or a branched or cyclic alkyl group having 3 to 10 carbon atoms or an alkenyl group having 2 to 10 carbon atoms, each of which may be substituted by one or more R² radicals, where one or more nonadjacent CH₂ groups may be replaced by O and where one or more hydrogen atoms may be replaced by D or F, or an aromatic or heteroaromatic ring system which has 6 to 24 aromatic ring atoms and may be substituted in each case by one or more R² radicals; at the same time, the two R¹ substituents may form a monocyclic or polycyclic, aliphatic, aromatic or heteroaromatic ring system which may be substituted by one or more R² radicals.

5. Compound according to one or more of Claims 1 to 4, selected from the compounds of the formulae (3a) and (3b) 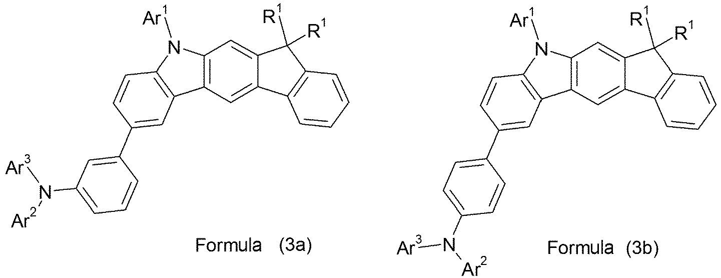 where the symbols used have the definitions given in Claim 1.

6. Compound according to one or more of Claims 1 to 5, **characterized in that** Ar¹ and Ar² are the same or different at each instance and are selected from the group consisting of phenyl, biphenyl, terphenyl, quaterphenyl, fluorenyl, spirobifluorenyl, dibenzofuranyl, dibenzothienyl and N-phenylcarbazolyl, where these groups may each be substituted by one or more nonaromatic R radicals.

7. Compound according to one or more of Claims 1 to 6, **characterized in that** the group of the formula (2) is selected from the formulae (2a) to (2c) 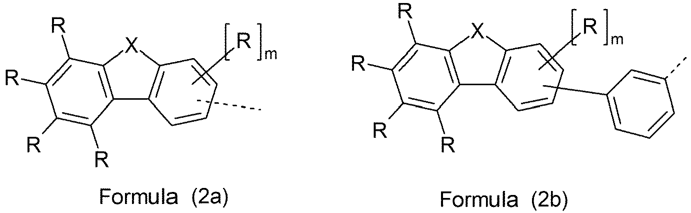 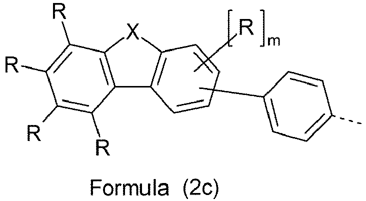 where the symbols and indices used have the definitions given in Claim 1 and the fluorene group or dibenzofuran group or dibenzothiophene group is bonded via the 1, 3 or 4 position.

8. Compound according to one or more of Claims 1 to 7, **characterized in that** the group of the formula (2) is selected from the groups of the formulae (2a-1) to (2a-9) 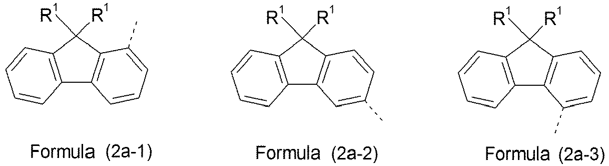 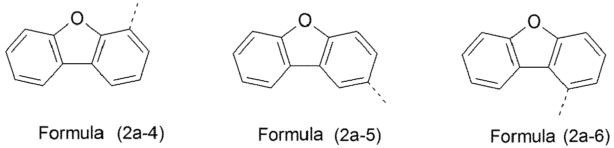 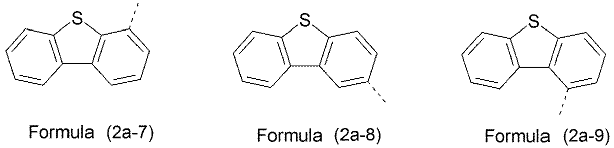 where the dotted bond represents the linkage of this group to the nitrogen atom and the further symbols used have the definitions given in Claim 1.

9. Compound according to one or more of Claims 1 to 8, **characterized in that** X is C(R¹)₂ or O, and **in that**, when X = C(R¹)₂, R¹ is the same or different at each instance and is selected from the group consisting of a straight-chain alkyl group having 1 to 10 carbon atoms or a branched or cyclic alkyl group having 3 to 10 carbon atoms or an alkenyl group having 2 to 10 carbon atoms, each of which may be substituted by one or more R² radicals, where one or more nonadjacent CH₂ groups may be replaced by O and where one or more hydrogen atoms may be replaced by D or F, or an aromatic or heteroaromatic ring system which has 6 to 24 aromatic ring atoms and may be substituted in each case by one or more R² radicals; at the same time, the two R¹ substituents may form a monocyclic or polycyclic, aliphatic, aromatic or heteroaromatic ring system which may be substituted by one or more R² radicals.

10. Compound according to one or more of Claims 1 to 9, **characterized in that** R is the same or different at each instance and is selected from the group consisting of H, D, F, CN, N(R²)₂, OR², a straight-chain alkyl group having 1 to 10 carbon atoms or a branched or cyclic alkyl group having 3 to 10 carbon atoms or an alkenyl group having 2 to 10 carbon atoms, each of which may be substituted by one or more R² radicals, where one or more nonadjacent CH₂ groups may be replaced by O and where one or more hydrogen atoms may be replaced by D or F, or an aromatic or heteroaromatic ring system which has 5 to 30 aromatic ring atoms and may be substituted in each case by one or more R² radicals.

11. Process for preparing a compound according to one or more of Claims 1 to 10, comprising a coupling reaction between the Ar²Ar³N-phenylene unit substituted by a reactive leaving group, and an indenocarbazole substituted by a reactive leaving group.

12. Formulation comprising at least one compound according to one or more of Claims 1 to 10 and at least one further compound, especially a solvent.

13. Use of a compound according to one or more of Claims 1 to 10 in an electronic device.

14. Electronic device comprising a compound according to one or more of Claims 1 to 10.

15. Electronic device according to Claim 14 which is an organic electroluminescent device, **characterized in that** the compound according to one or more of Claims 1 to 13 is used as matrix material for phosphorescent emitters in an emitting layer or in an electron blocker layer or in a hole transport or hole injection layer.

## Revendications

1. Composé selon la formule (1), 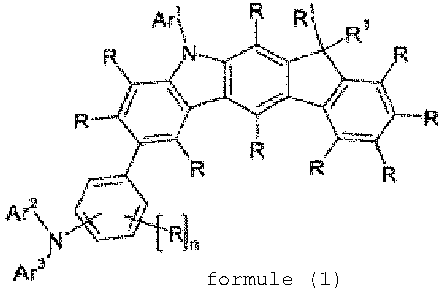 ceci s'appliquant pour les symboles et indices utilisés :
Ar¹, Ar², identiques ou différents en chaque occurrence, étant un système cyclique aromatique ou hétéroaromatique comportant 5 à 30 atomes de cycle aromatiques, qui peut être substitué par un ou plusieurs radicaux R ;
Ar³ étant un groupe selon la formule (2), 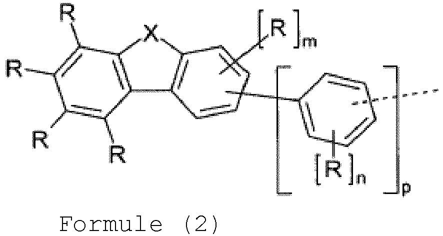 la liaison en pointillés indiquant le lien de ce groupe à l'atome d'azote et le groupe fluorène respectivement dibenzofuranne respectivement dibenzothiophène étant relié au groupe phénylène pour p = 1 par l'intermédiaire de la position 1, 3 ou 4, respectivement à l'atome d'azote pour p = 0 ;
X étant C(R¹)₂, O ou S ;
R, R¹, identiques ou différents en chaque occurrence, étant choisis dans le groupe constitué par H, D, F, Cl, Br, I, CN, NO₂, N(R²)₂, OR², SR², C(=O)R², P(=O)R², Si(R²)₃, un groupe alkyle à chaîne droite comportant 1 à 20 atome(s) de C ou un groupe alkyle ramifié ou cyclique comportant 3 à 20 atomes de C ou un groupe alcényle ou alcynyle comportant 2 à 20 atomes de C, le groupe alkyle, alcényle ou alcynyle pouvant à chaque fois être substitué par un ou plusieurs radicaux R² et un ou plusieurs groupes CH₂ non adjacents pouvant être remplacés par R²C=CR², C≡C, Si(R²)₂, C=O, C=NR², P(=O)(R²), SO, SO₂, NR², O, S ou CONR² et un ou plusieurs atomes de H pouvant être remplacés par D, F, Cl, Br, I, CN ou NO₂, un système cyclique aromatique ou hétéroaromatique comportant 5 à 40 atomes de cycle aromatiques, qui peut à chaque fois être substitué par un ou plusieurs radicaux R² ; à cet égard optionnellement deux substituants voisins R respectivement deux substituants voisins R¹ peuvent former un système cyclique monocyclique ou polycyclique, aliphatique, aromatique ou hétéroaromatique, qui peut être substitué par un ou plusieurs radicaux R² ;
R², identique ou différent en chaque occurrence, étant choisi dans le groupe constitué par H, D, F, CN, un radical hydrocarboné aliphatique comportant 1 à 20 atome(s) de C, ou un système cyclique aromatique ou hétéroaromatique comportant 5 à 30 atomes de cycle aromatiques, dans lequel un ou plusieurs atomes de H peuvent être remplacés par D, F, CN ou un groupe alkyle comportant 1 à 10 atome (s) de C, deux substituants adjacents R² ou plus pouvant former ensemble un système cyclique aliphatique monocyclique ou polycyclique ;
m étant 0, 1, 2 ou 3 ;
n, identique ou différent en chaque occurrence, étant 0, 1, 2, 3, ou 4 ;
p étant 0 ou 1,
la position 1, 3 ou 4 étant définie comme ci-dessous :

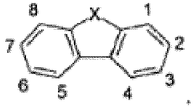

2. Composé selon la revendication 1, choisi parmi les composés des formules (1a) et (1b), 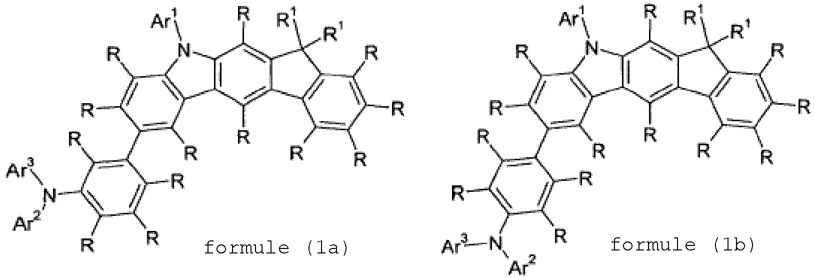 les symboles utilisés présentant les significations mentionnées dans la revendication 1.

3. Composé selon la revendication 1 ou 2 selon la formule (3), 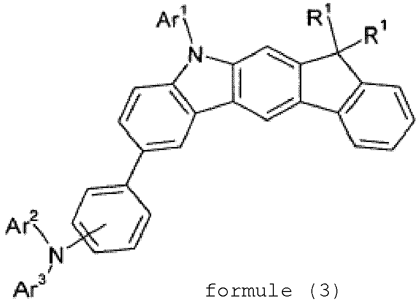 les symboles utilisés présentant les significations mentionnées dans la revendication 1.

4. Composé selon l'une ou plusieurs des revendications 1 à 3, **caractérisé en ce que** les radicaux R¹ au niveau de l'indénocarbazole, identiques ou différents en chaque occurrence, sont choisis dans le groupe constitué par un groupe alkyle à chaîne droite comportant 1 à 10 atome(s) de C ou un groupe alkyle ramifié ou cyclique comportant 3 à 10 atomes de C ou un groupe alcényle comportant 2 à 10 atomes de C, qui peut à chaque fois être substitué par un ou plusieurs radicaux R², un ou plusieurs groupes CH₂ non adjacents pouvant être remplacés par O et un ou plusieurs atomes de H pouvant être remplacés par D ou F, ou un système cyclique aromatique ou hétéroaromatique comportant 6 à 24 atomes de cycle aromatiques, qui peut à chaque fois être substitué par un ou plusieurs radicaux R² ; à cet égard, les deux substituants R¹ peuvent former un système cyclique monocyclique ou polycyclique, aliphatique, aromatique ou hétéroaromatique, qui peut être substitué par un ou plusieurs radicaux R².

5. Composé selon l'une ou plusieurs des revendications 1 à 4, choisi parmi les composés des formules (3a) et (3b), 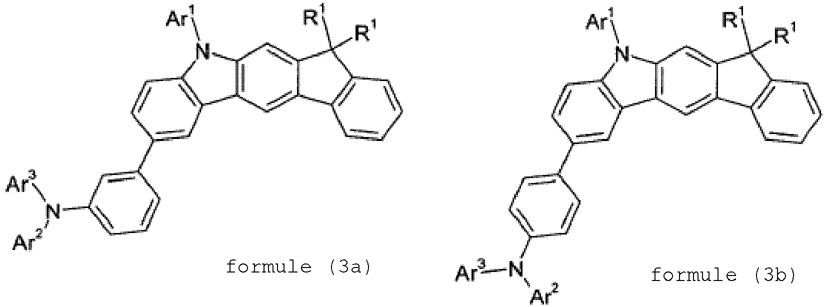 les symboles utilisés présentant les significations mentionnées dans la revendication 1.

6. Composé selon l'une ou plusieurs des revendications 1 à 5, **caractérisé en ce que** Ar¹ et Ar², identiques ou différents en chaque occurrence, sont choisis dans le groupe constitué par phényle, biphényle, terphényle, quaterphényle, fluorényle, spirobifluorényle, dibenzofurannyle, dibenzothiényle et N-phényl-carbazolyle, ces groupes pouvant à chaque fois être substitués par un ou plusieurs radicaux non aromatiques R.

7. Composé selon l'une ou plusieurs des revendications 1 à 6, **caractérisé en ce que** le groupe de formule (2) est choisi parmi les formules (2a) à (2c), 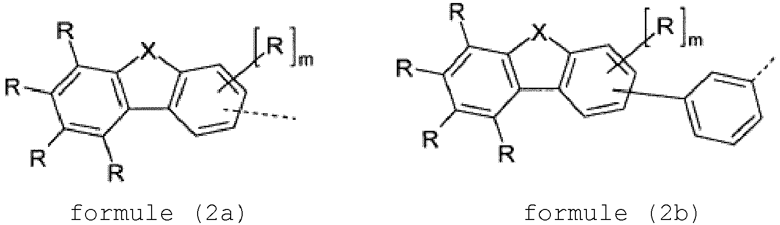 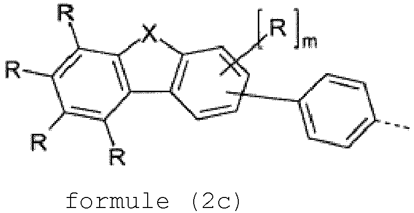 les symboles et les indices utilisés présentant les significations mentionnées dans la revendication 1 et le groupe fluorène respectivement le groupe dibenzofuranne respectivement le groupe dibenzothiophène étant relié par l'intermédiaire de la position 1, 3 ou 4.

8. Composé selon l'une ou plusieurs des revendications 1 à 7, **caractérisé en ce que** le groupe de formule (2) est choisi parmi les groupes des formules (2a-1) à (2a-9) 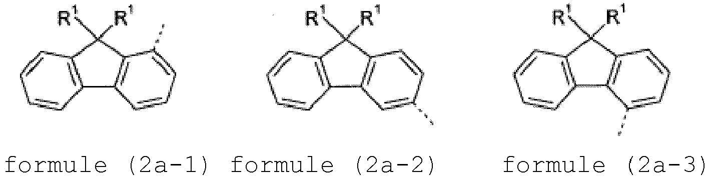 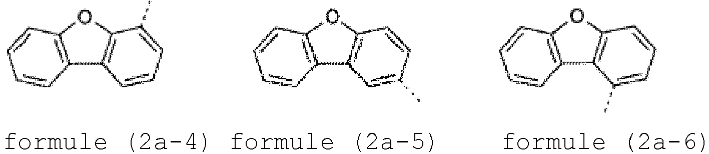 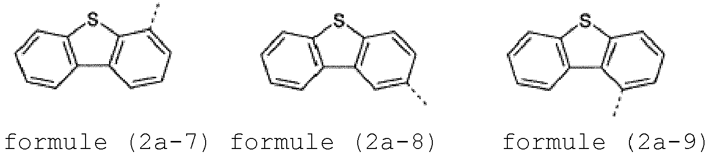 la liaison en pointillés indiquant le lien de ce groupe à l'atome d'azote et les autres symboles utilisés présentant les significations mentionnées dans la revendication 1.

9. Composé selon l'une ou plusieurs des revendications 1 à 8, **caractérisé en ce que** X représente C(R¹)₂ ou O et **en ce que** pour X = C(R¹)₂, R¹, identique ou différent en chaque occurrence, est choisi dans le groupe constitué par un groupe alkyle à chaîne droite comportant 1 à 10 atome(s) de C ou un groupe alkyle ramifié ou cyclique comportant 3 à 10 atomes de C ou un groupe alcényle comportant 2 à 10 atomes de C, qui peut à chaque fois être substitué par un ou plusieurs radicaux R², un ou plusieurs groupes CH₂ non adjacents pouvant être remplacés par O et un ou plusieurs atomes de H pouvant être remplacés par D ou F, ou un système cyclique aromatique ou hétéroaromatique comportant 6 à 24 atomes de cycle aromatiques, qui peut à chaque fois être substitué par un ou plusieurs radicaux R² ; à cet égard, les deux substituants R¹ peuvent former un système cyclique monocyclique ou polycyclique, aliphatique, aromatique ou hétéroaromatique, qui peut être substitué par un ou plusieurs radicaux R².

10. Composé selon l'une ou plusieurs des revendications 1 à 9, **caractérisé en ce que** R, identique ou différent en chaque occurrence, est choisi dans le groupe constitué par H, D, F, CN, N(R²)₂, OR², un groupe alkyle à chaîne droite comportant 1 à 10 atome (s) de C ou un groupe alkyle ramifié ou cyclique comportant 3 à 10 atome(s) de C ou un groupe alcényle comportant 2 à 10 atomes de C, qui peut être substitué à chaque fois par un ou plusieurs radicaux R², un ou plusieurs groupes CH₂ non adjacents pouvant être remplacés par O et un ou plusieurs atomes de H pouvant être remplacés par D ou F, ou un système cyclique aromatique ou hétéroaromatique comportant 5 à 30 atomes de cycle aromatiques, qui peut à chaque fois être substitué par un ou plusieurs radicaux R².

11. Procédé pour la préparation d'un composé selon l'une ou plusieurs des revendications 1 à 10, comprenant une réaction de couplage entre l'unité Ar²Ar³N-phénylène, qui est substituée par un groupe partant réactif, et un indénocarbazole, qui est substitué par un groupe partant réactif.

12. Formulation, contenant au moins un composé selon l'une ou plusieurs des revendications 1 à 10 et au moins un autre composé, en particulier un solvant.

13. Utilisation d'un composé selon l'une ou plusieurs des revendications 1 à 10 dans un dispositif électronique.

14. Dispositif électronique contenant un composé selon l'une ou plusieurs des revendications 1 à 10.

15. Dispositif électronique selon la revendication 14, qui est un dispositif organique d'électroluminescence, **caractérisé en ce que** le composé selon l'une ou plusieurs des revendications 1 à 13 est utilisé en tant que matériau de matrice pour des émetteurs phosphorescents dans une couche émettrice ou dans une couche de blocage d'électrons ou dans une couche de transport de trous ou dans une couche d'injection de trous.
